# EUROPEAN PATENT APPLICATION

(11) **EP 4 799 629 A1**
(43) Date of publication of application: **02.09.2026**
(21) Application number: 25161173.7
(22) Date of filing: 01.03.2025
(51) Int. Cl.: A61K 31/7088, A61P 27/06, C07K 16/24

(54) **MEANS AND METHODS FOR THE TREATMENT OF A SUBJECT SUFFERING FROM OR BEING AT RISK OF SUFFERING FROM GLAUCOMA**

(71) Applicant: TME Pharma AG, 10589 Berlin (DE); Singapore Health Services Pte Ltd, Singapore 168582 (SG); National University of Singapore, Singapore 119077 (SG)
(72) Inventor: WONG, Tina Tzee Ling, 168582 Singapore (SG); WANG, Xiaomeng, 169857 Singapore (SG); LIM, Seok Ting, 169857 Singapore (SG); EULBERG, Dirk, 10247 Berlin (DE); FRÖMMING, Anna, 14621 Schönwalde-Glien (DE)
(74) Representative: Bohmann, Armin K.

(57) **Abstract**

The present invention is related to an inhibitor of Monocyte Chemoattractant Protein-1 (CCL2) activity, for use in a method for treating a subject suffering from or being at risk of suffering from an eye disease, wherein the eye disease is glaucoma.

## Description

The present invention is related to a method for treating a subject suffering from or being at risk of suffering from glaucoma; a Monocyte Chemoattractant Protein-1 (also referred to as CCL2 or MCP-1) inhibitor for use in a method for treating a subject suffering from or being at risk of suffering from glaucoma; a method for treating a subject post glaucoma filtration surgery; a Monocyte Chemoattractant Protein-1 (also referred to as CCL2 or MCP-1) inhibitor for use in a method for treating a subject post glaucoma filtration surgery; a method for the treatment of a subject post glaucoma filtration surgery of an eye of the subject, wherein the treatment maintains functionality of vasculature of a bleb, preferably a bleb resulting from glaucoma filtration surgery; a Monocyte Chemoattractant Protein-1 (also referred to as CCL2 or MCP-1) inhibitor for use in a method for the treatment of a subject post glaucoma filtration surgery of an eye of the subject, wherein the treatment does not deteriorate the vasculature of a bleb, preferably a bleb resulting from glaucoma filtration surgery; a method for avoiding or decreasing deterioration of vasculature of a bleb, preferably a bleb resulting from glaucoma filtration surgery; a Monocyte Chemoattractant Protein-1 (also referred to as CCL2 or MCP-1) inhibitor for use in a method for avoiding or decreasing deterioration of vasculature of a bleb, preferably a bleb resulting from glaucoma filtration surgery; a method for treating a subject suffering from or being at risk of suffering from glaucoma, wherein the method avoids degeneration of vasculature of an eye of the subject, preferably degeneration of vasculature of an eye of the subject, wherein the eye is or has been subject to glaucoma filtration surgery; and a Monocyte Chemoattractant Protein-1 (also referred to as CCL2 or MCP-1) inhibitor for use in a method for treating a subject suffering from or being at risk of suffering from glaucoma, wherein the method avoids degeneration of vasculature of an eye of the subject (Yadgari 2018), preferably degeneration of vasculature of an eye of the subject, wherein the eye is or has been subject to glaucoma filtration surgery.

The human eye is a complex organ with a structure designed to capture light and convert it into neural signals that the brain interprets as vision. The general structure of the eye can be broken down into several key components, each of which can be involved in various disorders or pathologies (Kaplan 2007, Levin 2024). These elements are (i)the cornea, a transparent, dome-shaped surface that covers the front of the eye, functioning as a barrier to dirt, germs, and other particles and also helps focus incoming light; (ii) the aqueous humor, the clear fluid found between the cornea and the iris which provides nutrients to the avascular cornea and lens and maintains intraocular pressure; (iii) the iris, which controls the size of the pupil to regulate the amount of light entering the eye; (iv) the lens, a transparent, flexible structure that focuses light onto the retina and that, together with the cornea, refracts the light and ensure it is properly focused on the retina; (v) the vitreous humor, a clear gel-like substance that fills the space between the lens and retina which helps maintain the eye's shape and allows light to pass through to the retina; (vi) the retina, a thin layer of light-sensitive cells (photoreceptors) at the back of the eye which converts light into electrical signals that are sent to the brain via the optic nerve; (vii) the optic nerve which transmits visual information from the retina to the brain; (viii) the macula, a small central area of the retina responsible for focused, central vision, which also contains the fovea, which is the point of clearest vision; (ix) the sclera, the white, outer protective layer of the eye which maintains the eye's shape and provides protection; and (x) the choroid, a layer between the retina and sclera which is rich in blood vessels that provide oxygen and nutrients to the retina.

Disorders and pathologies associated with the eye (Salmon 2024) include refractive errors which can be subclassified into (i) myopia (nearsightedness), i.e. difficulty seeing distant objects clearly due to an elongated eyeball or overly curved cornea; (ii) hyperopia (farsightedness), i.e. difficulty seeing close objects clearly, typically due to a shorter eyeball or flatter cornea; (iii) astigmatism, i.e. irregular curvature of the cornea or lens causing blurred vision; (iv) presbyopia, i.e. age-related loss of near vision due to hardening of the lens; (v) cataract, clouding of the lens that can cause blurred or dimmed vision, a condition which is commonly age-related but can also be caused by trauma or other factors; (vi) glaucoma, a group of eye conditions characterized by increased intraocular pressure that can damage the optic nerve, leading to vision loss, where the two most frequent types are open-angle glaucoma (POAG) and angle-closure glaucoma (PACG); (vii) macular degeneration, which can be subclassified into (a) dry age-related macular degeneration (AMD) - the most common form of macular degeneration, accounting for about 85-90% of cases which occurs when the macula gradually thins and breaks down due to the accumulation of waste products called drusen (yellow deposits beneath the retina), a process that leads to a gradual loss of central vision; and (b) neovascular or wet or exudative age-related macular degeneration (nAMD) which occurs when abnormal blood vessels grow beneath the retina, leaking fluid and blood into the macula, causing rapid damage to the central vision due to the resulting inflammation which finally leads to scarring (fibrosis) and distortion of the macula and which can result in more rapid vision loss compared to dry AMD; (viii) diabetic retinopathy, a damage to retinal blood vessels caused by prolonged high blood sugar levels in diabetics which can lead to vision impairment or blindness if untreated; (ix) retinal detachment which occurs when the retina separates from the underlying layer of support tissue and which can lead to vision loss; (x) conjunctivitis, an inflammation of the conjunctiva, the thin layer covering the front of the eye which is caused by infections or allergic reactions; (xi) keratitis, an inflammation of the cornea, often caused by infection or trauma; (xii) strabismus, a misalignment of the eyes (crossed or wandering eyes), leading to double vision or poor depth perception, caused by neurological, muscular, or refractive issues; (xiii) blepharitis, an inflammation of the eyelids, often near the eyelashes, typically caused by bacterial infection or seborrheic dermatitis; (xiv) retinopathy of prematurity, abnormal growth of retinal blood vessels in premature infants, potentially leading to retinal detachment and blindness; (xv) dry eye syndrome, insufficient tear production or poor-quality tears, leading to discomfort, redness, and potential damage to the cornea; (xvi) optic neuritis, inflammation of the optic nerve, often linked with multiple sclerosis, causing vision loss or pain with eye movement; and (xvii) uveitis, an inflammation of the uvea which can lead to pain, light sensitivity, and vision loss.

According to the prior art, the goal of glaucoma treatment is to lower intraocular pressure to prevent further damage to the optic nerve. This can be achieved by medications, laser therapy and surgical therapy (Wagner, Stewart et al. 2022).

Medications that reduce IOP can be applied locally as eye drops or in the form of oral medications such as prostaglandin analogs such as latanoprost; beta-blockers such as timolol; alpha agonists such as brimonidine; carbonic anhydrase inhibitors such as dorzolamide; and Rho kinase inhibitors such as netarsudil. Advantages are that they are non-invasive and that they can effectively lower IOP, especially when started early. Disadvantages are adverse effects such as eye irritation, dry eyes, blurred vision, or systemic effects like fatigue or low blood pressure. Further disadvantages are that they require consistent use, and missing doses can lead to less effective management; some patients need to take multiple types of eye drops at different times of the day, which can be cumbersome and lead to missed doses or confusion, and, in general limited patient compliance. Over time, medications may become less effective which could be due to tolerance where the eye becomes less responsive to the medication.

Laser therapy can be used for POAG to improve drainage (selective laser trabeculoplasty or argon laser trabeculoplasty) whereas laser iridotomy is used in PACG patients to create a hole in the iris, improving fluid drainage. This is less invasive than surgical options with faster recovery time and is particularly helpful for POAG patients whose IOP isn't adequately controlled with medication. Laser therapy can sometimes reduce the need for long-term medications. Disadvantages are that effects are not always permanent and may wear off over time, requiring additional treatments. There is a potential for temporary inflammation, eye pain, or increases in IOP shortly after the procedure. Laser therapy is not suitable for all types of glaucoma, particularly advanced stages.

Surgical procedures to reduce IOP can be classified into trabeculectomy (glaucoma filtration surgery, GFS) that creates a fistula between the anterior chamber and the conjunctiva for fluid drainage and minimally invasive glaucoma surgery (MIGS). GFS is perceived as a long-term solution which can provide more lasting control over IOP, especially in advanced glaucoma, and it is effective for severe cases, i.e. patients who don't respond well to medications or laser treatments. Disadvantages are the invasiveness, risk of complications which includes the potential for scarring (fibrosis) which compromises long-term success of the intervention, infection, or over- or under-reduction of IOP, which might require additional interventions. Minimally invasive glaucoma surgery (MIGS) generally has fewer complications compared to traditional GFS and patients often experience a faster recovery time and less post-surgical discomfort, however it has only limited effectivity in severe cases and may not be as effective for patients with advanced glaucoma. Some MIGS techniques also may require further procedures over time.

GFS is a highly effective procedure for lowering IOP and remains one of the gold-standard surgeries for glaucoma, particularly for more complex or severe cases. The procedure involves a sclerectomy (a small hole in the sclera) covered by a partial-thickness scleral flap to the aqueous humor from the anterior chamber of the eye into the subconjunctival space (Weinreb, Leung et al. 2016); by creating a fistula between the anterior chamber and the exterior, the pressure is released. GFS can result in long-term control of IOP, with one study noting a stable reduction in IOP in 57% of patients (without the need for additional medication) and 88% of patients (with the need for additional medication), 20 years after GFS (Landers, Martin et al. 2012).

GFS has been the most frequently performed glaucoma surgery for more than 40 years (Weinreb, Leung et al. 2016), and it is estimated that approx. 20% of glaucoma patients will have incisional glaucoma surgery during the course of their disease (Hattenhauer, Johnson et al. 1999, Hu and Wang 2022).

The problem underlying the present invention is the provision of means and methods for treating diseases of the eye.

A further problem underlying the present invention is the provision of means and methods for treating a subject suffering from high intraocular pressure, whereby such intraocular pressure is a pathologically high intraocular pressure putting the subject at risk to become blind.

A still further problem underlying the present invention is the provision of means and methods for treating a subject who has undergone glaucoma filtration surgery or who will undergo glaucoma filtration surgery, preferably the subject is a subject suffering from or being at risk of suffering from glaucoma.

Another problem underlying the present invention is the provision of means and methods for reducing fibrosis in a subject suffering from or being at risk of suffering from an eye disease, preferably the eye disease is glaucoma.

A still further problem underlying the present invention is the provision of means and method for reducing fibrosis in a subject who has undergone glaucoma filtration surgery, who is undergoing glaucoma filtration surgery or who will be undergoing glaucoma filtration surgery, whereby preferably the subject is suffering from glaucoma or at risk of suffering from glaucoma.

In addition, a problem underlying the present invention is the provision of means and method for antifibrotic treatment while avoiding deterioration of health and/or functionality of a bleb, preferably a bleb generated in or by glaucoma filtration surgery, or for avoiding or decreasing deterioration of vasculature of a bleb, preferably a bleb generated in or by glaucoma filtration surgery.

Furthermore, a problem underlying the present invention is the provision of means and methods for avoiding side effects of mitomycin C and/or 5-fluorouracil used in the treatment of glaucoma and in particular in the treatment of a subject suffering from or being at risk of suffering from glaucoma and whereby the subject has undergone, is undergoing or will be undergoing glaucoma filtration surgery.

These and other problems underlying the present invention are solved by the subject matter of the attached independent claims. Preferred embodiments may be taken from the attached dependent claims.

Similarly, these and other problems underlying the present invention are solved by the subject matter of the Embodiments disclosed in the following. Additionally, these and other problems underlying the present invention are solved by the subject matter of the various aspect of the present invention, including any embodiment thereof, as disclosed herein.

Embodiment 1. An inhibitor of Monocyte Chemoattractant Protein-1 (CCL2) activity, for use in a method for treating a subject suffering from or being at risk of suffering from an eye disease, wherein the eye disease is glaucoma and wherein the inhibitor of CCL2 activity is locally and systemically administered to the subject.

Embodiment 2. The inhibitor of Monocyte Chemoattractant Protein-1 (CCL2) activity for use of Embodiment 1, wherein local administration is subconjunctival administration.

Embodiment 3. The inhibitor of Monocyte Chemoattractant Protein-1 (CCL2) activity for use of any one of Embodiments 1 to 2, wherein systemic administration is selected from the group comprising subcutaneous administration, intravenous administration and any combination thereof.

Embodiment 4. The inhibitor of Monocyte Chemoattractant Protein-1 (CCL2) activity for use of any one of Embodiments 1 to 3, wherein glaucoma is primary open-angle glaucoma (POAG) or primary angle-closure glaucoma (PACG).

Embodiment 5. The inhibitor of Monocyte Chemoattractant Protein-1 (CCL2) activity for use of any one of Embodiments 1 to 4, wherein the subject suffering from glaucoma or being at risk of suffering from glaucoma is a subject having one or more of a risk factor, wherein the risk factor is preferably selected from the group comprising age with older people being at higher risk; family history of glaucoma; ethnicity, with African Americans, Hispanics, and Asians being at higher risk; elevated intraocular pressure (IOP); systemic medical conditions such as diabetes, migraine, sickle cell anemia or high blood pressure; and eye conditions like high myopia or hyperopia, or previous eye injuries.

Embodiment 6. The inhibitor of Monocyte Chemoattractant Protein-1 (CCL2) activity for use of any one of Embodiments 1 to 5, wherein the subject is suffering from asymptomatic glaucoma.

Embodiment 7. The inhibitor of Monocyte Chemoattractant Protein-1 (CCL2) activity for use of any one of Embodiments 1 to 6, wherein the subject is or has been diagnosed as suffering from or being at risk of suffering from glaucoma by a method, wherein the method is selected from measurement of intraocular pressure (IOP) with a tonometer, optic nerve imaging using optical coherence tomography (OCT) or fundus photography, perimetry for assessing the field of vision, especially for peripheral vision loss; pachymetry for measuring the thickness of the cornea and a combination thereof.

Embodiment 8. The inhibitor of Monocyte Chemoattractant Protein-1 (CCL2) activity for use of any one of Embodiments 1 to 7, wherein the subject suffering from glaucoma or being at risk of suffering from glaucoma is a subject who qualifies for or is amenable to surgery for reducing intraocular pressure, preferably surgery for reducing intraocular pressure comprises glaucoma filtration surgery or minimally invasive glaucoma surgery.

Embodiment 9. The inhibitor of Monocyte Chemoattractant Protein-1 (CCL2) activity for use of Embodiment 8, wherein surgery for reducing intraocular pressure is glaucoma filtration surgery.

Embodiment 10. The inhibitor of Monocyte Chemoattractant Protein-1 (CCL2) activity for use of Embodiment 8, wherein surgery for reducing intraocular pressure is minimally invasive glaucoma surgery.

Embodiment 11. The inhibitor of Monocyte Chemoattractant Protein-1 (CCL2) activity for use of any one of Embodiments 8 to 10, preferably Embodiment 9, wherein the subject qualifying for or being amenable to surgery for reducing intraocular pressure, preferably glaucoma filtration surgery or minimally invasive glaucoma surgery, is characterized by a condition selected from the group consisting of uncontrolled intraocular pressure despite medication, advanced or severe glaucoma, failure of other surgeries or laser treatment, inability to tolerate medication, inability to adhere to a medication regimen, rapidly progressing glaucoma and glaucoma with high risk to prevent damage to the optic nerve.

Embodiment 12. The inhibitor of Monocyte Chemoattractant Protein-1 (CCL2) activity for use of Embodiment 11, wherein advanced or severe glaucoma is glaucoma with significant damage to the optic nerve, wherein failure of other surgeries or laser treatments means not providing sustained control of intraocular pressure.

Embodiment 13. The inhibitor of Monocyte Chemoattractant Protein-1 (CCL2) activity for use of any one of Embodiments 1 to 12, wherein the subject is a subject with risk of failure of surgery for reducing intraocular pressure, preferably glaucoma filtration surgery or minimally invasive glaucoma surgery, more preferably glaucoma filtration surgery.

Embodiment 14. The inhibitor of Monocyte Chemoattractant Protein-1 (CCL2) activity for use of Embodiment 13, wherein the subject is suffering from uveitic glaucoma.

Embodiment 15. The inhibitor of Monocyte Chemoattractant Protein-1 (CCL2) activity for use of any one of Embodiments 1 to 14, wherein the subject has undergone surgery for reducing intraocular pressure, preferably glaucoma filtration surgery or minimally invasive glaucoma surgery.

Embodiment 16. The inhibitor of Monocyte Chemoattractant Protein-1 (CCL2) activity for use of Embodiment 15, wherein the subject has undergone glaucoma filtration surgery.

Embodiment 17. The inhibitor of Monocyte Chemoattractant Protein-1 (CCL2) activity for use of Embodiment 15, wherein the subject has undergone minimally invasive glaucoma surgery

Embodiment 18. The inhibitor of Monocyte Chemoattractant Protein-1 (CCL2) activity for use of any one of Embodiments 15 to 17, wherein the subject has undergone surgery for reducing intraocular pressure, preferably glaucoma filtration surgery or minimally invasive glaucoma surgery, prior to administering the inhibitor of CCL2 activity.

Embodiment 19. The inhibitor of Monocyte Chemoattractant Protein-1 (CCL2) activity for use of any one of Embodiments 15 to 17, wherein the subject has undergone surgery for reducing intraocular pressure, preferably glaucoma filtration surgery or minimally invasive glaucoma surgery, after administering the inhibitor of CCL2 activity.

Embodiment 20. The inhibitor of Monocyte Chemoattractant Protein-1 (CCL2) activity for use of any one of Embodiments 15 to 17, wherein the subject has undergone surgery for reducing intraocular pressure, preferably glaucoma filtration surgery or minimally invasive glaucoma surgery, after systemically administering the inhibitor of CCL2 activity.

Embodiment 21. The inhibitor of Monocyte Chemoattractant Protein-1 (CCL2) activity for use of any one of Embodiments 18 to 20, wherein surgery for reducing intraocular pressure is glaucoma filtration surgery.

Embodiment 22. The inhibitor of Monocyte Chemoattractant Protein-1 (CCL2) activity for use of any one of Embodiments 18 to 21, wherein surgery for reducing intraocular pressure is or has been performed on an eye of the subject, preferably the eye is the eye of the subject to which the inhibitor of CCL2 activity is locally administered.

Embodiment 23. The inhibitor of Monocyte Chemoattractant Protein-1 (CCL2) activity for use of Embodiment 22, wherein surgery for reducing intraocular pressure is glaucoma filtration surgery.

Embodiment 24. The inhibitor of Monocyte Chemoattractant Protein-1 (CCL2) activity for use of Embodiment 22, wherein surgery for reducing intraocular pressure is minimally invasive glaucoma surgery.

Embodiment 25. The inhibitor of Monocyte Chemoattractant Protein-1 (CCL2) activity for use of any one of Embodiments 22 to 24, wherein the eye of the subject shows fibrosis or is at risk of showing fibrosis.

Embodiment 26. The inhibitor of Monocyte Chemoattractant Protein-1 (CCL2) activity for use of Embodiment 25, wherein fibrosis is or may be a consequence of surgery for reducing intraocular pressure, preferably of glaucoma filtration surgery or of minimally invasive glaucoma surgery.

Embodiment 27. The inhibitor of Monocyte Chemoattractant Protein-1 (CCL2) activity for use of Embodiment 26, wherein fibrosis is or may be a consequence of glaucoma filtration surgery.

Embodiment 28. The inhibitor of Monocyte Chemoattractant Protein-1 (CCL2) activity for use of any one of Embodiments 1 to 27, wherein the subject suffering from glaucoma or being at risk of suffering from glaucoma is a subject who underwent revision surgery after surgery for reducing intraocular pressure, who is undergoing revision surgery after surgery for reducing intraocular pressure or who will be undergoing revision surgery after surgery for reducing intraocular pressure .

Embodiment 29. The inhibitor of Monocyte Chemoattractant Protein-1 (CCL2) activity for use of Embodiment 28, wherein surgery for reducing intraocular pressure is glaucoma filtration surgery.

Embodiment 30. The inhibitor of Monocyte Chemoattractant Protein-1 (CCL2) activity for use of Embodiment 28, wherein surgery for reducing intraocular pressure is minimally invasive glaucoma surgery.

Embodiment 31. The inhibitor of Monocyte Chemoattractant Protein-1 (CCL2) activity for use of any one of Embodiments 25 to 30, wherein fibrosis causes an increase of intraocular pressure in an eye on which surgery for reducing intraocular pressure has been performed, preferably in an eye on which glaucoma filtration surgery or minimally invasive glaucoma surgery has been performed.

Embodiment 32. The inhibitor of Monocyte Chemoattractant Protein-1 (CCL2) activity for use of Embodiment 31, wherein fibrosis causes an increase in intraocular pressure in an eye on which glaucoma filtration surgery has been performed.

Embodiment 33. The inhibitor of Monocyte Chemoattractant Protein-1 (CCL2) activity for use of any one of Embodiments 1 to 32, wherein mitomycin C and/or 5-fluorouracil is administered to the subject, preferably a subtherapeutic amount of mitomycin C and/or 5-fluorouracil is administered to the subject, more preferably a subtherapeutic amount of mitomycin C and/or 5-fluorouracil is locally administered to the subject, most preferably a subtherapeutic amount of mitomycin C and/or 5-fluorouracil is locally administered to the subject at the site of glaucoma filtration surgery.

Embodiment 34. An inhibitor of Monocyte Chemoattractant Protein-1 (CCL2) activity, for use in a method for treating a subject post surgery for reducing intraocular pressure, preferably post glaucoma filtration surgery or post minimally invasive glaucoma surgery, wherein the inhibitor of CCL2 activity is locally and systemically administered to the subject.

Embodiment 35. The inhibitor of Monocyte Chemoattractant Protein-1 (CCL2) activity for use of Embodiment 34, wherein surgery for reducing intraocular pressure is glaucoma filtration surgery.

Embodiment 36. The inhibitor of Monocyte Chemoattractant Protein-1 (CCL2) activity for use of any one of Embodiments 34 and 35, wherein local administration is subconjunctival administration.

Embodiment 37. The inhibitor of Monocyte Chemoattractant Protein-1 (CCL2) activity for use of any one of Embodiments 34 to 36, wherein systemic administration is selected from the group comprising subcutaneous administration, intravenous administration and any combination thereof.

Embodiment 38. The inhibitor of Monocyte Chemoattractant Protein-1 (CCL2) activity for use of any one of Embodiments 34 to 37, wherein the subject is suffering from glaucoma, has been suffering from glaucoma or is at risk of suffering from glaucoma.

Embodiment 39. The inhibitor of Monocyte Chemoattractant Protein-1 (CCL2) activity for use of Embodiment 38, wherein glaucoma is primary open-angle glaucoma (POAG) or primary angle-closure glaucoma (PACG).

Embodiment 40. The inhibitor of Monocyte Chemoattractant Protein-1 (CCL2) activity for use of any one of Embodiments 34 to 39, wherein the subject is suffering from asymptomatic glaucoma.

Embodiment 41. The inhibitor of Monocyte Chemoattractant Protein-1 (CCL2) activity for use of any one of Embodiments 34 to 39, wherein the subject has been suffering from glaucoma prior to surgery for reducing intraocular pressure, preferably prior to glaucoma filtration surgery or prior to minimally invasive glaucoma surgery.

Embodiment 42. The inhibitor of Monocyte Chemoattractant Protein-1 (CCL2) activity for use of Embodiment 41, wherein surgery for reducing intraocular pressure is glaucoma filtration surgery.

Embodiment 43. The inhibitor of Monocyte Chemoattractant Protein-1 (CCL2) activity for use of any one of Embodiments 38 to 42, wherein the subject who is suffering from glaucoma, has been suffering from or is at risk of suffering from glaucoma is a subject having one or more of a risk factor, wherein the risk factor is preferably selected from the group comprising age with older people being at higher risk; family history of glaucoma; ethnicity, with African Americans, Hispanics, and Asians being at higher risk; elevated intraocular pressure (IOP); systemic medical conditions such as diabetes, migraine, sickle cell anemia or high blood pressure; and eye conditions like high myopia or hyperopia, or previous eye injuries.

Embodiment 44. The inhibitor of Monocyte Chemoattractant Protein-1 (CCL2) activity for use of any one of Embodiments 38 to 43, wherein the subject is or has been diagnosed as suffering from or being at risk of suffering from glaucoma by a method, wherein the method is selected from measurement of intraocular pressure (IOP) with a tonometer, optic nerve imaging using optical coherence tomography (OCT) or fundus photography, perimetry for assessing the field of vision, especially for peripheral vision loss; pachymetry for measuring the thickness of the cornea and a combination thereof.

Embodiment 45. The inhibitor of Monocyte Chemoattractant Protein-1 (CCL2) activity for use of any one of Embodiments 34 to 44, wherein the subject is a subject who qualifies for or is amenable to surgery for reducing intraocular pressure, preferably the subject is a subject suffering from glaucoma or being at risk of suffering from glaucoma.

Embodiment 46. The inhibitor of Monocyte Chemoattractant Protein-1 (CCL2) activity for use of Embodiment 45, wherein surgery for reducing intraocular pressure is glaucoma filtration surgery.

Embodiment 47. The inhibitor of Monocyte Chemoattractant Protein-1 (CCL2) activity for use of Embodiment 45, wherein surgery for reducing intraocular pressure is minimally invasive glaucoma surgery.

Embodiment 48. The inhibitor of Monocyte Chemoattractant Protein-1 (CCL2) activity for use of any one of Embodiments 45 to 47, wherein the subject qualifying for or being amenable to surgery for reducing intraocular pressure, preferably glaucoma filtration surgery or minimally invasive glaucoma surgery, is characterized by a condition selected from the group consisting of uncontrolled intraocular pressure despite medication, advanced or severe glaucoma, failure of other surgeries or laser treatment, inability to tolerate medication, inability to adhere to a medication regimen, rapidly progressing glaucoma and glaucoma with high risk to prevent damage to the optic nerve.

Embodiment 49. The inhibitor of Monocyte Chemoattractant Protein-1 (CCL2) activity for use of Embodiment 48, wherein advanced or severe glaucoma is glaucoma with significant damage to the optic nerve, wherein failure of other surgeries or laser treatments means not providing sustained control of intraocular pressure.

Embodiment 50. The inhibitor of Monocyte Chemoattractant Protein-1 (CCL2) activity for use of any one of Embodiments 48 to 49, wherein the subject qualifying for or being amenable to surgery for reducing intraocular pressure is a subject qualifying for or being amenable to glaucoma filtration surgery.

Embodiment 51. The inhibitor of Monocyte Chemoattractant Protein-1 (CCL2) activity for use of any one of Embodiments 34 to 50, wherein the subject is a subject with risk of failure of surgery for reducing intraocular pressure, preferably surgery for reducing intraocular pressure is glaucoma filtration surgery or minimally invasive glaucoma surgery.

Embodiment 52. The inhibitor of Monocyte Chemoattractant Protein-1 (CCL2) activity for use of Embodiment 51, wherein the subject is a subject with risk of failure of glaucoma filtration surgery.

Embodiment 53. The inhibitor of Monocyte Chemoattractant Protein-1 (CCL2) activity for use of any one of Embodiments 51 to 52, wherein the subject is suffering from uveitic glaucoma.

Embodiment 54. The inhibitor of Monocyte Chemoattractant Protein-1 (CCL2) activity for use of any one of Embodiments 34 to 53, wherein surgery for reducing intraocular pressure, preferably glaucoma filtration surgery or minimally invasive glaucoma surgery, is or will be performed on an eye of the subject, preferably the eye is the eye to which the inhibitor of CCL2 activity is locally administered.

Embodiment 55. The inhibitor of Monocyte Chemoattractant Protein-1 (CCL2) activity for use of Embodiment 54, wherein glaucoma filtration surgery is or will be performed on an eye of the subject, preferably the eye is the eye to which the inhibitor of CCL2 activity is locally administered.

Embodiment 56. The inhibitor of Monocyte Chemoattractant Protein-1 (CCL2) activity for use of any one of Embodiments 54 and 55, wherein the eye of the subject shows fibrosis or is at risk of showing fibrosis.

Embodiment 57. The inhibitor of Monocyte Chemoattractant Protein-1 (CCL2) activity for use of Embodiment 56, wherein fibrosis causes or may cause an increase in intraocular pressure in the eye on which surgery for reducing intraocular pressure is performed.

Embodiment 58. The inhibitor of Monocyte Chemoattractant Protein-1 (CCL2) activity for use of Embodiment 57, wherein surgery for reducing intraocular pressure is glaucoma filtration surgery.

Embodiment 59. The inhibitor of Monocyte Chemoattractant Protein-1 (CCL2) activity for use of any one of Embodiments 34 to 58, wherein mitomycin C and/or 5-fluorouracil is administered to the subject, preferably a subtherapeutic amount of mitomycin C and/or 5-fluorouracil is administered to the subject, more preferably a subtherapeutic amount of mitomycin C and/or 5-fluorouracil is locally administered to the subject, most preferably a subtherapeutic amount of mitomycin C and/or 5-fluorouracil is locally administered to the subject at the site of glaucoma filtration surgery.

Embodiment 60. An inhibitor of Monocyte Chemoattractant Protein-1 (CCL2) activity, for use in a method for treating a subject post glaucoma filtration surgery of an eye of the subject, wherein the method comprises administering the inhibitor of CCL2 activity to the subject for maintaining functionality of vasculature of a bleb or for not deteriorating the vasculature of the bleb, wherein the bleb results from glaucoma filtration surgery or for avoiding or decreasing deterioration of vasculature of a bleb resulting from glaucoma filtration surgery.

Embodiment 61. The inhibitor of Monocyte Chemoattractant Protein-1 (CCL2) activity for use of Embodiment 60, wherein the inhibitor of CCL2 activity is locally administered to the subject.

Embodiment 62. The inhibitor of Monocyte Chemoattractant Protein-1 (CCL2) activity for use of any one of Embodiments 60 to 61, wherein the inhibitor of CCL2 activity is systemically administered to the subject.

Embodiment 63. The inhibitor of Monocyte Chemoattractant Protein-1 (CCL2) activity for use of any one of Embodiments 60 to 62, wherein the inhibitor of CCL2 activity is administered both locally and systemically.

Embodiment 64. The inhibitor of Monocyte Chemoattractant Protein-1 (CCL2) activity for use of any one of Embodiments 61 to 63, wherein local administration is subconjunctival administration.

Embodiment 65. The inhibitor of Monocyte Chemoattractant Protein-1 (CCL2) activity for use of any one of Embodiments 62 to 63, wherein systemic administration is selected from the group comprising subcutaneous administration, intravenous administration and any combination thereof.

Embodiment 66. The inhibitor of Monocyte Chemoattractant Protein-1 (CCL2) activity for use of any one of Embodiments 60 to 65, wherein the subject is suffering from glaucoma, has been suffering from glaucoma or is at risk of suffering from glaucoma.

Embodiment 67. The inhibitor of Monocyte Chemoattractant Protein-1 (CCL2) activity for use of Embodiment 66, wherein glaucoma is primary open-angle glaucoma (POAG) or primary angle-closure glaucoma (PACG).

Embodiment 68. The inhibitor of Monocyte Chemoattractant Protein-1 (CCL2) activity for use of any one of Embodiments 60 to 67, wherein the subject is suffering from asymptomatic glaucoma.

Embodiment 69. The inhibitor of Monocyte Chemoattractant Protein-1 (CCL2) activity for use of any one of Embodiments 60 to 67, wherein the subject has been suffering from glaucoma prior to glaucoma filtration surgery.

Embodiment 70. The inhibitor of Monocyte Chemoattractant Protein-1 (CCL2) activity for use of Embodiment 69, wherein intraocular pressure of subject is or has been > 21 mm Hg prior to glaucoma filtration surgery.

Embodiment 71. The inhibitor of Monocyte Chemoattractant Protein-1 (CCL2) activity for use of any one of Embodiments 66 to 70, wherein the subject suffering from glaucoma or being at risk of suffering from glaucoma is a subject having one or more of a risk factor, wherein the risk factor is preferably selected from the group comprising age with older people being at higher risk; family history of glaucoma; ethnicity, with African Americans, Hispanics, and Asians being at higher risk; elevated intraocular pressure (IOP); systemic medical conditions such as diabetes, migraine, sickle cell anemia or high blood pressure; and eye conditions like high myopia or hyperopia, or previous eye injuries.

Embodiment 72. The inhibitor of Monocyte Chemoattractant Protein-1 (CCL2) activity for use of any one of Embodiments 66 to 71, wherein the subject is or has been diagnosed as suffering from or being at risk of suffering from glaucoma by a method, wherein the method is selected from measurement of intraocular pressure (IOP) with a tonometer, optic nerve imaging using optical coherence tomography (OCT) or fundus photography, perimetry for assessing the field of vision, especially for peripheral vision loss; pachymetry for measuring the thickness of the cornea and a combination thereof.

Embodiment 73. The inhibitor of Monocyte Chemoattractant Protein-1 (CCL2) activity for use of any one of Embodiments 60 to 72, wherein the subject is a subject who qualifies for or is amenable to glaucoma filtration surgery, preferably the subject is a subject suffering from glaucoma or being at risk of suffering from glaucoma.

Embodiment 74. The inhibitor of Monocyte Chemoattractant Protein-1 (CCL2) activity for use of Embodiment 73, wherein the subject qualifying for or being amenable to glaucoma filtration surgery is characterized by a condition selected from the group consisting of uncontrolled intraocular pressure despite medication, advanced or severe glaucoma, failure of other surgeries or laser treatment, inability to tolerate medication, inability to adhere to a medication regimen, rapidly progressing glaucoma and glaucoma with high risk to prevent damage to the optic nerve.

Embodiment 75. The inhibitor of Monocyte Chemoattractant Protein-1 (CCL2) activity for use of Embodiment 74, wherein advanced or severe glaucoma is glaucoma with significant damage to the optic nerve, wherein failure of other surgeries or laser treatments means not providing sustained control of intraocular pressure.

Embodiment 76. The inhibitor of Monocyte Chemoattractant Protein-1 (CCL2) activity for use of any one of Embodiments 60 to 75, wherein the subject is a subject with risk of failure of glaucoma filtration surgery.

Embodiment 77. The inhibitor of Monocyte Chemoattractant Protein-1 (CCL2) activity for use of Embodiment 76, wherein the subject is suffering from uveitic glaucoma.

Embodiment 78. The inhibitor of Monocyte Chemoattractant Protein-1 (CCL2) activity for use of any one of Embodiments 60 to 77, wherein glaucoma filtration surgery is or will be performed on an eye of the subject.

Embodiment 79. The inhibitor of Monocyte Chemoattractant Protein-1 (CCL2) activity for use of Embodiment 78, wherein the eye of the subject on which glaucoma filtration surgery is performed is the eye that shows fibrosis or is at risk of showing fibrosis.

Embodiment 80. The inhibitor of Monocyte Chemoattractant Protein-1 (CCL2) activity for use of Embodiment 79, wherein fibrosis causes or may cause an increase in intraocular pressure in the eye.

Embodiment 81. The inhibitor of Monocyte Chemoattractant Protein-1 (CCL2) activity for use of any one of Embodiments 60 to 80, wherein the inhibitor of CCL2 activity is anti-fibrotic.

Embodiment 82. An inhibitor of Monocyte Chemoattractant Protein-1 (also referred to as CCL2 or MCP-1) activity, for use in a method for avoiding or decreasing deterioration of vasculature of a bleb, preferably a bleb resulting from glaucoma filtration surgery, wherein the method comprises administering the inhibitor of CCL2 activity to the subject.

Embodiment 83. The inhibitor of Monocyte Chemoattractant Protein-1 (CCL2) activity for use of Embodiment 82, wherein the inhibitor of CCL2 activity is locally administered to the subject.

Embodiment 84. The inhibitor of Monocyte Chemoattractant Protein-1 (CCL2) activity for use of any one of Embodiments 82 to 83, wherein the inhibitor of CCL2 activity is systemically administered to the subject.

Embodiment 85. The inhibitor of Monocyte Chemoattractant Protein-1 (CCL2) activity for use of any one of Embodiments 82 to 84, wherein the inhibitor of CCL2 activity is administered both locally and systemically.

Embodiment 86. The inhibitor of Monocyte Chemoattractant Protein-1 (CCL2) activity for use of any one of Embodiments 83 to 85, wherein local administration is subconjunctival administration.

Embodiment 87. The inhibitor of Monocyte Chemoattractant Protein-1 (CCL2) activity for use of any one of Embodiments 84 to 85, wherein systemic administration is selected from the group comprising subcutaneous administration, intravenous administration and any combination thereof.

Embodiment 88. The inhibitor of Monocyte Chemoattractant Protein-1 (CCL2) activity for use of any one of Embodiments 82 to 87, wherein the subject is suffering from glaucoma, has been suffering from glaucoma or is at risk of suffering from glaucoma.

Embodiment 89. The inhibitor of Monocyte Chemoattractant Protein-1 (CCL2) activity for use of Embodiment 88, wherein glaucoma is primary open-angle glaucoma (POAG) or primary angle-closure glaucoma (PACG).

Embodiment 90. The inhibitor of Monocyte Chemoattractant Protein-1 (CCL2) activity for use of any one of Embodiments 82 to 89, wherein the subject is suffering from asymptomatic glaucoma.

Embodiment 91. The inhibitor of Monocyte Chemoattractant Protein-1 (CCL2) activity for use of any one of Embodiments 82 to 89, wherein the subject has been suffering from glaucoma prior to glaucoma filtration surgery.

Embodiment 92. The inhibitor of Monocyte Chemoattractant Protein-1 (CCL2) activity for use of Embodiment 91, wherein intraocular pressure of subject is or has been > 21 mm Hg prior to glaucoma filtration surgery.

Embodiment 93. The inhibitor of Monocyte Chemoattractant Protein-1 (CCL2) activity for use of any one of Embodiments 88 to 92, wherein the subject suffering from glaucoma or being at risk of suffering from glaucoma is a subject having one or more of a risk factor, wherein the risk factor is preferably selected from the group comprising age with older people being at higher risk; family history of glaucoma; ethnicity, with African Americans, Hispanics, and Asians being at higher risk; elevated intraocular pressure (IOP); systemic medical conditions such as diabetes, migraine, sickle cell anemia or high blood pressure; and eye conditions like high myopia or hyperopia, or previous eye injuries.

Embodiment 94. The inhibitor of Monocyte Chemoattractant Protein-1 (CCL2) activity for use of any one of Embodiments 88 to 93, wherein the subject is or has been diagnosed as suffering from or being at risk of suffering from glaucoma by a method, wherein the method is selected from measurement of intraocular pressure (IOP) with a tonometer, optic nerve imaging using optical coherence tomography (OCT) or fundus photography, perimetry for assessing the field of vision, especially for peripheral vision loss; pachymetry for measuring the thickness of the cornea and a combination thereof.

Embodiment 95. The inhibitor of Monocyte Chemoattractant Protein-1 (CCL2) activity for use of any one of Embodiments 82 to 94, wherein the subject is a subject who qualifies for or is amenable to glaucoma filtration surgery, preferably the subject is a subject suffering from glaucoma or being at risk of suffering from glaucoma.

Embodiment 96. The inhibitor of Monocyte Chemoattractant Protein-1 (CCL2) activity for use of Embodiment 95, wherein the subject qualifying for or being amenable to glaucoma filtration surgery is characterized by a condition selected from the group consisting of uncontrolled intraocular pressure despite medication, advanced or severe glaucoma, failure of other surgeries or laser treatment, inability to tolerate medication, inability to adhere to a medication regimen, rapidly progressing glaucoma and glaucoma with high risk to prevent damage to the optic nerve.

Embodiment 97. The inhibitor of Monocyte Chemoattractant Protein-1 (CCL2) activity for use of Embodiment 96, wherein advanced or severe glaucoma is glaucoma with significant damage to the optic nerve, wherein failure of other surgeries or laser treatments means not providing sustained control of intraocular pressure.

Embodiment 98. The inhibitor of Monocyte Chemoattractant Protein-1 (CCL2) activity for use of any one of Embodiments 82 to 97, wherein the subject is a subject with risk of failure of glaucoma filtration surgery.

Embodiment 99. The inhibitor of Monocyte Chemoattractant Protein-1 (CCL2) activity for use of Embodiment 98, wherein the subject is suffering from uveitic glaucoma.

Embodiment 100. The inhibitor of Monocyte Chemoattractant Protein-1 (CCL2) activity for use of any one of Embodiments 88 to 99, wherein glaucoma filtration surgery is or will be performed on an eye of the subject.

Embodiment 101. The inhibitor of Monocyte Chemoattractant Protein-1 (CCL2) activity for use of Embodiment 100, wherein the eye of the subject on which glaucoma filtration surgery is performed is the eye that shows fibrosis or is at risk of showing fibrosis.

Embodiment 102. The inhibitor of Monocyte Chemoattractant Protein-1 (CCL2) activity for use of Embodiment 101, wherein fibrosis causes or may cause an increase in intraocular pressure in the eye.

Embodiment 103. The inhibitor of Monocyte Chemoattractant Protein-1 (CCL2) activity for use of any one of Embodiments 82 to 102, wherein the inhibitor of CCL2 activity is anti-fibrotic.

Embodiment 104. An inhibitor of Monocyte Chemoattractant Protein-1 (also referred to as CCL2 or MCP-1) activity, for use in a method for treating a subject suffering from or being at risk of suffering from glaucoma, wherein the method avoids degeneration of vasculature of an eye of the subject or is for avoiding degeneration of vasculature of an eye of the subject, wherein the method comprises administering the inhibitor of CCL2 activity to the subject.

Embodiment 105. The inhibitor of Monocyte Chemoattractant Protein-1 (CCL2) activity for use of Embodiment 104, wherein the eye is or has been subject to glaucoma filtration surgery.

Embodiment 106. The inhibitor of Monocyte Chemoattractant Protein-1 (CCL2) activity for use of any one of Embodiments 104 to 105, wherein the vasculature is vasculature of a bleb, preferably a bleb resulting from glaucoma filtration surgery.

Embodiment 107. The inhibitor of Monocyte Chemoattractant Protein-1 (CCL2) activity for use of any one of Embodiments 104 to 106, wherein the inhibitor of CCL2 activity is locally administered to the subject.

Embodiment 108. The inhibitor of Monocyte Chemoattractant Protein-1 (CCL2) activity for use of any one of Embodiments 104 to 107, wherein the inhibitor of CCL2 activity is systemically administered to the subject.

Embodiment 109. The inhibitor of Monocyte Chemoattractant Protein-1 (CCL2) activity for use of any one of Embodiments 104 to 108, wherein the inhibitor of CCL2 activity is administered both locally and systemically.

Embodiment 110. The inhibitor of Monocyte Chemoattractant Protein-1 (CCL2) activity for use of any one of Embodiments 107 to 109, wherein local administration is subconjunctival administration.

Embodiment 111. The inhibitor of Monocyte Chemoattractant Protein-1 (CCL2) activity for use of any one of Embodiments 108 to 109, wherein systemic administration is selected from the group comprising subcutaneous administration, intravenous administration and any combination thereof.

Embodiment 112. The inhibitor of Monocyte Chemoattractant Protein-1 (CCL2) activity for use of any one of Embodiments 104 to 111, wherein the subject is suffering from glaucoma, has been suffering from glaucoma or is at risk of suffering from glaucoma.

Embodiment 113. The inhibitor of Monocyte Chemoattractant Protein-1 (CCL2) activity for use of Embodiment 112, wherein glaucoma is primary open-angle glaucoma (POAG) or primary angle-closure glaucoma (PACG).

Embodiment 114. The inhibitor of Monocyte Chemoattractant Protein-1 (CCL2) activity for use of any one of Embodiments 104 to 113, wherein the subject is suffering from asymptomatic glaucoma.

Embodiment 115. The inhibitor of Monocyte Chemoattractant Protein-1 (CCL2) activity for use of any one of Embodiments 104 to 113, wherein the subject has been suffering from glaucoma prior to glaucoma filtration surgery.

Embodiment 116. The inhibitor of Monocyte Chemoattractant Protein-1 (CCL2) activity for use of Embodiment 115, wherein intraocular pressure of subject is or has been > 21 mm Hg prior to glaucoma filtration surgery.

Embodiment 117. The inhibitor of Monocyte Chemoattractant Protein-1 (CCL2) activity for use of any one of Embodiments 112 to 116, wherein the subject suffering from glaucoma or being at risk of suffering from glaucoma is a subject having one or more of a risk factor, wherein the risk factor is preferably selected from the group comprising age with older people being at higher risk; family history of glaucoma; ethnicity, with African Americans, Hispanics, and Asians being at higher risk; elevated intraocular pressure (IOP); systemic medical conditions such as diabetes, migraine, sickle cell anemia or high blood pressure; and eye conditions like high myopia or hyperopia, or previous eye injuries.

Embodiment 118. The inhibitor of Monocyte Chemoattractant Protein-1 (CCL2) activity for use of any one of Embodiments 112 to 117, wherein the subject is or has been diagnosed as suffering from or being at risk of suffering from glaucoma by a method, wherein the method is selected from measurement of intraocular pressure (IOP) with a tonometer, optic nerve imaging using optical coherence tomography (OCT) or fundus photography, perimetry for assessing the field of vision, especially for peripheral vision loss; pachymetry for measuring the thickness of the cornea and a combination thereof.

Embodiment 119. The inhibitor of Monocyte Chemoattractant Protein-1 (CCL2) activity for use of any one of Embodiments 104 to 118, wherein the subject is a subject who qualifies for or is amenable to glaucoma filtration surgery, preferably the subject is a subject suffering from glaucoma or being at risk of suffering from glaucoma.

Embodiment 120. The inhibitor of Monocyte Chemoattractant Protein-1 (CCL2) activity for use of Embodiment 119, wherein the subject qualifying for or being amenable to glaucoma filtration surgery is characterized by a condition selected from the group consisting of uncontrolled intraocular pressure despite medication, advanced or severe glaucoma, failure of other surgeries or laser treatment, inability to tolerate medication, inability to adhere to a medication regimen, rapidly progressing glaucoma and glaucoma with high risk to prevent damage to the optic nerve.

Embodiment 121. The inhibitor of Monocyte Chemoattractant Protein-1 (CCL2) activity for use of Embodiment 120, wherein advanced or severe glaucoma is glaucoma with significant damage to the optic nerve, wherein failure of other surgeries or laser treatments means not providing sustained control of intraocular pressure.

Embodiment 122. The inhibitor of Monocyte Chemoattractant Protein-1 (CCL2) activity for use of any one of Embodiments 104 to 121, wherein the subject is a subject with risk of failure of glaucoma filtration surgery.

Embodiment 123. The inhibitor of Monocyte Chemoattractant Protein-1 (CCL2) activity for use of Embodiment 122, wherein the subject is suffering from uveitic glaucoma.

Embodiment 124. The inhibitor of Monocyte Chemoattractant Protein-1 (CCL2) activity for use of any one of Embodiments 112 to 123, wherein glaucoma filtration surgery is or will be performed on an eye of the subject.

Embodiment 125. The inhibitor of Monocyte Chemoattractant Protein-1 (CCL2) activity for use of Embodiment 124, wherein the eye of the subject on which glaucoma filtration surgery is performed is the eye that shows fibrosis or is at risk of showing fibrosis.

Embodiment 126. The inhibitor of Monocyte Chemoattractant Protein-1 (CCL2) activity for use of Embodiment 125, wherein fibrosis causes or may cause an increase in intraocular pressure in the eye.

Embodiment 127. The inhibitor of Monocyte Chemoattractant Protein-1 (CCL2) activity for use of any one of Embodiments 1 to 126, wherein the inhibitor of CCL2 activity is anti-fibrotic.

Embodiment 128. The inhibitor of Monocyte Chemoattractant Protein-1 (CCL2) activity for use of any one of Embodiments 1 to 127, wherein the inhibitor of CCL2 activity is a compound selected from the group comprising a compound binding to CCL2, a compound binding to CCR2, a compound inhibiting activity of CCR2 and a compound inhibiting signaling of CCR2.

Embodiment 129. The inhibitor of Monocyte Chemoattractant Protein-1 (CCL2) activity for use of Embodiment 128, wherein the compound inhibiting activity of CCR2 is a compound binding to CCR2 or the compound inhibiting activity of CCR2 is a compound binding to CCL2.

Embodiment 130. The inhibitor of Monocyte Chemoattractant Protein-1 (CCL2) activity for use of and one of Embodiments 1 to 129, wherein the inhibitor of CCL2 is a compound inhibiting signaling of CCR2, preferably the compound is binding to CCL2 or is binding to CCR2.

Embodiment 131. The inhibitor of Monocyte Chemoattractant Protein-1 (CCL2) activity for use of any one of Embodiments 1 to 130, wherein the inhibitor of CCL2 activity is a compound selected from the group comprising an aptamer binding to CCL2, an aptamer binding to CCR2, an aptamer inhibiting signaling of CCR2, preferably by binding to CCL2 or to CCR2, a Spiegelmer binding to CCL2, a Spiegelmer binding to CCR2, a Spiegelmer inhibiting signaling of CCR2, preferably by binding to CCL2 or to CCR2, an antibody binding to CCL2, an antibody binding to CCR2, an antibody inhibiting signaling of CCR2, preferably by binding to CCL2 or to CCR2, an antibody fragment binding to CCL2, an antibody fragment binding to CCR2, an antibody fragment inhibiting signaling of CCR2, preferably by binding to CCL2 or to CCR2, a protein binding to CCL2, a protein binding to CCR2, a protein inhibiting signaling of CCR2, preferably by binding to CCL2 or to CCR2, an anticalin binding to CCL2, an anticalin binding to CCR2, an anticalin inhibiting signaling of CCR2, preferably by binding to CCL2 or to CCR2, a small molecule binding to CCL2, a small molecule binding to CCR2 and a small molecule inhibiting signaling of CCR2, preferably by binding to CCL2 or to CCR2.

Embodiment 132. An inhibitor of Monocyte Chemoattractant Protein-1 (CCL2) activity for use of any one of Embodiments 1 to 131, wherein the inhibitor of CCL2 activity is an L-nucleic acid, preferably binding to MCP-1, selected from the group comprising type 1A nucleic acids, type 1B nucleic acids, type 2 nucleic acids, type 3 nucleic acids, type 4 nucleic acids and nucleic acids having a nucleic acid sequence according to any of SEQ.ID.No. 87 to 115.

Embodiment 133. The inhibitor of Monocyte Chemoattractant Protein-1 (CCL2) activity for use of Embodiment 132, wherein the type 1A nucleic acid comprises in 5'->3' direction a first stretch Box B1A, a second stretch Box B2, a third stretch Box B3, a fourth stretch Box B4, a fifth stretch Box B5, a sixth stretch Box B6 and a seventh stretch Box B1B, wherein
the first stretch Box B1A and the seventh stretch Box B1B optionally hybridize with each other, wherein upon hybridization a double-stranded structure is formed,
the first stretch Box B1A comprises a nucleotide sequence of AGCRUG,
the second stretch Box B2 comprises a nucleotide sequence of CCCGGW,
the third stretch Box B3 comprises a nucleotide sequence of GUR,
the fourth stretch Box B4 comprises a nucleotide sequence of RYA,
the fifth stretch Box B5 comprises a nucleotide sequence of GGGGGRCGCGAYC
the sixth stretch Box B6 comprises a nucleotide sequence of UGCAAUAAUG or URYAWUUG, and
the seventh stretch Box B1B comprises a nucleotide sequence of CRYGCU.

Embodiment 134. The inhibitor of Monocyte Chemoattractant Protein-1 (CCL2) activity for use of Embodiment 133, wherein the first stretch Box B1A comprises a nucleotide sequence of AGCGUG.

Embodiment 135. The inhibitor of Monocyte Chemoattractant Protein-1 (CCL2) activity for use of Embodiments 133 or 134, wherein the second stretch Box B2 comprises a nucleotide sequence of CCCGGU.

Embodiment 136. The inhibitor of Monocyte Chemoattractant Protein-1 (CCL2) activity for use of any of Embodiments 133 to 135, wherein the third stretch Box B3 comprises a nucleotide sequence of GUG.

Embodiment 137. The inhibitor of Monocyte Chemoattractant Protein-1 (CCL2) activity for use of any of Embodiments 133 to 136, wherein the fourth stretch Box B4 comprises a nucleotide sequence of GUA.

Embodiment 138. The inhibitor of Monocyte Chemoattractant Protein-1 (CCL2) activity for use of any of Embodiments 133 to 137, wherein the fifth stretch Box B5 comprises a nucleotide sequence of GGGGGGCGCGACC.

Embodiment 139. The inhibitor of Monocyte Chemoattractant Protein-1 (CCL2) activity for use of any of Embodiments 133 to 138, wherein the sixth stretch Box B6 comprises a nucleotide sequence of UACAUUUG.

Embodiment 140. The inhibitor of Monocyte Chemoattractant Protein-1 (CCL2) activity for use of any of Embodiments 133 to 139, wherein the seventh stretch Box B1B comprises a nucleotide sequence of CACGCU.

Embodiment 141. The inhibitor of Monocyte Chemoattractant Protein-1 (CCL2) activity for use of any of Embodiments 133 to 140, wherein the nucleic acid comprises a nucleic acid sequence according to SEQ.ID. No 21.

Embodiment 142. The inhibitor of Monocyte Chemoattractant Protein-1 (CCL2) activity for use of Embodiment 132, wherein the type 1B nucleic acid comprises in 5'->3' direction a first stretch Box B1A, a second stretch Box B2, a third stretch Box B3, a fourth stretch Box B4, a fifth stretch Box B5, a sixth stretch Box B6 and a seventh stretch Box B1B, wherein
the first stretch Box B1A and the seventh stretch Box B1B optionally hybridize with each other, wherein upon hybridization a double-stranded structure is formed,
the first stretch Box B1A comprises a nucleotide sequence of AGYRUG,
the second stretch Box B2 comprises a nucleotide sequence of CCAGCU or CCAGY,
the third stretch Box B3 comprises a nucleotide sequence of GUG,
the fourth stretch Box B4 comprises a nucleotide sequence of AUG,
the fifth stretch Box B5 comprises a nucleotide sequence of GGGGGGCGCGACC
the sixth stretch Box B6 comprises a nucleotide sequence of CAUUUUA or CAUUUA, and
the seventh stretch Box B1B comprises a nucleotide sequence of CAYRCU.

Embodiment 143. The inhibitor of Monocyte Chemoattractant Protein-1 (CCL2) activity for use of Embodiment 142, wherein the first stretch Box B1A comprises a nucleotide sequence of AGCGUG.

Embodiment 144. The inhibitor of Monocyte Chemoattractant Protein-1 (CCL2) activity for use of Embodiments 142 or 43, wherein the second stretch Box B2 comprises a nucleotide sequence of CCAGU.

Embodiment 145. The inhibitor of Monocyte Chemoattractant Protein-1 (CCL2) activity for use of any of Embodiments 142 to 144, wherein the sixth stretch Box B6 comprises a nucleotide sequence of CAUUUUA.

Embodiment 146. The inhibitor of Monocyte Chemoattractant Protein-1 (CCL2) activity for use of any of Embodiments 142 to 145, wherein the seventh stretch Box B1B comprises a nucleotide sequence of CACGCU.

Embodiment 147. The inhibitor of Monocyte Chemoattractant Protein-1 (CCL2) activity for use of any of Embodiments 142 to 146, wherein the nucleic acid comprises a nucleic acid sequence according to SEQ.ID.No 28 and SEQ.ID.No 27.

Embodiment 148. The inhibitor of Monocyte Chemoattractant Protein-1 (CCL2) activity for use of Embodiment 132, wherein the type 2 nucleic acid comprises in 5'->3' direction a first stretch Box B1A, a second stretch Box B2, and a third stretch Box B1B, wherein
the first stretch Box B1A and the third stretch Box B1B optionally hybridize with each other, wherein upon hybridization a double-stranded structure is formed,
the first stretch Box B1A comprises a nucleotide sequence selected from the group comprising ACGCA, CGCA and GCA,
the second stretch Box B2 comprises a nucleotide sequence of CSUCCCUCACCGGUGCAAGUGAAGCCGYGGCUC, and
the third stretch Box B1B comprises a nucleotide sequence selected from the group comprising UGCGU, UGCG and UGC.

Embodiment 149. The inhibitor of Monocyte Chemoattractant Protein-1 (CCL2) activity for use of Embodiment 148, wherein the second stretch Box B2 comprises a nucleotide sequence of CGUCCCUCACCGGUGCAAGUGAAGCCGUGGCUC.

Embodiment 150. The inhibitor of Monocyte Chemoattractant Protein-1 (CCL2) activity for use of any of Embodiments 148 to 149, wherein
a) the first stretch Box B1A comprises a nucleotide sequence of ACGCA,
   and
   the third stretch Box B1B comprises a nucleotide sequence of UGCGU; or
b) the first stretch Box B1A comprises a nucleotide sequence of CGCA,
   and
   the third stretch Box B1B comprises a nucleotide sequence of UGCG; or
c) the first stretch Box B1A comprises a nucleotide sequence of GCA,
   and
   the third stretch Box B1B comprises a nucleotide sequence of UGC or UGCG.

Embodiment 151. The inhibitor of Monocyte Chemoattractant Protein-1 (CCL2) activity for use of any of Embodiments 148 to 150, wherein the first stretch Box B1A comprises a nucleotide sequence of GCA.

Embodiment 152. The inhibitor of Monocyte Chemoattractant Protein-1 (CCL2) activity for use of any of Embodiments 148 to 151 and preferably Embodiment 151, wherein the third stretch Box B1B comprises a nucleotide sequence of UGCG.

Embodiment 153. The inhibitor of Monocyte Chemoattractant Protein-1 (CCL2) activity for use of any of Embodiments 148 to 152, wherein the nucleic acid comprises a nucleic acid sequence according to SEQ.ID.No 37, SEQ.ID.No 116, SEQ.ID.No 117 and SEQ.ID.No 278.

Embodiment 154. The inhibitor of Monocyte Chemoattractant Protein-1 (CCL2) activity for use of Embodiment 132 wherein the type 3 nucleic acid comprises in 5'->3' direction a first stretch Box B1A, a second stretch Box B2A, a third stretch Box B3, a fourth stretch Box B2B, a fifth stretch Box B4, a sixth stretch Box B5A, a seventh stretch Box B6, an eighth stretch Box B5B and a ninth stretch Box B1B, wherein
the first stretch Box B1A and the ninth stretch Box B1B optionally hybridize with each other, wherein upon hybridization a double-stranded structure is formed,
the second stretch Box B2A and the fourth Box B2B optionally hybridize with each other, wherein upon hybridization a double-stranded structure is formed,
the sixth stretch Box B5A and the eighth Box B5B optionally hybridize with each other, wherein upon hybridization a double-stranded structure is formed,
the first stretch Box B1A comprises a nucleotide sequence which is selected from the group comprising GURCUGC, GKSYGC, KBBSC and BNGC,
the second stretch Box B2A comprises a nucleotide sequence of GKMGU,
the third stretch Box B3 comprises a nucleotide sequence of KRRAR,
the fourth stretch Box B2B comprises a nucleotide sequence of ACKMC,
the fifth stretch Box B4 comprises a nucleotide sequence selected from the group comprising CURYGA, CUWAUGA, CWRMGACW and UGCCAGUG,
the sixth stretch Box B5A comprises a nucleotide sequence selected from the group comprising GGY and CWGC,
the seventh stretch Box B6 comprises a nucleotide sequence selected from the group comprising YAGA, CKAAU and CCUUUAU,
the eighth stretch Box B5B comprises a nucleotide sequence selected from the group comprising GCYR and GCWG, and
the ninth stretch Box B1B comprises a nucleotide sequence selected from the group comprising GCAGCAC, GCRSMC, GSVVM and GCNV.

Embodiment 155. The inhibitor of Monocyte Chemoattractant Protein-1 (CCL2) activity for use of Embodiment 154, wherein the third stretch Box B3 comprises a nucleotide sequence of GAGAA or UAAAA

Embodiment 156. The inhibitor of Monocyte Chemoattractant Protein-1 (CCL2) activity for use of Embodiments 154 or 155, wherein the fifth stretch Box B4 comprises a nucleotide sequence of CAGCGACU or CAACGACU.

Embodiment 157. The inhibitor of Monocyte Chemoattractant Protein-1 (CCL2) activity for use of any of Embodiments 154 to 156, wherein the fifth stretch Box B4 comprises a nucleotide sequence of CAGCGACU and Box B3 comprises a nucleotide sequence of UAAAA.

Embodiment 158. The inhibitor of Monocyte Chemoattractant Protein-1 (CCL2) activity for use of any of Embodiments 154 to 157, wherein the fifth stretch Box B4 comprises a nucleotide sequence of CAACGACU and the third stretch Box B3 comprises a nucleotide sequence of GAGAA.

Embodiment 159. The inhibitor of Monocyte Chemoattractant Protein-1 (CCL2) activity for use of any of Embodiments 154 to 158, wherein the seventh stretch Box B6 comprises a nucleotide sequence of UAGA.

Embodiment 160. The inhibitor of Monocyte Chemoattractant Protein-1 (CCL2) activity for use of any of Embodiments 154 to 159, wherein
a) the first stretch Box B1A comprises a nucleotide sequence of GURCUGC,
   and
   the ninth stretch Box B1B comprises a nucleotide sequence of GCAGCAC; or
b) the first stretch Box B1A comprises a nucleotide sequence of GKSYGC,
   and
   the ninth stretch Box B1B comprises a nucleotide sequence of GCRSMC; or
c) the first stretch Box B1A comprises a nucleotide sequence of KBBSC,
   and
   the ninth stretch Box B1B comprises a nucleotide sequence of GSVVM; or
d) the first stretch Box B1A comprises a nucleotide sequence of BNGC,
   and
   the ninth stretch Box B1B comprises a nucleotide sequence of GCNV.

Embodiment 161. The inhibitor of Monocyte Chemoattractant Protein-1 (CCL2) activity for use of Embodiment 156, wherein
a) the first stretch Box B1A comprises a nucleotide sequence of GUGCUGC,
   and
   the ninth stretch Box B1B comprises a nucleotide sequence of GCAGCAC; or
b) the first stretch Box B1A comprises a nucleotide sequence of GUGCGC,
   and
   the ninth stretch Box B1B comprises a nucleotide sequence of GCGCAC; or
c) the first stretch Box B1A comprises a nucleotide sequence of KKSSC,
   and
   the ninth stretch Box B1B comprises a nucleotide sequence of GSSMM; or
d) the first stretch Box B1A comprises a nucleotide sequence of SNGC,
   and
   the ninth stretch Box B1B comprises a nucleotide sequence of GCNS.

Embodiment 162. The inhibitor of Monocyte Chemoattractant Protein-1 (CCL2) activity for use of Embodiment 161, wherein
the first stretch Box B1A comprises a nucleotide sequence of GGGC,
   and
the ninth stretch Box B1B comprises a nucleotide sequence of GCCC.

Embodiment 163. The inhibitor of Monocyte Chemoattractant Protein-1 (CCL2) activity for use of any of Embodiments 154 to 162, wherein the second stretch Box B2A comprises a nucleotide sequence of GKMGU and the fourth stretch Box B2B comprises a nucleotide sequence of ACKMC.

Embodiment 164. The inhibitor of Monocyte Chemoattractant Protein-1 (CCL2) activity for use of Embodiment 163, wherein the second stretch Box B2A comprises a nucleotide sequence of GUAGU and the fourth stretch Box B2B comprises a nucleotide sequence of ACUAC.

Embodiment 165. The inhibitor of Monocyte Chemoattractant Protein-1 (CCL2) activity for use of any of Embodiments 154 to 164, wherein
a) the sixth stretch Box B5A comprises a nucleotide sequence of GGY,
   and
   the eighth stretch Box B5B comprises a nucleotide sequence of GCYR; or
b) the sixth stretch Box B5A comprises a nucleotide sequence of CWGC,
   and
   the eighth stretch Box B5B comprises a nucleotide sequence of GCWG.

Embodiment 166. The inhibitor of Monocyte Chemoattractant Protein-1 (CCL2) activity for use of Embodiment 165, wherein
the sixth stretch Box B5A comprises a nucleotide sequence of GGC,
   and
the eighth stretch Box B5B comprises a nucleotide sequence of GCCG.

Embodiment 167. The inhibitor of Monocyte Chemoattractant Protein-1 (CCL2) activity for use of any of Embodiments 154 to 166, wherein the sixth stretch Box B5A hybridizes with the nucleotides GCY of the eighth stretch Box B5B.

Embodiment 168. The inhibitor of Monocyte Chemoattractant Protein-1 (CCL2) activity for use of any of Embodiments 154 to 157 and 159 to 167, wherein the nucleic acid comprises a nucleic acid sequence according to SEQ.ID.No 56.

Embodiment 169. The inhibitor of Monocyte Chemoattractant Protein-1 (CCL2) activity for use of any of Embodiments 154 to 156 and 159 to 167, wherein the nucleic acid comprises a nucleic acid sequence selected from the group comprising the nucleic acid sequences according to SEQ.ID.No 57 to 61, SEQ.ID.No 67 to 71 and SEQ.ID.No 73.

Embodiment 170. The inhibitor of Monocyte Chemoattractant Protein-1 (CCL2) activity for use of Embodiment 132, wherein the type 4 nucleic acid comprises in 5'->3' direction a first stretch Box B1A, a second stretch Box B2, a third stretch Box B1B wherein
the first stretch Box B1A and the third stretch Box B1B optionally hybridize with each other, wherein upon hybridization a double-stranded structure is formed,
the first stretch Box B1A comprises a nucleotide sequence selected from the group comprising AGCGUGDU, GCGCGAG, CSKSUU, GUGUU, and UGUU;
the second stretch Box B2 comprises a nucleotide sequence selected from the group comprising AGNDRDGBKGGURGYARGUAAAG, AGGUGGGUGGUAGUAAGUAAAG and CAGGUGGGUGGUAGAAUGUAAAGA, and
the third stretch Box B1B comprises a nucleotide sequence selected from the group comprising GNCASGCU, CUCGCGUC, GRSMSG, GRCAC, and GGCA.

Embodiment 171. The inhibitor of Monocyte Chemoattractant Protein-1 (CCL2) activity for use of Embodiment 170, wherein
a) the first stretch Box B1A comprises a nucleotide sequence of GUGUU,
   and
   the third stretch Box B1B comprises a nucleotide sequence of GRCAC;
b) the first stretch Box B1A comprises a nucleotide sequence of GCGCGAG,
   and
   the third stretch Box B1B comprises a nucleotide sequence of CUCGCGUC; or
c) the first stretch Box B1A comprises a nucleotide sequence of CSKSUU,
   and
   the third stretch Box B1B comprises a nucleotide sequence of GRSMSG, or
d) the first stretch Box B1A comprises a nucleotide sequence of UGUU,
   and
   the third stretch Box B1B comprises a nucleotide sequence of GGCA, or
e) the first stretch Box B1A comprises a nucleotide sequence of AGCGUGDU,
   and
   the third stretch Box B1B comprises a nucleotide sequence of GNCASGCU.

Embodiment 172. The inhibitor of Monocyte Chemoattractant Protein-1 (CCL2) activity for use of Embodiment 171, wherein the first stretch Box B1A comprises a nucleotide sequence of CSKSUU and the third stretch Box B1B comprises a nucleotide sequence of GRSMSG.

Embodiment 173. The inhibitor of Monocyte Chemoattractant Protein-1 (CCL2) activity for use of Embodiments 172, wherein the first stretch Box B1A comprises a nucleotide sequence of CCGCUU and the third stretch Box B1B comprises a nucleotide sequence of GGGCGG.

Embodiment 174. The inhibitor of Monocyte Chemoattractant Protein-1 (CCL2) activity for use of any of Embodiments 170 to 173, wherein
the second stretch Box B2 comprises a nucleotide sequence of AGGUGGGUGGUAGUAAGUAAAG.

Embodiment 175. The inhibitor of Monocyte Chemoattractant Protein-1 (CCL2) activity for use of any of Embodiments 170 to 174, wherein the nucleic acid comprises a nucleic acid sequence according to SEQ.ID.No 80.

Embodiment 176. The inhibitor of Monocyte Chemoattractant Protein-1 (CCL2) activity for use of any of Embodiments 132 to 175, wherein the nucleic acid is capable of binding human MCP-1.

Embodiment 177. The inhibitor of Monocyte Chemoattractant Protein-1 (CCL2) activity for use of any of Embodiments 132 to 176, preferably Embodiment 176, wherein the MCP-1 has an amino acid sequence according to SEQ ID No. 1.

Embodiment 178. The inhibitor of Monocyte Chemoattractant Protein-1 (CCL2) activity for use of any of Embodiments 132 to 177, wherein the nucleic acid comprises a modification, wherein the modification is preferably a high molecular weight moiety and/or wherein the modification preferably allows to modify the characteristics of the nucleic acid according to any of Embodiments 1 to 46 in terms of residence time in the animal or human body, preferably the human body.

Embodiment 179. The inhibitor of Monocyte Chemoattractant Protein-1 (CCL2) activity for use of Embodiment 178, wherein the modification is selected from the group comprising a HES moiety and a PEG moiety.

Embodiment 180. The inhibitor of Monocyte Chemoattractant Protein-1 (CCL2) activity for use of Embodiment 179, wherein the modification is a PEG moiety consisting of a straight or branched PEG, wherein the molecular weight of the PEG moiety is preferably from about 20 to 120 kD, more preferably from about 30 to 80 kD and most preferably about 40 kD.

Embodiment 181. The inhibitor of Monocyte Chemoattractant Protein-1 (CCL2) activity for use of Embodiment 179, wherein the modification is a HES moiety, wherein preferably the molecular weight of the HES moiety is from about 10 to 130 kD, more preferably from about 30 to 130 kD and most preferably about 100 kD.

Embodiment 182. The inhibitor of Monocyte Chemoattractant Protein-1 (CCL2) activity for use of any of Embodiments of 178 to 181, wherein the modification is coupled to the nucleic acid via a linker.

Embodiment 183. The inhibitor of Monocyte Chemoattractant Protein-1 (CCL2) activity for use of any of Embodiments of 181 to 182, wherein the modification is coupled to the nucleic acid at its 5'-terminal nucleotide and/or its 3'-terminal nucleotide and/or to a nucleotide of the nucleic acid between the 5'-terminal nucleotide and the 3'-terminal nucleotide.

Embodiment 184. The inhibitor of Monocyte Chemoattractant Protein-1 (CCL2) activity for use of any of Embodiments 132 to 183, wherein the nucleotides of or the nucleotides forming the nucleic acid are L-nucleotides.

Embodiment 185. The inhibitor of Monocyte Chemoattractant Protein-1 (CCL2) activity for use of any of Embodiments 132 to 184, wherein the moiety of the nucleic acid capable of binding MCP-1 consists of L-nucleotides.

Embodiment 186. The inhibitor of Monocyte Chemoattractant Protein-1 (CCL2) activity for use of any one of Embodiments 132, 148 to 152 and 176 to 185, wherein the type 2 nucleic acid comprises in 5'->3' direction a first stretch Box B1A, a second stretch Box B2, and a third stretch Box B1B, wherein
the first stretch Box B1A comprises a nucleotide sequence selected from the group comprising ACGCA, CGCA and GCA,
the second stretch Box B2 comprises a nucleotide sequence of CSUCCCUCACCGGUGCAAGUGAAGCCGYGGCUC, and
the third stretch Box B1B comprises a nucleotide sequence selected from the group comprising UGCGU, UGCG and UGC.

Embodiment 187. The inhibitor of Monocyte Chemoattractant Protein-1 (CCL2) activity for use of any one of Embodiments 132, 148 to 153 and 176 to 186, wherein the nucleic acid comprises a nucleotide sequence of SEQ IQ NO: 37 or a nucleotide sequence having an identity of at least 85 % to the nucleotide sequence of SEQ ID NO: 37.

Embodiment 188. The inhibitor of Monocyte Chemoattractant Protein-1 (CCL2) activity for use of any one of Embodiments 132, 148 to 153 and 176 to 187, wherein the nucleic acid comprises a nucleotide sequence of SEQ ID NO: 37.

Embodiment 189. The inhibitor of Monocyte Chemoattractant Protein-1 (CCL2) activity for use any one of Embodiments 186 to 188, wherein the nucleic acid comprises a modification.

Embodiment 190. The inhibitor of Monocyte Chemoattractant Protein-1 (CCL2) activity for use of Embodiment 189, wherein the modification is coupled to the nucleic acid via a linker.

Embodiment 191. The inhibitor of Monocyte Chemoattractant Protein-1 (CCL2) activity for use of any one of Embodiments 189 to 190, wherein the modification comprises a polyethylene glycol (PEG) molecule.

Embodiment 192. The inhibitor of Monocyte Chemoattractant Protein-1 (CCL2) activity for use of Embodiments 191, wherein the PEG molecule is at the 5' terminus of the nucleic acid.

Embodiment 193. The inhibitor of Monocyte Chemoattractant Protein-1 (CCL2) activity for use of Embodiment 191, wherein the PEG molecule is at the 3' terminus of the nucleic acid.

Embodiment 194. The inhibitor of Monocyte Chemoattractant Protein-1 (CCL2) activity for use of any one of Embodiments 191 to 193, wherein the PEG molecule is straight chain or branched.

Embodiment 195. The inhibitor of Monocyte Chemoattractant Protein-1 (CCL2) activity for use of any one of Embodiments 191 to 194, wherein the PEG molecule has a molecular weight from about 20 kDa to about 120 kDa, preferably from about 30 kDa to about 80 kDa.

Embodiment 196. The inhibitor of Monocyte Chemoattractant Protein-1 (CCL2) activity for use of any one of Embodiments 191 to 195, wherein the PEG molecule has a molecular weight of about 40 kDa.

Embodiment 197. The inhibitor of Monocyte Chemoattractant Protein-1 (CCL2) activity for use of any one of Embodiments 191 to 196, wherein the PEG is a polydisperse PEG or a monodisperse PEG.

Embodiment 198. The inhibitor of Monocyte Chemoattractant Protein-1 (CCL2) activity for use of any one of Embodiments 1 to 197, wherein the inhibitor of CCL2 activity is compound NOX-E36.

Embodiment 199. The inhibitor of Monocyte Chemoattractant Protein-1 (CCL2) activity for use of any one of Embodiments 1 to 197, wherein the inhibitor of CCL2 activity is selected from the group comprising a compound targeting CCL2, wherein the compound is selected from the group comprising Bindarit, a small molecule CCL2 inhibitor; Carlumab (CNTO888), a fully-human anti-CCL2 Mab; ABN-912, a fully-human anti-CCL2 Mab; CGEN-54 and a recombinant CCL2-inhibiting protein.

Embodiment 200. The inhibitor of Monocyte Chemoattractant Protein-1 (CCL2) activity for use of any one of Embodiments 1 to 198, wherein the inhibitor of CCL2 activity is selected from the group comprising a compound targeting CCR2, wherein the compound is selected from the group comprising AZ-889, a small molecule CCR2 antagonist; AZD-2423 a small molecule CCR2 antagonist, a small molecule CCR2 antagonist; BL-2030, a soluble CCR2 receptor fused to a human antibody Fc component; BMS-741672, a small molecule CCR2 antagonist; BMS-753426, a small molecule CCR2 antagonist; BMS-813160, a small molecule CCR2 antagonist; CCX-140, a small molecule CCR2 inhibitor; CCX-598, a small molecule CCR2 inhibitor; CCX-872, a small molecule CCR2 inhibitor; CCX-915, a small molecule CCR2 inhibitor; cenicriviroc, a small molecule CCR2/CCR5 antagonist; CNTX-6970, a small molecule CCR2 antagonist; CPD-B, a small molecule CCR2 antagonist; EPX-102216, a small molecule CCR2 antagonist; INCB-3344, a small molecule CCR2 inhibitor; INCB-3284, a small molecule CCR2 inhibitor; INCB-8696, a small molecule CCR2 inhibitor; LF-0376, a small molecule CCR2/5 antagonist; MK-812, a small molecule CCR2 inhibitor; NIBR-6465, a small molecule CCR2/5 antagonist; OB-004 a small molecule CCR2 antagonist; OPL-CCL2-LPM, a human CCL2 chemokine fusion protein with cytotoxic payload; PD-172084, a small molecule CCR2 antagonist; PF-04634817, a small molecule CCR2/5 antagonist; PF-4136309, a small molecule CCR2 antagonist; Plozalizumab (MLN-1202), a humanized anti-CCR2 mAb; R-103, an oral Dala1-peptide T-amide analogue targeting CCR2, 5 and 8; RAP-103; RAP-310, both small molecule CCR2/5 antagonists; SB-380732, a small molecule CCR2 antagonist; SPR-3, a small molecule CCR2 antagonist; STI-B0201, a fully human anti-CCR2 mAb; STI-B0211, a fully human anti-CCR2 mAb; STI-B0221, a fully human anti-CCR2 mAb; STI-B0234, a fully human anti-CCR2 mAb; TAK-779, a small molecule CCR2/5 antagonist; and TLK-19705, small molecule CCR2 antagonist.

The problem underlying the present invention is solved in a first aspect, by an inhibitor of Monocyte Chemoattractant Protein-1 (CCL2) activity, for use in a method for treating a subject suffering from or being at risk of suffering from an eye disease, wherein the eye disease is glaucoma and wherein the inhibitor of CCL2 activity is locally and systemically administered to the subject. Without wishing to be bound by any theory, the present inventors have surprisingly found that the combined local, preferably subconjunctival, and systemic, preferably subcutaneous administration of an inhibitor of activity CCL2 is more effective than subconjunctival administration of an inhibitor of CCL2 activity alone. Such increase in efficacy is an increase in inhibition of fibrosis in an eye of a subject which had been or will be subject to glaucoma filtration surgery.

The problem underlying the present invention is solved in a second aspect by an inhibitor of Monocyte Chemoattractant Protein-1 (CCL2) activity, for use in a method for treating a subject post-surgery for reducing intraocular pressure, preferably post glaucoma filtration surgery or post minimally invasive glaucoma surgery, wherein the inhibitor of CCL2 activity is locally and systemically administered to the subject.

It will be appreciated by a person skilled in the art that such use of the inhibitor of CCL2 activity according to the second aspect intends to improve and actually improves the health condition of the subject and more specifically the health condition of the eye which was subject to a surgery for reducing intraocular pressure, wherein such surgery for reducing intraocular pressure is, e.g., either glaucoma filtration surgery of minimally invasive glaucoma surgery, preferably glaucoma filtration surgery.

The problem underlying the present invention is solved in a third aspect by an inhibitor of Monocyte Chemoattractant Protein-1 (CCL2) activity, for use in a method for treating a subject post glaucoma filtration surgery of an eye of the subject, wherein the method comprises administering the inhibitor of CCL2 activity to the subject for maintaining functionality of vasculature of a bleb resulting from glaucoma filtration surgery or for avoiding or decreasing deterioration of vasculature of a bleb resulting from glaucoma filtration surgery.

It will be appreciated by a person skilled in the art that such use of the inhibitor of CCL2 activity according to the third aspect maintains functionality of vasculature of a bleb resulting from glaucoma filtration surgery and, respectively, avoids or decreases deterioration of vasculature of a bleb resulting from glaucoma filtration surgery, whereby there is no toxic effect to the vasculature of the bleb and its functionality arising from such treating of the subject post glaucoma filtration surgery of an eye of the subject. In a preferred embodiment, the toxic effect would result in a thin, avascular bleb and/or a leakage of the bleb.

In particular with this third aspect, it is important to note that the inhibitor of CCL2 activity, preferable the L-nucleic acid molecules binding to CCL2 disclosed herein and also described, for example, in WO 2007/093409, more preferably compound NOX-E36, are effective in being antifibrotic while not deteriorating the vasculature of the bleb as seen, for example, when using mitomycin C or 5-fluorouracil upon administration on the site of glaucoma filtration surgery. Typically, such deterioration of the vasculature of the bleb goes along with or results in a thin, avascular bleb which is typically prone to leakage.

The problem underlying the present invention is solved in a fourth aspect by an inhibitor of Monocyte Chemoattractant Protein-1 (also referred to as CCL2 or MCP-1) activity for use in a method for avoiding or decreasing deterioration of vasculature of a bleb, preferably a bleb resulting from glaucoma filtration surgery, wherein the method comprises administering the inhibitor of CCL2 activity to the subject.

The problem underlying the present invention is solved in a fifth aspect by an inhibitor of Monocyte Chemoattractant Protein-1 (also referred to as CCL2 or MCP-1) activity, for use in a method for treating a subject suffering from or being at risk of suffering from glaucoma, wherein the method avoids degeneration of vasculature of an eye of the subject or is for avoiding degeneration of vasculature of an eye of the subject, wherein the method comprises administering the inhibitor of CCL2 activity to the subject.

The problem underlying the present invention is solved in a sixth aspect by a method for treating a subject suffering from or being at risk of suffering from an eye disease, wherein the eye disease is glaucoma and wherein the inhibitor of Monocyte Chemoattractant Protein-1 (CCL2) activity is locally and systemically administered to the subject.

The problem underlying the present invention is solved in a seventh aspect by the use of an inhibitor of Monocyte Chemoattractant Protein-1 (CCL2) activity in the manufacture of a medicament for the treatment or prevention of an eye disease, wherein the eye disease is glaucoma and wherein the inhibitor of CCL2 activity is for local and systemic administration. In an embodiment, the medicament comprises the inhibitor of CCL2 activity suitable for local administration and the inhibitor of CCL2 activity suitable for systemic administration. In an embodiment thereof, the inhibitor of CCL2 activity for local administration is formulated in a first formulation and the inhibitor of CCL2 activity for systemic administration is formulated in a second formulation. In an embodiment, the first formulation and the second formulation are the same. In an alternative embodiment, the first formulation and the second formulation are different.

The problem underlying the present invention is solved in an eighth aspect by the use of an inhibitor of Monocyte Chemoattractant Protein-1 (CCL2) activity in the manufacture of a medicament for the treatment or prevention of an eye disease, wherein the eye disease is glaucoma and wherein the inhibitor of CCL2 activity is for local administration, preferably formulated for local administration, wherein the medicament is for use together with an inhibitor of Monocyte Chemoattractant Protein-1 (CCL2) activity for systemic administration, preferably formulated for systemic administration.

The problem underlying the present invention is solved in a ninth aspect by the use of an inhibitor of Monocyte Chemoattractant Protein-1 (CCL2) activity in the manufacture of a medicament for the treatment or prevention of an eye disease, wherein the eye disease is glaucoma and wherein the inhibitor of CCL2 activity is for systemic administration, preferably formulated for systemic administration, wherein the medicament is for use together with an inhibitor of Monocyte Chemoattractant Protein-1 (CCL2) activity for local administration, preferably formulated for local administration.

It will be appreciated by a person skilled in the art that the first, seventh, eighth and ninth aspect are related and that each and any embodiment of one of these aspects, including any embodiment of these aspect, will also be an embodiment of the other of these aspects, including any embodiments of the other of these aspects.

The problem underlying the present invention is solved in a tenth aspect by a method for treating a subject post (or after) surgery for reducing intraocular pressure, preferably post glaucoma filtration surgery or post minimally invasive glaucoma surgery, wherein an inhibitor of Monocyte Chemoattractant Protein-1 (CCL2) activity is locally and systemically administered to the subject.

The problem underlying the present invention is solved in an eleventh aspect by the use of an inhibitor of Monocyte Chemoattractant Protein-1 (CCL2) activity in the manufacture of a medicament for use after surgery for reducing intraocular pressure, preferably post glaucoma filtration surgery or post minimally invasive glaucoma surgery, wherein the inhibitor of CCL2 activity is for local and systemic administration. Preferably, the medicament is for preventing fibrosis or reducing fibrosis in or at the eye which was subject to said surgery for reducing intraocular pressure. In an embodiment, the medicament comprises the inhibitor of CCL2 activity suitable for local administration and the inhibitor of CCL2 activity suitable for systemic administration. In an embodiment thereof, the inhibitor of CCL2 activity for local administration is formulated in a first formulation and the inhibitor of CCL2 activity for systemic administration is formulated in a second formulation. In an embodiment, the first formulation and the second formulation are the same. In an alternative embodiment, the first formulation and the second formulation are different.

The problem underlying the present invention is solved in a twelfth aspect by the use of an inhibitor of Monocyte Chemoattractant Protein-1 (CCL2) activity in the manufacture of a medicament for use after surgery for reducing intraocular pressure, preferably post glaucoma filtration surgery or post minimally invasive glaucoma surgery, wherein the inhibitor of CCL2 activity is for local administration, preferably formulated for local administration, wherein the medicament is for use together with an inhibitor of Monocyte Chemoattractant Protein-1 (CCL2) activity for systemic administration, preferably formulated for systemic administration.

The problem underlying the present invention is solved in a 13^{th} aspect by the use of an inhibitor of Monocyte Chemoattractant Protein-1 (CCL2) activity in the manufacture of a medicament for use after surgery for reducing intraocular pressure, preferably post glaucoma filtration surgery or post minimally invasive glaucoma surgery, wherein the inhibitor of CCL2 activity is for systemic administration, preferably formulated for systemic administration, wherein the medicament is for use together with an inhibitor of Monocyte Chemoattractant Protein-1 (CCL2) activity for local administration, preferably formulated for local administration.

It will be appreciated by a person skilled in the art that the second, tenth, eleventh, twelfth and 13^{th} aspect are related and that each and any embodiment of one of these aspects, including any embodiment of these aspect, will also be an embodiment of the other of these aspects, including any embodiments of the other of these aspects.

The problem underlying the present invention is solved in an 14^{th} aspect by a method for treating a subject post glaucoma filtration surgery of an eye of the subject, wherein the method comprises administering the inhibitor of Monocyte Chemoattractant Protein-1 (CCL2) activity to the subject for maintaining functionality of vasculature of a bleb, preferably resulting from glaucoma filtration surgery or for avoiding or decreasing deterioration of vasculature of a bleb resulting from glaucoma filtration surgery.

The problem underlying the present invention is solved in a 15^{th} aspect by the use of an inhibitor of Monocyte Chemoattractant Protein-1 (CCL2) activity in the manufacture of a medicament for the treatment of a subject post glaucoma filtration surgery of an eye of the subject, preferably for maintaining functionality of vasculature of a bleb resulting from glaucoma filtration surgery or for avoiding or decreasing deterioration of vasculature of a bleb resulting from glaucoma filtration surgery.

The problem underlying the present invention is solved in a 16^{th} aspect by the use of an inhibitor of Monocyte Chemoattractant Protein-1 (CCL2) activity in the manufacture of a medicament for the treatment of a subject post glaucoma filtration surgery of an eye of the subject, preferably for maintaining functionality of vasculature of a bleb resulting from glaucoma filtration surgery or for avoiding or decreasing deterioration of vasculature of a bleb resulting from glaucoma filtration surgery, wherein the inhibitor of CCL2 activity is for local administration, preferably the inhibitor of CCL2 activity is formulated for local administration.

The problem underlying the present invention is solved in a 17^{st} aspect by the use of an inhibitor of Monocyte Chemoattractant Protein-1 (CCL2) activity in the manufacture of a medicament for the treatment of a subject post glaucoma filtration surgery of an eye of the subject, preferably for maintaining functionality of vasculature of a bleb resulting from glaucoma filtration surgery or for avoiding or decreasing deterioration of vasculature of a bleb resulting from glaucoma filtration surgery, wherein the inhibitor of CCL2 activity is for systemic administration, preferably the inhibitor of CCL2 activity is formulated for systemic administration.

It will be appreciated by a person skilled in the art that the third, 14^{th}, 15^{th}, 16^{th} and 17^{th} aspect are related and that each and any embodiment of one of these aspects, including any embodiment of these aspect, will also be an embodiment of the other of these aspects, including any embodiments of the other of these aspects.

The problem underlying the present invention is solved in an 18^{th} aspect by a method for avoiding or decreasing deterioration of vasculature of a bleb, preferably a bleb resulting from glaucoma filtration surgery, wherein the method comprises administering the inhibitor of CCL2 activity to the subject.

The problem underlying the present invention is solved in an 19^{th} aspect by the use of an inhibitor of Monocyte Chemoattractant Protein-1 (CCL2) activity in the manufacture of a medicament for the treating vasculature of a bleb, preferably a bleb resulting from glaucoma filtration surgery or prevention, or for preventing vasculature of a bleb, preferably a bleb resulting from glaucoma filtration surgery. Preferably, the inhibitor of CCL2 activity is for local administration, more preferably, the inhibitor of CCL2 activity is formulated for local administration. Alternatively, the inhibitor of CCL2 activity is for systemic administration, more preferably, the inhibitor of CCL2 activity is formulated for systemic administration.

It will be appreciated by a person skilled in the art that the fourth, 18^{th} and 19^{th} aspect are related and that each and any embodiment of one of these aspects, including any embodiment of these aspect, will also be an embodiment of the other of these aspects, including any embodiments of the other of these aspects.

The problem underlying the present invention is solved in 20^{th} aspect by a method for treating a subject suffering from or being at risk of suffering from glaucoma, wherein the method avoids degeneration of vasculature of an eye of the subject or is for avoiding degeneration of vasculature of an eye of the subject, wherein the method comprises administering the an inhibitor of Monocyte Chemoattractant Protein-1 (CCL2) activity to the subject.

The problem underlying the present invention is solved in a 21^{st} aspect by the use of an inhibitor of Monocyte Chemoattractant Protein-1 (CCL2) activity in the manufacture of a medicament for treating or preventing glaucoma by avoiding degeneration of vasculature of an eye of the subject or by avoiding degeneration of vasculature of an eye of the subject.

It will be appreciated by a person skilled in the art that the fifth, 20^{th} and 21^{st} aspect are related and that each and any embodiment of one of these aspects, including any embodiment of these aspect, will also be an embodiment of the other of these aspects, including any embodiments of the other of these aspects.

Without wishing to be bound by any theory, the present inventors have surprisingly found that the combined local, preferably subconjunctival, and systemic, preferably subcutaneous administration of an inhibitor of activity CCL2 is more effective than subconjunctival administration of an inhibitor of CCL2 activity alone. Such increase in efficacy is an increase in inhibition of fibrosis in an eye of a subject which had been or will be subject to glaucoma filtration surgery.

In addition, and again without wishing to be bound by any theory, the present inventors have surprisingly found that local, preferably subconjunctival, administration of an inhibitor of CCL2 activity as an anti-fibrotic does not deteriorate health and in particular preserves the vasculature of a bleb, preferably a bleb resulting from glaucoma filtration surgery. Similarly, the present inventors have surprisingly found that local, preferably subconjunctival, administration of an inhibitor of CCL2 activity avoids or decreases deterioration of vasculature of a bleb, preferably a bleb resulting from glaucoma filtration surgery.

Bleb leaks have a reported incidence of up to 18% (Edmunds, Thompson et al. 2002) and can be classified as early (< 1 month) or late. Risk factors for early leaks include age, ocular inflammatory disease, a prior history of incisional surgery, long-term use of eye drops, surgical technique, and type of conjunctival flap used (Parrish, Schiffman et al. 2001). Early leaks can cause a variety of problems, including choroidal effusion or hypotony maculopathy (Bitrian, Song et al. 2014, Jamjoom, Osman et al. 2020). The most common complication of late leaks is an increased risk of infection. The conjunctiva and sclera are the main barriers to intraocular infection from indigenous bacteria. A disruption of the conjunctiva allows these bacteria to travel from the ocular surface into the bleb and, ultimately, into the anterior chamber and vitreous. Several studies have shown a higher risk of blebitis and endophthalmitis with bleb leaks (Soltau, Rothman et al. 2000, Sugimoto, Mochizuki et al. 2015).

Finally, and again without wising to be bound by any theory, the present inventors have surprisingly found that local administration, preferably subconjunctival administration of an inhibitor of CCL2 activity is at least as effective in terms of anti-fibrotic effect, as local administration of Mitomycin C (MMC) or 5-fluorouracil (5FU) in the treatment of a subject suffering from glaucoma and having undergone or will be undergoing glaucoma filtration surgery. In this context, efficacy means in particular the reduction of fibrosis at the site of glaucoma filtration surgery while not deteriorating health of and in particular functionality of vasculature of a bleb formed in such glaucoma filtration surgery. In particular, replacing local administration of MMC or 5FU by local administration, preferably subconjunctival administration, of an inhibitor of CCL2 activity reduces damage of the vasculature of a bleb and in particular a bleb which formed in glaucoma filtration surgery.

It will be understood that according to the present invention including each and any aspect thereof, including any embodiment of such each and any aspect, the inhibitor of CCL2 activity is administered to an eye of the subject to be treated, preferably an eye of the subject suffering from glaucoma or an eye of a subject who has undergone or will undergo glaucoma filtration surgery.

Fibrosis is a reaction observed after surgical treatment of glaucoma which affects the success of some therapeutic interventions, including but not limited to glaucoma filtration surgery.

In general, fibrosis is a histopathological definition in which there is permanent deposition of extracellular matrix (ECM) components that include collagen, fibronectin, elastin, and others, as a result of tissue injury. Fibrosis is a stereotypical physiological response aimed at restoring anatomical integrity with minimal or no return of function (Armendariz and Chakravarthy 2024). The functional impairment caused by permanent fibrotic tissue deposition is seen in a spectrum of diseases including interstitial lung disease, end-stage liver and kidney diseases, heart failure and nAMD (Franceschi, Bonafe et al. 2000). Fibrosis is characterized by four stages: inflammatory, proliferative, matrix remodelling and wound quiescence (Singer and Clark 1999). In the proliferative phase, there is formation of provisional matrix that functions as a scaffold for permanent matrix deposition, i.e. a scar or fibrotic tissue.

Surgical treatment of glaucoma decreases aqueous inflow or increases aqueous outflow. The latter can be accomplished by augmenting existing outflow pathways or creating artificial outflow pathways into the subconjunctival or sub-Tenon's space, often referred to as traditional incisional glaucoma filtration surgery (GFS). Micro-invasive glaucoma surgical (MIGS) approaches allow for enhancement of the conventional aqueous outflow pathways through the trabecular meshwork and into Schlemm's canal or alternatively from the anterior chamber into the suprachoroidal space. Traditional incisional glaucoma filtering surgery allows the aqueous humor to drain into the space between the sclera and conjunctiva and/or Tenon's tissues (Shao, Sinha et al. 2023).

Unlike many surgeries, where the goal is to heal tissue with the restoration of normal architecture, traditional incisional glaucoma filtering surgery aims to modulate wound healing. Wound healing modulation allows for continued aqueous egress into the subconjunctival and/or sub-Tenon's space, thereby preventing surgical failure (Conlon, Saheb et al. 2017, Lusthaus and Goldberg 2019). Episcleral and subconjunctival fibrosis remain major impediments to the success of glaucoma filtering surgery. Conjunctival fibrosis at the site of filtration may impede aqueous outflow, leading to inadequate IOP reduction. Surgical failure is associated with poor postoperative IOP control, consequent progression of glaucomatous disc cupping, and visual field loss (Hollo 2017). Fibrosis is therefore a direct correlate of functionality, i.e. IOP control, through the filtering bleb/ fistula between the anterior chamber and the subconjunctival space.

According to the present invention including each and any aspect thereof, including any embodiment of such each and any aspect, the inhibitor of CCL2 activity is administered locally. Such local administration includes but is not limited to subconjunctival administration. In an embodiment, local administration is any form of administration different from systemic administration; preferably local administration is administration close to the eye so that transport of the inhibitor of CCL2 activity to the eye and to a site of glaucoma filtration surgery, if any, occurs through tear fluid or though intraocular transport mechanisms and intraocular fluids. In an embodiment, local administration comprises administration of the inhibitor of CCL2 activity present in or on a structure such as a sponge, where the sponge is applied to the eye and the site of glaucoma filtration surgery in particular.

It will be understood that according to the present invention including each and any aspect thereof, including any embodiment of such each and any aspect, systemic administration is selected from the group comprising subcutaneous administration, intravenous administration and any combination thereof. It will be equally understood that system administration encompasses any administration of an inhibitor of CCL2 activity to a subject where the inhibitor of CCL2 activity is administered to the body of the subject so that the inhibitor of CCL2 activity is transported in the vasculature of the body or the lymphatic system of the body.

It will be understood that according to the present invention including each and any aspect thereof, including any embodiment of such each and any aspect, the subject is suffering from or at risk of suffering from glaucoma, preferably the subject is suffering from glaucoma.

It will be understood that according to the present invention including each and any aspect thereof, including any embodiment of such each and any aspect, that glaucoma is a group of eye diseases that cause damage to the optic nerve, often due to increased intraocular pressure (IOP), which can lead to vision loss and blindness if not treated. When pressure inside the eye becomes too high, it can damage the optic nerve, leading to gradual loss of vision (Weinreb, Aung et al. 2014). In an embodiment, a subject suffering from glaucoma is a subject suffering from an increased intraocular pressure (IOP), i.e. higher than 21 mmHg. An increased IOP is preferably one which is regarded by an ophthalmologist as pathological requiring therapeutic intervention and/or which will ultimately lead to vision loss and blindness if not treated.

There are five main types of glaucoma:
Primary open-angle glaucoma (POAG), the most common form of glaucoma, a chronic, progressive optic neuropathy, that has in common characteristic morphological changes at the optic nerve head and retinal nerve fibre layer in the absence of other ocular disease or congenital anomalies. Progressive retinal ganglion cells death and visual field loss are associated with these changes. POAG accounts for about 70-90% of cases. POAG occurs when the drainage angle between the cornea and iris remains open, but the trabecular meshwork - the drainage system of the eye - becomes less efficient over time, leading to an increase in intraocular pressure. POAG is typically asymptomatic in its early stages. Vision loss is gradual and may not be noticed until significant damage has occurred. POAG is managed with medications (eye drops, oral medications) to reduce intraocular pressure, laser therapy, or surgery in more advanced cases.

Primary angle-closure glaucoma (PACG), a less common but more acute form of glaucoma. It is estimated that around 10-20% of all glaucoma cases globally are PACG which is a significant cause of glaucoma-related blindness, particularly in certain regions of the world as the prevalence of PACG varies widely depending on the population and geographical region. For instance, PACG is more prevalent in Asia, particularly in East and Southeast Asia, including countries like China, India, and Thailand. However, it is much less common in populations of European and African descent. PACG occurs when the drainage angle between the cornea and iris becomes blocked, often suddenly, which causes a rapid increase in intraocular pressure. Symptoms can include sudden eye pain, nausea, vomiting, blurred vision, and halos around lights. It is considered a medical emergency. Treatment requires immediate medical intervention to lower eye pressure, which may include medications, laser therapy, or surgery.

Normal-tension glaucoma, a form of glaucoma where optic nerve damage occurs despite normal intraocular pressure levels. The cause is not fully understood, but it may be due to poor blood flow to the optic nerve. Similar to other types of glaucoma, normal-tension glaucoma often progresses without noticeable symptoms until significant vision loss occurs.

Congenital glaucoma, a rare form of glaucoma that occurs in infants and young children, typically due to abnormal eye development. Symptoms include enlarged eyes, cloudiness of the cornea, and sensitivity to light. Usually, surgery is required to treat this condition.

Secondary glaucoma, which is caused by other underlying eye conditions or systemic diseases, such as cataracts, eye trauma, tumors, or diabetes. Secondary glaucoma is treated by addressing the underlying cause, along with medications or surgery to manage eye pressure.

In an embodiment of the present invention including each and any aspect thereof, including any embodiment of such each and any aspect, glaucoma is primary open-angle glaucoma (POAG) or primary angle-closure glaucoma (PACG).

There is a number of risk factors for glaucoma, in particular (i) age with older people at higher risk; (ii) family history; (iii) ethnicity, with African Americans, Hispanics, and Asians being at higher risk for developing certain types of glaucoma than Caucasians; (iv) elevated intraocular pressure (IOP); (v) certain systemic medical conditions such as diabetes, migraine, sickle cell anemia or high blood pressure; (vi) eye conditions like high myopia or hyperopia, or previous eye injuries.

In its early stages, glaucoma typically is asymptomatic. As the disease progresses, however, it can cause loss of peripheral vision as first sign in most cases and tunnel vision in advanced stages; blurred vision or difficulty seeing at night in some cases. In PACG, typical symptoms are severe eye pain, nausea, vomiting and halos around lights. In light thereof, in an embodiment of the present invention including each and any aspect thereof, including any embodiment of such each and any aspect, the subject is a subject suffering from asymptomatic glaucoma. In an embodiment of the present invention, a subject suffering asymptomatic glaucoma is a subject being at risk of suffering from glaucoma, preferably suffering from symptomatic glaucoma.

Glaucoma is diagnosed through a series of tests, including measurement of intraocular pressure (IOP) with a tonometer; optic nerve imaging using optical coherence tomography (OCT) or fundus photography; perimetry to assess the field of vision, especially for peripheral vision loss; and pachymetry to measure the thickness of the cornea, which can influence IOP readings. In accordance therewith in an embodiment of the present invention including each and any aspect thereof, including any embodiment of such each and any aspect, the subject is a subject who has been or is diagnosed as suffering from or being at risk of suffering from glaucoma using a test of the above tests or a combination thereof.

GFS (trabeculectomy or glaucoma filtration surgery) is a surgical procedure with the goal to reduce IOP by creating a new drainage pathway for the fluid in the eye (aqueous humor) to escape, bypassing the blocked or dysfunctional drainage system (Koike and Chang 2018). The GFS procedure involves separating the conjunctiva from the sclera by making an incision at the junction of the cornea and the sclera (on the part of the eye normally hidden under the upper eyelid), to form a conjunctival flap that is folded back to expose the underlying sclera. A half-thickness incision is made into the sclera (usually 4 x 4 mm) at the corneo-scleral junction. The half-thickness scleral flap is raised towards the limbus and a small section of the sclera under the flap is removed (sclerostomy) allowing aqueous to leave the anterior chamber of the eye. The scleral flap is repositioned and loosely sutured. The flap guards the sclerostomy, preventing excessive egress of aqueous that leads to hypotony (a very soft eye). Finally, the conjunctiva is replaced. Aqueous passes through the sclera and collects under the conjunctiva as a bleb. Fluid in the bleb is absorbed by capillaries and lymphatics within the conjunctiva or evaporates across the conjunctiva. Final IOP is determined by many factors including the size of the bleb, the thickness of the conjunctiva and how adherent the conjunctiva around the bleb is to the sclera. If the conjunctiva overlying the operation site scars down onto the scleral flap then less aqueous can leave the eye, resulting in the return of raised IOP (Wilkins, Indar et al. 2005). The procedure is often very effective at lowering intraocular pressure, but like any surgery, it carries potential risks, such as infection, bleeding, or the possibility that the new drainage channel might become blocked or scarred over time. To prevent excessive scarring (fibrosis) and to increase the success of the surgical procedure, medication to control fibrosis is usually applied during the procedure, mitomycin C (MMC) or 5-fluorouracil (5FU).

GFS is typically used when other, less invasive treatments for glaucoma have not been effective in controlling IOP. It is usually considered when other interventions such as medications or laser treatments fail to adequately lower IOP or when the glaucoma is more advanced. Common situations where GFS might be used are (i) uncontrolled IOP despite medication; (ii) advanced or severe glaucoma, where there has already been significant damage to the optic nerve, to prevent further vision loss; (iii) failure of other surgeries or laser treatments have not been effective or have not provided sustained IOP control; (iv) patients who cannot tolerate medications or have difficulty adhering to a strict medication regimen; (v) in cases where glaucoma is progressing rapidly or is considered high-risk to prevent further damage to the optic nerve (Raj 2018). A decision to proceed with GFS is usually based on a careful assessment of the severity of the glaucoma, the patient's overall health, and their ability to manage other treatment options. In light thereof, in an embodiment of the present invention including each and any aspect thereof, including any embodiment of such each and any aspect, the subject is a subject suffering from glaucoma or being at risk of suffering from glaucoma who qualifies for glaucoma filtration surgery.

Glaucoma is staged using different glaucoma staging systems; one of the most common systems used in research was proposed by Mills et al. It classifies glaucoma stages from stage 0 to stage 5, with stage 0 being defined as high pressure in the eye but no signs of visual loss; stage 1 early glaucoma; stage 2 moderate glaucoma; stage 3 advanced glaucoma; stage 4 severe glaucoma and stage 5 end stage with no vision in the worst eye (Mills, Budenz et al. 2006).

Sustained control of IOP within the correct physiological range is necessary to maintain the anatomical conditions and to reduce the risk of glaucoma (Musch, Gillespie et al. 2008).

The use of GFS varies by region due to several factors such as healthcare infrastructure, availability of alternative treatments, and regional variations in the prevalence of different types of glaucoma. In many developed countries, in particular in the US, GFS is still a standard procedure for managing advanced glaucoma, but there has been a rise in the use of minimally invasive glaucoma surgeries (MIGS) in recent years. However, IOP control by MIGS is less efficient, therefore these options are rather considered for glaucoma patients with less severe disease.

The GFS procedure has potential risks and complications, so this option is typically reserved for cases where other treatments have not provided adequate results. There is a 5-year failure rate of >30% due to fibrotic closure of the channel created to allow fluid to leave the eye, despite administration of Mitomycin C during surgery (Capitena Young 2018, Craven, Singh et al. 2022, Hubner, Schlotzer-Schrehardt et al. 2022). Such failure rate will be decreased by the instant application using an inhibitor of CCL2 activity, in particular when such inhibitor of CCL2 activity is administered to a subject both locally and systemically.

Surgical treatment for glaucoma has undergone a dramatic change over the last decade. GFS has been the main surgical procedure worldwide for almost 50 years. However, there is a growth in development of new novel devices and surgical techniques designed to lower intraocular pressure (IOP) in a less invasive fashion. In fact, a plethora of new surgical glaucoma treatments have been added to the treatment options. The term minimally invasive glaucoma surgery (MIGS) has been coined (Bloom and Au 2018).

Minimally invasive glaucoma surgery (MIGS) has emerged as a novel approach in the glaucoma treatment spectrum, offering a range of diverse procedures and devices aimed at reducing intraocular pressure (IOP). MIGS is considered to have a shorter surgical time and fewer severe complications when compared to traditional glaucoma filtration surgery (Balas and Mathew 2023). MIGS can be broadly classified based on their target anatomical site, approach, and mechanism of action, and they are designed to improve the safety of surgical intervention for glaucoma (Ahmed 2015). Most MIGS procedures enhance physiologic outflow and are aimed at a different patient population than traditional GFS. MIGS can be broadly classified into several categories: those that enhance trabecular outflow (Trabectome, iStent, Hydrus Microstent, Kahook Dual Blade, high frequency deep sclerotomy, and gonioscopy-assisted transluminal trabeculotomy), those that augment suprachoroidal outflow (CyPass Microstent and iStent Supra), those that target Schlemm's canal (TRAB360 and the OMNI Surgical System, Streamline, and Ab Interno Canaloplasty), and conjunctival bleb-forming procedures (EXPRESS Glaucoma Filtration Device, Xen Gel Stent and PreserFlo MicroShunt) (Balas and Mathew 2023). As opposed to competing with traditional GFS, MIGS seems to be more of an alternative to medical therapy in an effort to address adherence challenges, adverse events, and quality-of-life (QOL) issues with local medications. Thus, MIGS devices often are used earlier in the glaucoma treatment algorithm.Reduction of IOP after GFS depends on the baseline IOP of each patient, the type of glaucoma, and the response to surgery. The goal is to reduce the pressure to a level that prevents further optic nerve damage while avoiding complications associated with too low an IOP (hypotony). The typical IOP target is usually between 6 and 18 mmHg, depending on the individual's baseline IOP. In a cohort study with glaucoma patients, GFS reduced the mean IOP from 27.5 mmHg to 15.0 mmHg, i.e. by more than 50% (Musch, Gillespie et al. 2008). In an embodiment of the present invention including each and any aspect thereof, including any embodiment of such each and any aspect, a subject not suffering from glaucoma is a subject having an intraocular pressure of about 6 to about 18 mm Hg, a subject not being at risk of suffering from glaucoma is a subject having an intraocular pressure of about 6 to about 18 mm Hg, and a subject successfully treated in accordance with the present invention is a subject having an intraocular pressure of between about 15 mm Hg and about 28 mm Hg, preferably an intraocular pressure of between about 6 mm Hg and about 18 mm Hg.

In a recent retrospective cohort study with 379 patients, GFS was successful in approx. 75% of cases after 5 years; the procedure was considered as a failure if one of the following criteria was met: IOP >18 mmHg, or <5 mmHg (hypotony), surgical complications, revision surgery or loss of light perception; the procedure was considered a complete success if it did not fail by these criteria and did not require supplemental medical therapy to lower the IOP (Wagner, Schuster et al. 2023). However, while some studies have reported similarly high success rates (Reibaldi, Uva et al. 2008), others have reported considerably lower survival rates without the use of IOP-lowering medication, for example, approximately 35% after 5 years (Chiu, Su et al. 2022) or 45.8% after 2 years (de Leon and Pionela 2021). Those patients with highest risk of GFS failure were younger or had uveitic glaucoma (Landers, Martin et al. 2012); in fact, patients under 30 years of age at surgery were at a higher risk for bleb failure (Chawla, Mercieca et al. 2013). Such success rate will be increased by the instant application using an inhibitor of CCL2 activity, in particular when such inhibitor of CCL2 activity is administered to a subject both locally and systemically. The latter in particular for those cases, where failure is due to fibrosis occurring as a result of surgical therapeutic intervention such as GFS. In light thereof, in an embodiment of the present invention including each and any aspect thereof, including any embodiment of such each and any aspect, a subject suffering from glaucoma is a subject having uveitic glaucoma.

Revision surgery after GFS is often performed to address complications or restore adequate intraocular pressure (IOP) control. It is typically considered when IOP is not well-controlled despite medical management or when there are complications that cannot be addressed conservatively, i.e. the optimal range between approx. 6 and 18 mmHg can no longer be maintained. However, there is a lack of consensus with regards to management of a failed GFS bleb and the decision depends largely on the surgeon's expertise and patient-specific factors. Reinstitution of medical therapy is usually the initial approach. If the GFS bleb is perceived as salvageable, then it may be revived by either needling or same-site revision of the bleb (Nikita and Murdoch 2018). Adjunctive wound-healing-modifying agents, such as 5FU and MMC are used intraoperatively for the majority of these cases (Broadway, Bloom et al. 2004, Coote, Gupta et al. 2011, Maestrini, Cronemberger et al. 2011). If these interventions fail, alternative approaches include laser trabeculoplasty, new augmented trabeculectomy, aqueous shunt implantation, or cyclodiode laser (Ederer, Gaasterland et al. 2004, Olali, Rotchford et al. 2011, Gedde, Schiffman et al. 2012). Revision of the GFS bleb involves exploration of the previous surgical site, opening the scleral flap, and ensuring a functional fistula, usually with the concomitant use of an antimetabolite (Anand and Arora 2007, Coote, Gupta et al. 2011), which presents significant potential advantages compared to other surgical approaches, as it utilizes the optimal position for a GFS bleb, minimizes intraocular manipulation, by not repeating a peripheral iridectomy, and does not traumatize new areas of conjunctiva and underlying tissues (Nikita and Murdoch 2018). In light thereof, in an embodiment of the present invention including each and any aspect thereof, including any embodiment of such each and any aspect, the subject suffering from glaucoma is a subject who underwent revision surgery after glaucoma filtration surgery, who is undergoing revision surgery after glaucoma filtration surgery or who will be undergoing revision surgery after glaucoma filtration surgery. In a further embodiment of the present invention including each and any aspect thereof, including any embodiment of such each and any aspect, the subject is treated by means of an inhibitor of CCL2 activity, wherein such inhibitor of CCL2 activity is locally, preferably subconjunctivally, or both locally, preferably subconjuntivally, and systemically administered to the subject in revision surgery after glaucoma filtration surgery to replace treatment with 5FU and MMC.

In accordance with the present invention, including each and any aspect thereof, including any embodiment of such each and any aspect, the administering an inhibitor of CCL2 activity either locally, preferably subconjunctivally, or both locally, preferably subconjunctivally, and systemically replaces local administration of antimetabolites mitomycin C and/or 5-fluorouracil in particular, in the treatment of glaucoma, and in particular in the treatment of glaucoma comprising glaucoma filtration surgery.

Antimetabolites, including mitomycin C (MMC) and 5-fluorouracil (5FU), were first used to modify the wound healing response in the early 1980s. MMC's alkylating properties inhibit DNA replication, which led to its use first as an anti-cancer drug. Much of the laboratory work on MMC's effectiveness followed a clinical report by Chen (Chen, Huang et al. 1990). At the concentrations used clinically MMC and 5FU inhibit or kill the fibroblast cells involved in the scarring response (Khaw, Sherwood et al. 1992).

5-FU is a pyrimidine analogue that blocks DNA synthesis through the inhibition of thymidylate synthesis (Green, Wilkins et al. 2014). The first successful animal model demonstrating the effectiveness of 5FU in bleb formation in the owl monkey was reported in 1984 (Gressel, Parrish et al. 1984). The same group published the findings of a pilot study in humans of the use of 5-FU in glaucoma filtering surgery (Heuer, Parrish et al. 1984). Subsequent laboratory research indicated that a single intraoperative application of 5-FU might be sufficient to control postoperative proliferation of scar tissue at the drainage site (Khaw, Sherwood et al. 1992).

Both antimetabolites increase the success rate of filtration surgery in animal models of filtration surgery (Khaw, Sherwood et al. 1992). The main side effect from inhibiting wound healing is that the conjunctiva overlying the sclerostomy may become very thin. In the early postoperative period, flow of aqueous through the sclerostomy may be greater leading to hypotony. With longer follow-up, holes can form in the conjunctiva that permit bacteria to enter the eye, triggering endophthalmitis. It has been argued that MMC and 5FU have a direct toxic effect on the ciliary body and epithelium, possibly reducing the production of aqueous (Wilkins, Indar et al. 2005, Green, Wilkins et al. 2014).

Both MMC and 5FU are usually administered to the sclera before or after the half-thickness scleral flap incision is made using sponges soaked with MMC (therapeutical concentration range 0.2-0.5 mg/mL) or 5-FU (therapeutical concentration range 25-50 mg/mL) solution applied for between two and five minutes (Wilkins, Indar et al. 2005, Green, Wilkins et al. 2014). Both MMC and 5FU are only applied once.

### Subtherapeutic range 0.004-0.2 mg/mL for MMC and 0.5-25 mg/mL 5FU

The terms inhibitor of Monocyte Chemoattractant Protein-1 (CCL2) activity, inhibitor of CCL2 activity, inhibitor of Monocyte Chemoattractant Protein-1 (MCP-1) activity and inhibitor of MCP-1 activity, and CCL2 antagonist are interchangeably used herein.

The inhibitor of CCL2 activity used in accordance with the present invention may make use of different modes of action. Such modes of action include that the inhibitor of CCL2 activity inhibits signaling of CCL2 via its receptor CCR2. In a preferred embodiment, the inhibitor of CCL2 activity binds directly to CCL2, such as by physical interaction, whereupon, preferably, one or several effects otherwise caused by the C-C chemokine receptor type 2 (CCR2; UniprotKB indentifier P41597, www.uniprot.org/uniprotkb/P41597/entry, accessed Feb 18, 2025) will be decreased or abolished. CCR2 serves as the main receptor for CCL2 and is expressed on a variety of immune cell types including monocytes, macrophages, and activated T cells (Frade, Mellado et al. 1997, Nieto, Navarro et al. 1998). CCR2 is among the most extensively studied receptors, mediating monocyte and macrophage recruitment to sites of inflammation and monocyte egress from the bone marrow (BM) (Boring, Gosling et al. 1997, Kurihara, Warr et al. 1997, Kuziel, Morgan et al. 1997). Preferably such decreased or abolished effects are effective in or helpful in the treatment of a disease, preferably the disease is glaucoma. In a more preferred embodiment, the binding of the inhibitor of CCL2 activity to CCR2 interferes with the binding of CCL2 to CCR2. In an alternative preferred embodiment, the inhibitor of CCL2 activity binds directly to CCR2, such as by physical interaction, whereupon CCL2 is no longer binding to CCR2 or at a reduced rate, with a decreased affinity or with a decreased shorter on-time. Preferably, due to such interference of the binding of CCL2 to CCR2 one or several effects otherwise caused by CCL2 will be decreased or abolished. Preferably such decreased or abolished effects are effective in or helpful in the treatment of a disease, preferably the disease is glaucoma.

In embodiments of each and any aspect, including any embodiment of each and any aspect, and as preferably used herein an inhibitor of CCL2 activity is a compound which binds to CCL2 or a compound which binds to the receptor of CCL2. Preferably the receptor of CCL2 is CCR2. Preferable, the binding of the inhibitor of CCL2 activity to CCL2 inhibits the binding to CCR2 of CCL2 bound by the inhibitor of CCL2 activity. Also preferably, the binding of the inhibitor of CCL2 activity to CCR2 inhibits the binding of CCL2 to CCR2 bound by the inhibitor of CCL2 activity. In these embodiments, activity of the inhibitor of CCL2 activity is its activity of inhibiting the binding of CCL2 to its receptor CCR2 and, respectively, the binding of CCR2 to its ligand CCL2.

In an alternative embodiment of each and any aspect, including any embodiment of each and any aspect, and as preferably used herein, an inhibitor of CCL2 activity is a compound that binds to or is capable of binding to CCL2 (both terms are used herein synonymously), whereby CCL2 bound by the inhibitor of CCL2 activity shows or results in reduced activity of CCR2 signaling. Such reduced activity of CCR2 can be determined in or by means of a Ca²⁺-release assay described in Example 8 herein or can be determined in or by means of a chemotaxis assay as described in Example 9 herein. In this embodiment, activity of the inhibitor of CCL2 activity is its activity of reducing the activity of CCR2, preferably resulting from inhibiting or reducing the binding of CCL2 to CCR2, wherein the inhibitor of CCL2 activity is bound to CCL2. It will be appreciated by a person skilled in the art that, preferably, the term inhibiting is a generic term, and that, preferably, the term reducing is a generic term, whereby the term reducing refers to a generic numerical concept of inhibition.

In a further alternative embodiment of each and any aspect, including any embodiment of each and any aspect, and as preferably used herein, an inhibitor of CCL2 activity is a compound that binds to CCR2, whereby CCR2 bound by the inhibitor of CCL2 activity shows or results in reduced activity of CCR2, preferably because the binding of CCL2 to CCR2 is blocked or reduced. Such reduced activity of CCR2 can be determined in or by means of a Ca²⁺-release assay described in Example 8 herein or can be determined in or by means of a chemotaxis assay as described in Example 9 herein. In this embodiment, activity of the inhibitor of CCL2 activity is its activity of reducing the activity of CCR2, preferably resulting from inhibiting or reducing the binding of CCL2 to CCR2, wherein the inhibitor of CCL2 activity is bound to CCR2. It will be appreciated by a person skilled in the art that, preferably, the term inhibiting is a generic term, and that, preferably, the term reducing is a generic term, whereby the term reducing refers to a generic numerical concept of inhibition.

In an embodiment of each and any aspect, including any embodiment of each and any aspect, the inhibitor of CCL2 activity is selected from the group comprising an aptamer binding to CCL2, an aptamer binding to CCR2, a Spiegelmer binding to CCL2, a Spiegelmer binding to CCR2, an antibody binding to CCL2, an antibody binding to CCR2, an antibody fragment binding to CCL2, an antibody fragment binding to CCR2, a protein binding to CCL2, a protein binding to CCR2, an anticalin binding to CCL2, an anticalin binding to CCR2, a small molecule binding to CCL2 and a small molecule binding to CCR2.

Aptamers are nucleic acid molecules made of D-nucleotides as building blocks which specifically bind to a target molecule through a mechanism different from Watson Crick base pairing. Spiegelmers are nucleic acid molecules made of L-nucleotides as building blocks which specifically bind to a target molecule through a mechanism different from Watson Crick base pairing. Both aptamers and spiegelmers can be made of ribonucleotides, of deoxyribonucleotides are a combination of both ribonucleotides and deoxyribonucleotides. Aptamers and spiegelmers as such are known to a person skilled in the art and are, among others, described in 'The Aptamer Handbook' (eds. Klussmann, 2006) and spiegelmers comprising both ribonucleotides and deoxyribonucleotides are disclosed in international patent application WO 2012/095303.

Anticalines are a class of target binding polypeptides is, among others, described in German patent application DE 197 42 706.

The generation of antibodies, including antibody fragments against targets such as CCL2 and CCR2 is within the skills of an ordinary person of the art.

In accordance with the present invention and in an embodiment of each and any aspect of the present invention, including any embodiment of each and any aspect, the inhibitor of CCL2 activity is an L-nucleic acid molecule which is binding to MCP-1, wherein the L-nucleic acid molecule is selected from the group comprising type 1A nucleic acids, type 1B nucleic acids, type 2 nucleic acids, type 3 nucleic acids, type 4 nucleic acids and nucleic acids having a nucleic acid sequence according to any of SEQ.ID.NOs. 87 to 115. These L-nucleic acid molecules are, for example, disclosed in WO 2007/093409 the disclosure of which is incorporated herein by reference.

As outlined in more detail in the embodiments and example 4, these CCL2 binding nucleic acid molecules can be characterised in terms of stretches of nucleotide which are also referred to herein as Boxes. The various CCL2 binding nucleic acid molecules can be categorised based on said Boxes and some structural features and elements, respectively. The various categories thus defined are also referred to herein as types and more specifically as type 1A, type 1B, type 2, type 3 and type 4.

These nucleic acids molecules also comprise nucleic acids which are essentially identical or homologous to the particular sequences disclosed herein. The term substantially identical or homologous shall be understood such that the identity and homology, respectively, is at least 75%, preferably 85%, more preferably 90% and most preferably more than 95 %, 96 %, 97 %, 98 % or 99%. Both terms identity and homology are used interchangeably and synonymously herein, unless indicated otherwise. Also, the terms identical and homologous are used interchangeably and synonymously herein, unless indicated otherwise.

The actual percentage of identical nucleotides present in the nucleic acid according to the present invention will depend on the total number of nucleotides present in the nucleic acid. The percent modification can be based upon the total number of nucleotides present in the nucleic acid.

The homology or identity can be determined as known to the person skilled in the art. More specifically, a sequence comparison algorithm then calculates the percent sequence identity for the test sequence(s) relative to the reference sequence, based on the designated program parameters. The test sequence is preferably the sequence or nucleic acid molecule which is said to be or to be tested whether it is homologous, and if so, to what extent, to another nucleic acid molecule, whereby such another nucleic acid molecule is also referred to as the reference sequence. In an embodiment, the reference sequence is a nucleic acid molecule as described herein, more preferably a nucleic acid molecule having a sequence according to any of SEQ. ID. NOs. 10 to 129, 132 to 256 and 278 - 282. Optimal alignment of sequences for comparison can be conducted, e.g., by the local homology algorithm of Smith & Waterman (Smith & Waterman, 1981) by the homology alignment algorithm of Needleman & Wunsch (Needleman & Wunsch, 1970) by the search for similarity method of Pearson & Lipman (Pearson & Lipman, 1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al (Altschul et al. 1990 and Altschul et al, 1997). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al (McGinnis et al, 2004).

In an embodiment of each and any aspect, including any embodiment thereof, the terms nucleic acid and nucleic acid molecule are used interchangeably and synonymously herein, unless indicated otherwise.

If not indicated to the contrary, any nucleotide sequence is set forth herein in 5' → 3' direction.

The nucleic acid molecules binding to CCL2 or CCR2 may be modified. Such modifications may be related to the single nucleotide of the nucleic acid and are well known in the art. Examples for such modification are described in, among others, Venkatesan (2003); Kusser (2000); Aurup (1994); Cummins (1995); Eaton (1995); Green (1995); Kawasaki (1993); Lesnik (1993); and Miller (1993). Such modification can be a H atom, a F atom or O-CH3 group or NH2-group at the 2' position of the individual nucleotide of which the nucleic acid consists. Also, the nucleic acid according to the present invention can comprises at least one LNA nucleotide. In an embodiment the nucleic acid according to the present invention consists of LNA nucleotides.

The nucleic acid molecules suitable for the practicing of the present invention preferably exhibit a very favourable K_{D} value range.

A possibility to determine the binding constant is the use of the so called biacore device, which is also known to the one skilled in the art. Affinity as used herein was also measured by the use of the "pull-down assay" as described in the example part herein. An appropriate measure in order to express the intensity of the binding between the nucleic acid according to the target which is in the present case CCL2, is the so-called K_{D} value which as such as well the method for its determination are known to the one skilled in the art.

The nucleic acids nucleic acid molecules suitable for the practicing of the present invention are characterized by a certain K_{D} value. Preferably, the K_{D} value shown by the nucleic acid molecules is below 1 µM. A K_{D} value of about 1 µM is said to be characteristic for a non-specific binding of a nucleic acid molecule to a target. As will be acknowledged by the ones in the art, the K_{D} value of a group of compounds such as the nucleic acid molecule suitable for the practicing of the present invention are within a certain range. The above-mentioned K_{D} of about 1 µM is a preferred upper limit for the K_{D} value. The preferred lower limit for the K_{D} of target binding nucleic acid molecules can be about 10 picomolar or higher. It is within the present invention that the K_{D} values of individual nucleic acids binding to CCL2 is preferably within this range. Preferred ranges can be defined by choosing any first number within this range and any second number within this range. Preferred upper values are 250 nM and 100 nM, preferred lower values are 50 nM, 10 nM, 1 nM, 100 pM and 10 pM.

The nucleic acid molecules suitable for the practicing of the present invention may have any length provided that they are still able to bind to the target molecule. It will be acknowledged in the art that there are preferred lengths of the nucleic acid molecules suitable for the practicing of the present invention. Typically, the length is between 15 and 120 nucleotides. It will be acknowledged by the ones skilled in the art that any integer between 15 and 120 is a possible length for the nucleic acid molecules suitable for the practicing of the present invention. More preferred ranges for the length of such nucleic acid molecules are lengths of about 20 to 100 nucleotides, about 20 to 80 nucleotides, about 20 to 60 nucleotides, about 20 to 50 nucleotides and about 30 to 50 nucleotides.

In an embodiment of each and any aspect of the invention, including any embodiment thereof, nucleic acid molecules comprise a moiety which preferably is a high molecular weight moiety and/or which preferably allows to modify the characteristics of the nucleic acid molecule in terms of, among others, residence time in the animal body, preferably the human body. A particularly preferred embodiment of such modification is PEGylation and HESylation of the nucleic acid molecules suitable for the practicing of the invention. As used herein PEG stands for poly(ethylene glycole) and HES for hydroxyethly starch. PEGylation as preferably used herein is the modification of said nucleic acid molecules, whereby such modification consists of a PEG moiety which is attached to said nucleic acid molecule. HESylation as preferably used herein is the modification of said nucleic acid molecule, whereby such modification consists of a HES moiety which is attached to the nucleic acid molecule. These modifications as well as the process of modifying a nucleic acid using such modifications, is described in European patent application EP 1 306 382, the disclosure of which is herewith incorporated in its entirety by reference.

Preferably, the molecular weight of a modification consisting of or comprising a high molecular weight moiety is about from 2,000 to 200,000 Da, preferably 20,000 to 120,000 Da, particularly in case of PEG being such high molecular weight moiety, and is preferably about from 3,000 to 180,000 Da, more preferably from 5,000 to 130,000 Da, particularly in case of HES being such high molecular weight moiety. The process of HES modification is, e.g., described in German patent application DE 1 2004 006 249.8 the disclosure of which is herewith incorporated in its entirety by reference.

PEG and HES may be used as either a linear or branched from as further described in the patent applications WO2005074993 and PCT/EP02/11950. Such modification can, in principle, be made to the nucleic acid molecules at any position thereof. Preferably such modification is made either to the 5' -terminal nucleotide, the 3'-terminal nucleotide and/or any nucleotide between the 5' nucleotide and the 3' nucleotide of the nucleic acid molecule.

The modification and preferably the PEG and/or HES moiety can be attached to the nucleic acid molecule suitable for the practicing of the present invention either directly or through a linker. It will be understood by a person skilled in the art that the nucleic acid molecule may comprise one or more modifications, preferably one or more PEG and/or HES moiety. In an embodiment, the individual linker molecule attaches more than one PEG moiety or HES moiety to the nucleic acid molecule. The linker used for attaching the modification to the nucleic acid molecule can itself be either linear or branched. This kind of linkers are known to the ones skilled in the art and are further described in the patent applications WO2005074993 and PCT/EP02/11950.

In an embodiment of each and any aspect, including any embodiment of each and any aspect, a compound which may be used as an inhibitor of CCL2 activity is selected from the group comprising a compound targeting CCL2, wherein the compound is selected from the group comprising Bindarit, a small molecule CCL2 inhibitor; Carlumab (CNTO888), a fully-human anti-CCL2 Mab; ABN-912, a fully-human anti-CCL2 Mab; CGEN-54 and a recombinant CCL2-inhibiting protein.

In an embodiment of each and any aspect, including any embodiment of each and any aspect, a compound which may be used as an inhibitor of CCL2 activity is selected from the group comprising a compound targeting CCR2, wherein the compound is selected from the group comprising AZ-889, a small molecule CCR2 antagonist; AZD-2423 a small molecule CCR2 antagonist, a small molecule CCR2 antagonist; BL-2030, a soluble CCR2 receptor fused to a human antibody Fc component; BMS-741672, a small molecule CCR2 antagonist; BMS-753426, a small molecule CCR2 antagonist; BMS-813160, a small molecule CCR2 antagonist; CCX-140, a small molecule CCR2 inhibitor; CCX-598, a small molecule CCR2 inhibitor; CCX-872, a small molecule CCR2 inhibitor; CCX-915, a small molecule CCR2 inhibitor; Cenicriviroc, a small molecule CCR2/CCR5 antagonist; CNTX-6970, a small molecule CCR2 antagonist; CPD-B, a small molecule CCR2 antagonist; EPX-102216, a small molecule CCR2 antagonist; INCB-3344, a small molecule CCR2 inhibitor; INCB-3284, a small molecule CCR2 inhibitor; INCB-8696, a small molecule CCR2 inhibitor; LF-0376, a small molecule CCR2/5 antagonist; MK-812, a small molecule CCR2 inhibitor; NIBR-6465, a small molecule CCR2/5 antagonist; OB-004 a small molecule CCR2 antagonist; OPL-CCL2-LPM, a human CCL2 chemokine fusion protein with cytotoxic payload; PD-172084, a small molecule CCR2 antagonist; PF-04634817, a small molecule CCR2/5 antagonist; PF-4136309, a small molecule CCR2 antagonist; Plozalizumab (MLN-1202), a humanized anti-CCR2 mAb; R-103, an oral Dala1-peptide T-amide analogue targeting CCR2, 5 and 8; RAP-103; RAP-310, both small molecule CCR2/5 antagonists; SB-380732, a small molecule CCR2 antagonist; SPR-3, a small molecule CCR2 antagonist; STI-B0201, a fully human anti-CCR2 mAb; STI-B0211, a fully human anti-CCR2 mAb; STI-B0221, a fully human anti-CCR2 mAb; STI-B0234, a fully human anti-CCR2 mAb; TAK-779, a small molecule CCR2/5 antagonist; and TLK-19705, small molecule CCR2 antagonist.

In an embodiment of each and any aspect, including any embodiment of each and any aspect, an inhibitor of CCL2 activity is characterized by slow elimination from the surgical site after subconjunctival administration to ensure durable inhibition of CCL2-mediated (i) attraction of monocytes/macrophages from peripheral blood to the surgical site and (ii) crosstalk between fibroblasts and macrophages, with both processes promoting fibrosis. At the same time, as the combination of local and systemic administration has been shown to be very efficient, the inhibitor of CCL2 activity also has pharmacokinetic properties in the blood plasma compartment that allow sustained pharmacological inhibition of CCL2, i.e. a plasma half-life of at least 24 h. Preferably, the agent should be stable in biological fluids and tissues.

It will be acknowledged by a person skilled in the art given the disclosure presented herein, possible administration schemes can be perceived by a person skilled in the art as a matter of routine. For example, the inhibitor of CCL2 activity is locally and in particular subconjunctivally administered to the subject on the day of glaucoma filtration surgery and then until week 8 after glaucoma filtration surgery. Within these 8 weeks, the inhibitor of CCL2 activity may be locally and in particular subconjunctivally administered to the subject in week 1, 2, 3, 4, 5, 6, 7 and 8; alternatively, the inhibitor of CCL2 activity may be locally and in particular subconjunctivally administered to the subject in week 1, 2, 4, 6 and 8. The inhibitor of CCL2 activity is systemically and in particular subcutaneously administered to the subject on the day of glaucoma filtration surgery and several times within the two weeks subsequent to glaucoma filtration surgery. Within these two weeks, the inhibitor of CCL2 activity may be administered in week 1 and in week 2. It will be equally acknowledged by a person skilled in the art that, in principle, any of the above schemes for local administration of the inhibitor of CCL2 activity may be combined with systemic administration of the inhibitor of CCL2 activity.

It will be acknowledged by a person skilled in the art given the disclosure presented herein, possible formulation schemes can be perceived by a person skilled in the art as a matter of routine. For example, if the inhibitor of CCL2 activity is compound NOX-E36, NOX-E36 is formulated at a concentration of 20 mg/ml formulation or less. In an embodiment, the concentration of NOX-E-36 is 13 mg/ml formulation. In a further embodiment, 200 µl of such formulation are locally, preferably subconjunctivally administered to the subject. In an embodiment, systemic, preferably subcutaneous administration of the inhibitor of CCL2 activity comprises administration of 40 mg NOX-E36 as a flat fixed dose. In connection with any concentration expressed as mg/ml, it is to be noted that in case the inhibitor of CCL2 activity is a compound comprising both a nucleic acid moiety which is formed by a sequence of nucleotides, and a non-nucleic acid moiety such as a PEG moiety, the milligram refer to the weight of the nucleic acid moiety of the inhibitor of CCL2 activity only.

In an embodiment of each and any aspect, including any embodiment thereof, the terms surgical procedure to reduce intraocular pressure and surgery for reducing intraocular pressure are used interchangeably and synonymously herein, unless indicated otherwise.

In an embodiment of each and any aspect, including any embodiment thereof, the terms surgical procedure to reduce intraocular pressure and surgery for reducing intraocular pressure are used interchangeably and synonymously herein, unless indicated otherwise.

In an embodiment of each and any aspect, including any embodiment thereof, the terms inhibitor of Monocyte Chemoattractant Protein-1 (CCL2) activity and inhibitor of CCL2 activity are used interchangeably and synonymously herein, unless indicated otherwise.

In an embodiment of each and any aspect, including any embodiment thereof, the terms CCL2, CCL-2 and MCP-1 are used interchangeably and synonymously herein, unless indicated otherwise.

Any reference to the invention or to the present invention refers in particular to each and any aspect of the invention, including any embodiment of such each and any aspect. Also, the terms aspect and aspect of the invention or aspect of the present invention are used interchangeably and synonymously herein, unless indicated otherwise.

In an embodiment of each and any aspect, including any embodiment thereof, the terms systemically administering and systemic administration have an interchangeable and synonymous meaning herein, unless indicated otherwise.

In an embodiment of each and any aspect, including any embodiment thereof, the terms topically administering and topical administration have an interchangeable and synonymous meaning herein, unless indicated otherwise. It will be appreciated by a person skilled in the art that a similar interchangeable and synonymous meaning exists for other routes of administration disclosed herein, whereby such administration is described by the noun and the characterizing adjective on the one hand and by the verb and the characterizing adverb on the other hand.

In an embodiment of each and any aspect, including any embodiment thereof, the term anti-fibrotic means that fibrosis is inhibited, reduced or avoided.

In an embodiment of each and any aspect, including any embodiment thereof, the term anti-fibrotic means that fibrosis is reduced.

In an embodiment of each and any aspect, including any embodiment thereof, the term anti-fibrotic means that fibrosis is avoided.

In an embodiment of each and any aspect, including any embodiment thereof, the term anti-fibrotic means that fibrosis is inhibited.

If reference is made to an embodiment of the invention or an embodiment of the present invention, such embodiment constitutes an embodiment of each and any aspect of the invention, including any embodiment of each and any aspect.

If reference is made herein to "any of Embodiments X to Y", this means that backreference is made to each and any individual embodiment contained within the range defined by X and Y, including the embodiments numbered as X and Y, respectively. In accordance therewith, all of said embodiments are disclosed individually and back-referenced. For example, if back-reference is to any one of embodiments 1 to 4, such back-reference means any one of embodiment 1, embodiment 2, embodiment 3 and embodiment 4.

The various SEQ.ID. Nos., the chemical nature of the nucleic acid molecules according to the present invention and the target molecules MCP-1 as used herein, the actual sequence thereof and the internal reference number is summarized in the following table.

| Seq.-ID | ***RNA*/*Peptide*** | **Sequence** | ***Internal Reference*** |
|---|---|---|---|
| 1 | L-protein | QPDAINAPVTCCYNFTNRKISVQRLASYRRITSSKCPKEAVIFKTIVAKEICADPKQKWV QDSMDHLDKQTQTPKT | human MCP-1, huMCP-1, CCL2 |
| 2 | L-protein | QPDAVNAPLTCCYSFTSKMIPMSRLESYKRITSSRCPKEAVVFVTKLKREVCADPKKEWV QTYIKNLDRNQMRSEPTTLFKTASALRSSAPLNVKLTRKSEANASTTFSTTTSSTSVGVT SVTVN | mouse MCP-1, mCCL2, mMCP-1, murine MCP-1 (Mus musculus) |
| 3 | L-protein | QPDAINAPVTCCYNFTNRKISVQRLASYRRITSSKCPKEAVIFKTIVAKEICADPKQKWV QDSMDHLDKQIQTPKP | monkey MCP-1 (Macaca mulatta) |
| 4 | L-protein | QPDAINSPVTCCYTLTSKKISMQRLMSYRRVTSSKCPKEAVIFKTIAGKEICAEPKQKWV QDSISHLDKKNQTPKP | pig MCP-1 (Sus scrofa) |
| 5 | L-protein | QPDAIISPVTCCYTLTNKKISIQRLASYKRVTSSKCPKEAVIFKTVLNKEICADPKQKWV QDSMAHLDKKSQTQTA | dog MCP-1 (Canis familiaris) |
| 6 | L-protein | QPDAVNSPVTCCYTFTNKTISVKRLMSYRRINSTKCPKEAVIFMTKLAKGICADPKQKWV QDAIANLDKKMQTPKTLTSYSTTQEHTTNLSSTRTPSTTTSL | rabbit MCP-1 (Oryctolagus cuniculus) |
| 7 | L-protein | QPVGINTSTTCCYRFINKKIPKQRLESYRRTTSSHCPREAVIFKTKLDKEICADPTQKWV QDFMKHLDKKTQTPKL | human MCP-3, CCL7, huMCP-3 |
| 8 | L-protein | GPASVPTTCCFNLANRKIPLQRLESYRRITSGKCPQKAVIFKTKLAKDICADPKKKWVQD SMKYLDQKSPTPKP | human eotaxin/CCL11 |
| 9 | L-protein | QPDSVSIPITCCFNVINRKIPIQRLESYTRITNIQCPKEAVIFKTKRGKEVCADPKERWV RDSMKHLDQIFQNLKP | human MCP-2, CCL8, huMCP-2 |
| 10 | L-RNA | AGCGUGCCCGGAGUGGCAGGGGGACGCGACCUGCAAUAAUGCACGCU | 169-B1trc |
| 11 | L-RNA | AGCGUGCCCGGAGUGGCAGGGGGACGCGACCUGCAAUUGCACGCU | 169-F3trc |
| 12 | L-RNA | AGCGUGCCCGGAGUGGCAGGGGGACGCGACCUGUAAUAAUGCACGCU | 169-C1trc |
| 13 | L-RNA | AGCGUGCCCGGUGUGGCAGGGGGACGCGACCUGCAAUAAUGCGCGCU | 169-A3trc |
| 14 | L-RNA | AGCGUGCCCGGAGUAGCAGGGGGGCGCGACCUGCAAUAAUGCACGCU | 169-B2trc |
| 15 | L-RNA | AGCGUGCCCGGUGUGGUAGGGGGGCGCGAUCUACAAUUGCACGCU | 176-B12trc |
| 16 | L-RNA | AGCGUGCCCGGUGUGACAGGGGGGCGCGACCUGCAUUUGCACGCU | 176-D9trc |
| 17 | L-RNA | AGCGUGCCCGGUGUGGCAGGGGGGCGCGACCUGUAUUUGCACGCU | 176-B10trc |
| 18 | L-RNA | AGCGUGCCCGGAGUGGCAGGGGGGCGCGACCUGCAAUAAUGCACGCU | 169-F2trc |
| 19 | L-RNA | AGCGUGCCCGGUGUGGCAGGGGGGCGCGACCUGCAAUUGCACGCU | 176-B9trc |
| 20 | L-RNA | AGCAUGCCCGGUGUGGCAGGGGGGCGCGACCUGCAUUUGCAUGCU | 176-H9trc |
| 21 | L-RNA | AGCGUGCCCGGUGUGGUAGGGGGGCGCGACCUACAUUUGCACGCU | 176-E10trc |
| 22 | L-RNA | AGUGUGCCAGCUGUGAUGGGGGGGCGCGACCCAUUUUACACACU | 176-G9trc |
| 23 | L-RNA | AGUGUGCCAGCGUGAUGGGGGGGCGCGACCCAUUUUACACACU | 176-F9trc |
| 24 | L-RNA | AGUGUGCGAGCGUGAUGGGGGGGCGCGACCCAUUUUACAUACU | 176-C11trc |
| 25 | L-RNA | AGUGUGCCAGCGUGAUGGGGGGGCGCGACCCAUUUUACAUACU | 176-E11trc |
| 26 | L-RNA | AGUAUGCCAGCGUGAUGGGGGGGCGCGACCCAUUUACAUACU | 176-D10trc |
| 27 | L-RNA | AGUGUGCCAGUGUGAUGGGGGGGCGCGACCCAUUUUACACACU | 176-H10trc |
| 28 | L-RNA | AGCGUGCCAGUGUGAUGGGGGGGCGCGACCCAUUUUACACGCU | 176-C9trc |
| 29 | L-RNA | ACGCACGUCCCUCACCGGUGCAAGUGAAGCCGCGGCUCUGCGU | 180-B1-001 |
| 30 | L-RNA | ACGCACCUCCCUCACCGGUGCAAGUGAAGCCGUGGCUCUGCGC | 180-A4-002 |
| 31 | L-RNA | ACGCACGUCCCUCACCGGUGCAAGUGAAGCCGUGGCUCUGCGU | 180-D1-002 |
| 32 | L-RNA | GCACGUCCCUCACCGGUGCAAGUGAAGCCGUGGCUCUGCGU | 180-D1-011 |
| 33 | L-RNA | ACGCACGUCCCUCACCGGUGCAAGUGAAGCCGUGGCUCUGC | 180-D1-012 |
| 34 | L-RNA | GCACGUCCCUCACCGGUGCAAGUGAAGCCGUGGCUCUGC | 180-D1-018 |
| 35 | L-RNA | CGCACGUCCCUCACCGGUGCAAGUGAAGCCGUGGCUCUGCGU | 180-D1-034 |
| 36 | L-RNA | CGCACGUCCCUCACCGGUGCAAGUGAAGCCGUGGCUCUGCG | 180-D1-035 |
| 37 | L-RNA | GCACGUCCCUCACCGGUGCAAGUGAAGCCGUGGCUCUGCG | 180-D1-036 = NOX-E36 |
| 38 | L-RNA | GUGCUGCGUAGUGGAAGACUACCUAAUGACAGCCGAAUGCUGGCAGCAC | 178-A8 |
| 39 | L-RNA | GUGCUGCGUAGUGGAAGACUACCUAAUGACAGCCUAAUGCUGGCAGCAC | 178-F7 |
| 40 | L-RNA | GUGCUGCGUAGUGGAAGACUACCUUAUGACAGCCGAAUGCUGGCAGCAC | 178-G7 |
| 41 | L-RNA | GUGCUGCGUAGUGAAAAACUACUGCCAGUGGGUCAGAGCUAGCAGCAC | 178-C6 |
| 42 | L-RNA | GUGCUGCGGAGUUAAAAACUCCCUAAGACAGGCCAGAGCCGGCAGCAC | 178-E7 |
| 43 | L-RNA | GUGCUGCGGAGUUGAAAACUCCCUAAGACAGGCCAGAGCCGGCAGCAC | 178-G6 |
| 44 | L-RNA | GUGCUGCGUAGUGGAAGACUACCUAUGACAGCCUAAUGCUGGCAGCAC | 178-A7 |
| 45 | L-RNA | GUGCUGCGGAGUUAAAAACUCCCUAAGACAGGCUAGAGCCGGCAGCAC | 178-C7 |
| 46 | L-RNA | GUGCUGCGGCGUGAAAAACGCCCUGCGACUGCCCUUUAUGCAGGCAGCAC | 178-E5 |
| 47 | L-RNA | GUGCUGCGUAGUGAAAAACUACCAACGACUGGCUAGAGCCGGCAGCAC | 181-F1 |
| 48 | L-RNA | GUGCUGCGUAGUGAAAGACUACCUGUGACAGCCGAAUGCUGGCAGCAC | 181-B2 |
| 49 | L-RNA | GUACUGCGUAGUUAAAAACUACCAACGACUGGCUAGAGCCGGCAGCAC | 181-C2 |
| 50 | L-RNA | GUGCUGCGUAGUUAAAAACUACCAACGACUGGCUAGAGCCGGCAGCAC | 178-A6 |
| 51 | L-RNA | GUGCUGCGUAGUUAAAAACUACCAGCGACAGGCUAGAGCCGGCAGCAC | 178-D6 |
| 52 | L-RNA | GUGCUGCGUAGUUAAAAACUACCAGCGACUGGCUAGAGCCGGCAGCAC | 178-D5 |
| 53 | L-RNA | GUGCUGCGUAGUGAGAAACUACCAACGACUGGCUAGAGCCGGCAGCAC | 181-A2 |
| 54 | L-RNA | GGCUGCGUAGUUAAAAACUACCAGCGACUGGCUAGAGCCGGCAGCC | 178-D5-020 |
| 55 | L-RNA | GGCGCGUAGUUAAAAACUACCAGCGACUGGCUAGAGCCGGCGCC | 178-D5-027 |
| 56 | L-RNA | GUGCGCGUAGUUAAAAACUACCAGCGACUGGCUAGAGCCGGCGCAC | 178-D5-030 |
| 57 | L-RNA | GUGCGCGUAGUGAGAAACUACCAACGACUGGCUAGAGCCGGCGCAC | 181-A2-002 |
| 58 | L-RNA | GUGCCGUAGUGAGAAACUACCAACGACUGGCUAGAGCCGGGCAC | 181-A2-004 |
| 59 | L-RNA | GUGGCGUAGUGAGAAACUACCAACGACUGGCUAGAGCCGGCCAC | 181-A2-005 |
| 60 | L-RNA | GUCGCGUAGUGAGAAACUACCAACGACUGGCUAGAGCCGGCGAC | 181-A2-006 |
| 61 | L-RNA | UGCGCGUAGUGAGAAACUACCAACGACUGGCUAGAGCCGGCGCA | 181-A2-007 |
| 62 | L-RNA | GCUGCGUAGUGAGAAACUACCAACGACUGGCUAGAGCCGGCAGC | 181-A2-008 |
| 63 | L-RNA | GCUGCGUAGUGAGAAACUACCAACGACUGGCUAGAGCCGGCAGC | 181-A2-011 |
| 64 | L-RNA | GGUGCGUAGUGAGAAACUACCAACGACUGGCUAGAGCCGGCACC | 181-A2-012 |
| 65 | L-RNA | UGGCGUAGUGAGAAACUACCAACGACUGGCUAGAGCCGGC-CA | 181-A2-015 |
| 66 | L-RNA | GCGCGUAGUGAGAAACUACCAACGACUGGCUAGAGCCGGCGC | 181-A2-016 |
| 67 | L-RNA | GUGCGUAGUGAGAAACUACCAACGACUGGCUAGAGCCGGCAC | 181-A2-017 |
| 68 | L-RNA | GG-GCGUAGUGAGAAACUACCAACGACUGGCUAGAGCCGGCCC | 181-A2-018 |
| 69 | L-RNA | GAGCGUAGUGAGAAACUACCAACGACUGGCUAGAGCCGGCUC | 181-A2-019 |
| 70 | L-RNA | CGGCGUAGUGAGAAACUACCAACGACUGGCUAGAGCCGGCCG | 181-A2-020 |
| 71 | L-RNA | CCGCGUAGUGAGAAACUACCAACGACUGGCUAGAGCCGGCGG | 181-A2-021 |
| 72 | L-RNA | CAGCGUAGUGAGAAACUACCAACGACUGGCUAGAGCCGGCUG | 181-A2-022 |
| 73 | L-RNA | CUGCGUAGUGAGAAACUACCAACGACUGGCUAGAGCCGGCAG | 181-A2-023 |
| 74 | L-RNA | AGCGUGUUAGUGAAGUGGGUGGCAGGUAAAGGACACGCU | 184-B8trc |
| 75 | L-RNA | AGCGUGGUAGCGGUGUGGGUGGUAGGUAAAGGCCACGCU | 184-C6trc |
| 76 | L-RNA | AGCGUGAUAGAAGAGCGGGUGGUAGGUAAAGGUCAGGCU | 184-H5trc |
| 77 | L-RNA | AGCGUGUUAGGUAGGGUGGUAGUAAGUAAAGGACACGCU | 184-A7trc |
| 78 | L-RNA | AGCGUGUUAGGUGGGUGGUAGUAAGUAAAGGACACGCU | 187-A5trc |
| 79 | L-RNA | AGCGUGUUAGGUGGGUGGUAGUAAGUAAAGGGCACGCU | 187-H5trc |
| 80 | L-RNA | CCGCUUAGGUGGGUGGUAGUAAGUAAAGGGGCGG | 174-D4-004 |
| 81 | L-RNA | GCGCGAGCAGGUGGGUGGUAGAAUGUAAAGACUCGCGUC | 166-A4-002 |
| 82 | L-RNA | CGUGUUAGGUGGGUGGUAGUAAGUAAAGGACACG | 187-A5trc-001 |
| 83 | L-RNA | GUGUUAGGUGGGUGGUAGUAAGUAAAGGACAC | 187-A5trc-002 |
| 84 | L-RNA | CGUGUUAGGUGGGUGGUAGUAAGUAAAGGGCACG | 187-H5trc-002 |
| 85 | L-RNA | GUGUUAGGUGGGUGGUAGUAAGUAAAGGGCAC | 187-H5trc-003 |
| 86 | L-RNA | UGUUAGGUGGGUGGUAGUAAGUAAAGGGCA | 187-H5trc-004 |
| 87 | L-RNA | GGACGAGAGUGACAAAUGAUAUAACCUCCUGACUAACGCUGCGGGCGACAGG | 177-B3 |
| 88 | L-RNA | GGACCUAUCGCUAAGACAACGCGCAGUCUACGGGACAUUCUCCGCGGACAGG | 177-C1 |
| 89 | L-RNA | GGACAAUUGUUACCCCCGAGAGAGACAAAUGAGACAACCUCCUGAAGACAGG | 177-C2 |
| 90 | L-RNA | GGACGAAAGUGAGAAAUGAUACAACCUCCUGUUGCUGCGAAUCCGGACAGG | 177-E3 |
| 91 | L-RNA | GGACGUAAAAGACGCUACCCGAAAGAAUGUCAGGAGGGUAGACCGACAGG | 177-D1 |
| 92 | L-RNA | GGACUAGAAACUACAAUAGCGGCCAGUUGCACCGCGUUAUCAACGACAGG | 177-E1 |
| 93 | L-RNA | GGACUAGUCAGCCAGUGUGUAUAUCGGACGCGGGUUUAUUUACUGACAGG | 177-A1 |
| 94 | L-RNA | GGACUGUCCGGAGUGUGAAACUCCCCGAGACCGCCAGAAGCGGGGACAGG | 177-G3 |
| 95 | L-RNA | GGACUUCUAUCCAGGUGGGUGGUAGUAUGUAAAGAGAUAGAAGUGACAGG | 177-C3 |
| 96 | L-RNA | GGACGAGAGCGAACAAUGAUAUAACCUCCUGACGGAAAGAGAUCGACAGG | 177-A2 |
| 97 | L-RNA | CCUGUGCUACACGCAGUAAGAAGUGAACGUUCAGUAUGUGUGCACAGG | 170-E4trc |
| 98 | L-RNA | CGUGAGCCAGGCACCGAGGGCGUUAACUGGCUGAUUGGACACGACACG | 166-D2trc |
| 99 | L-RNA | CGUGAACAUGCAAGCUAAGCGGGGCUGUUGGUUGCUUGGCCCGCCACG | 174-A2trc |
| 100 | L-RNA | CGUGCAGAGAGAGACCAACCACGUAAAAUCAACCUAAUGGGCCGCACG | 174-E2trc |
| 101 | L-RNA | CGUGCAGAGAGAGACCAACCACGUAAAAUCAACCUAAUGGGCCGCACG | 183-G3trc |
| 102 | L-RNA | CGUGAACAUUCAAGCUAAGCGGGGCUGUUGGUUGCUUGGCCCGCCACG | 183-B2trc |
| 103 | L-RNA | CGUGCCGAGGCGGCGACCAGCGUUACUUAGAGAGGCUUUGGCACCACG | 166-B2trc |
| 104 | L-RNA | CGUGAUAACAGCCGUCGGUCAAGAAAACAAAGUUCGGGCGGCGCACG | 166-G3trc |
| 105 | L-RNA | CGUGGGUGGCGCACCGAGGGCGAAAAGCCACCAGUAAAGAUAGACCG | 166-D1trc |
| 106 | L-RNA | CGUGUGAUCUCCUUUGGGGUGAUUAGCUUAGAGACUUCCCACACG | 183-H2trc |
| 107 | L-RNA | GCACCUUCGCCUAAUACACGUGCCGGCUAGCUAAUACUCGUCCGC | 167-A7trc |
| 108 | L-RNA | GCACGACUUGGGCGACCAGUGAUACUUAGAGAGCAAGUCGUCGGC | 167-C7trc |
| 109 | L-RNA | GCGCGCGCUCAGUAAGAAAUUGAAAGUUCAGAAUGUCGUCGCGC | 167-B5trc |
| 110 | L-RNA | AGUGUGUGGCAGGCUAAGGAGAUAUUCCGAGACCACGCU | 184-D7trc |
| 111 | L-RNA | AGUGUGUGGCAGACUAUGGAUAGACUCCGAGACCACGCU | 184-D6trc |
| 112 | L-RNA | AGCGUGAGGCGACCAGCGGAUUACUUAGAGAGUCACGCU | 184-E5trc |
| 113 | L-RNA | AGCGUGAAGGGGACCAGCGUUACUUACAGAGUUCACGCU | 184-G6trc |
| 114 | L-RNA | AGCGUGUGAUGUAUGUAGCACCGUAUCAGAGGACACGCU | 184-B7trc |
| 115 | L-RNA | AGCGUGAGGCGACCCGUGUUUCGUAGAGAGUCACGCU | 184-B6trc |
| 116 | L-RNA | 5' PEG-GCACGUCCCUCACCGGUGCAAGUGAAGCCGUGGCUCUGCG | NOX-E36-5' PEG |
| 117 | L-RNA | GCACGUCCCUCACCGGUGCAAGUGAAGCCGUGGCUCUGCG-3'PEG | NOX-E36-3' PEG |
| 118 | L-RNA | GAGAUGGCGACAUUGGUUGGGCAUGAGGCGAGGCCCUUUGAUGAAUCCGCGGCCAUUC | 188-A3-001 |
| 119 | L-RNA | GAUGGCGACAUUGGUUGGGCAUGAGGCGAGGCCCUUUGAUGAAUCCGCGGCCAUUC | 188-A3-004 |
| 120 | L-RNA | GGCGACAUUGGUUGGGCAUGAGGCGAGGCCCUUUGAUGAAUCCGCGGCCAUUC | 188-A3-005 |
| 121 | L-RNA | GGCGACAUUGGUUGGGCAUGAGGCGAGGCCCUUUGAUGAAUCCGCGGCCAUU | 188-A3-006 |
| 122 | L-RNA | GGCGACAUUGGUUGGGCAUGAGGCGAGGCCCUUUGAUGAAUCCGCGGCCA | 188-A3-007 = mNOX-E36 |
| 123 | L-RNA | GCUGGUUACCGAGGGGGCGUCGUUGGAGUUUGGUUGGUUGUCACCAGC | 189-G7-001 |
| 124 | L-RNA | CUGGUUACCGAGGGGGCGUCGUUGGAGUUUGGUUGGUUGUCACCAG | 189-G7-002 |
| 125 | L-RNA | UGGUUACCGAGGGGGCGUCGUUGGAGUUUGGUUGGUUGUCACCA | 189-G7-003 |
| 126 | L-RNA | GCCGGUUACCGAGGGGGCGUCGUUGGAGUUUGGUUGGUUGUCACCGGC | 189-G7-007 |
| 127 | L-RNA | GCCGGCUACCGAGGGGGCGUCGUUGGAGUUUGGUUGGUUGUCGCCGGC | 189-G7-008 |
| 128 | L-RNA | GCGCGUACCGAGGGGGCGUCGUUGGAGUUUGGUUGGUUGUCCGCGC | 189-G7-010 |
| 129 | L-RNA | GGGCCUACCGAGGGGGCGUCGUUGGAGUUUGGUUGGUUGUCGGCCC | 189-G7-012 |
| 130 | D-protein | Biotin- QPDAINAPVTCCYNFTNRKISVQRLASYRRITSSKCPKEAVIFKTIVAKEICADPKQKWV QDSMDHLDKQTQTPKT | biotinylated human D-MCP-1 |
| 131 | D-protein | Biotin- QPDAVNAPLTCCYSFTSKMIPMSRLESYKRITSSRCPKEAVVFVTKLKREVCADPKKEWV QTYIKNLDRNQMRSEP-Biotin | biotinylated mouse D-MCP-1 |
| 132 | D-RNA | AGCGUGCCCGGAGUGGCAGGGGGACGCGACCUGCAAUAAUGCACGCU | 169-B1trc |
| 133 | D-RNA | AGCGUGCCCGGAGUGGCAGGGGGACGCGACCUGCAAUUGCACGCU | 169-F3trc |
| 134 | D-RNA | AGCGUGCCCGGAGUGGCAGGGGGACGCGACCUGUAAUAAUGCACGCU | 169-C1trc |
| 135 | D-RNA | AGCGUGCCCGGUGUGGCAGGGGGACGCGACCUGCAAUAAUGCGCGCU | 169-A3trc |
| 136 | D-RNA | AGCGUGCCCGGAGUAGCAGGGGGGCGCGACCUGCAAUAAUGCACGCU | 169-B2trc |
| 137 | D-RNA | AGCGUGCCCGGUGUGGUAGGGGGGCGCGAUCUACAAUUGCACGCU | 176-B12trc |
| 138 | D-RNA | AGCGUGCCCGGUGUGACAGGGGGGCGCGACCUGCAUUUGCACGCU | 176-D9trc |
| 139 | D-RNA | AGCGUGCCCGGUGUGGCAGGGGGGCGCGACCUGUAUUUGCACGCU | 176-B10trc |
| 140 | D-RNA | AGCGUGCCCGGAGUGGCAGGGGGGCGCGACCUGCAAUAAUGCACGCU | 169-F2trc |
| 141 | D-RNA | AGCGUGCCCGGUGUGGCAGGGGGGCGCGACCUGCAAUUGCACGCU | 176-B9trc |
| 142 | D-RNA | AGCAUGCCCGGUGUGGCAGGGGGGCGCGACCUGCAUUUGCAUGCU | 176-H9trc |
| 143 | D-RNA | AGCGUGCCCGGUGUGGUAGGGGGGCGCGACCUACAUUUGCACGCU | 176-E10trc |
| 144 | D-RNA | AGUGUGCCAGCUGUGAUGGGGGGGCGCGACCCAUUUUACACACU | 176-G9trc |
| 145 | D-RNA | AGUGUGCCAGCGUGAUGGGGGGGCGCGACCCAUUUUACACACU | 176-F9trc |
| 146 | D-RNA | AGUGUGCGAGCGUGAUGGGGGGGCGCGACCCAUUUUACAUACU | 176-C11trc |
| 147 | D-RNA | AGUGUGCCAGCGUGAUGGGGGGGCGCGACCCAUUUUACAUACU | 176-E11trc |
| 148 | D-RNA | AGUAUGCCAGCGUGAUGGGGGGGCGCGACCCAUUUACAUACU | 176-D10trc |
| 149 | D-RNA | AGUGUGCCAGUGUGAUGGGGGGGCGCGACCCAUUUUACACACU | 176-H10trc |
| 150 | D-RNA | AGCGUGCCAGUGUGAUGGGGGGGCGCGACCCAUUUUACACGCU | 176-C9trc |
| 151 | D-RNA | ACGCACGUCCCUCACCGGUGCAAGUGAAGCCGCGGCUCUGCGU | 180-B1-001 |
| 152 | D-RNA | ACGCACCUCCCUCACCGGUGCAAGUGAAGCCGUGGCUCUGCGC | 180-A4-002 |
| 153 | D-RNA | ACGCACGUCCCUCACCGGUGCAAGUGAAGCCGUGGCUCUGCGU | 180-D1-002 |
| 154 | D-RNA | GCACGUCCCUCACCGGUGCAAGUGAAGCCGUGGCUCUGCGU | 180-D1-011 |
| 155 | D-RNA | ACGCACGUCCCUCACCGGUGCAAGUGAAGCCGUGGCUCUGC | 180-D1-012 |
| 156 | D-RNA | GCACGUCCCUCACCGGUGCAAGUGAAGCCGUGGCUCUGC | 180-D1-018 |
| 157 | D-RNA | CGCACGUCCCUCACCGGUGCAAGUGAAGCCGUGGCUCUGCGU | 180-D1-034 |
| 158 | D-RNA | CGCACGUCCCUCACCGGUGCAAGUGAAGCCGUGGCUCUGCG | 180-D1-035 |
| 159 | D-RNA | GCACGUCCCUCACCGGUGCAAGUGAAGCCGUGGCUCUGCG | (D-)180-D1-036, (D-)NOX-E36 |
| 160 | D-RNA | GUGCUGCGUAGUGGAAGACUACCUAAUGACAGCCGAAUGCUGGCAGCAC | 178-A8 |
| 161 | D-RNA | GUGCUGCGUAGUGGAAGACUACCUAAUGACAGCCUAAUGCUGGCAGCAC | 178-F7 |
| 162 | D-RNA | GUGCUGCGUAGUGGAAGACUACCUUAUGACAGCCGAAUGCUGGCAGCAC | 178-G7 |
| 163 | D-RNA | GUGCUGCGUAGUGAAAAACUACUGCCAGUGGGUCAGAGCUAGCAGCAC | 178-C6 |
| 164 | D-RNA | GUGCUGCGGAGUUAAAAACUCCCUAAGACAGGCCAGAGCCGGCAGCAC | 178-E7 |
| 165 | D-RNA | GUGCUGCGGAGUUGAAAACUCCCUAAGACAGGCCAGAGCCGGCAGCAC | 178-G6 |
| 166 | D-RNA | GUGCUGCGUAGUGGAAGACUACCUAUGACAGCCUAAUGCUGGCAGCAC | 178-A7 |
| 167 | D-RNA | GUGCUGCGGAGUUAAAAACUCCCUAAGACAGGCUAGAGCCGGCAGCAC | 178-C7 |
| 168 | D-RNA | GUGCUGCGGCGUGAAAAACGCCCUGCGACUGCCCUUUAUGCAGGCAGCAC | 178-E5 |
| 169 | D-RNA | GUGCUGCGUAGUGAAAAACUACCAACGACUGGCUAGAGCCGGCAGCAC | 181-F1 |
| 170 | D-RNA | GUGCUGCGUAGUGAAAGACUACCUGUGACAGCCGAAUGCUGGCAGCAC | 181-B2 |
| 171 | D-RNA | GUACUGCGUAGUUAAAAACUACCAACGACUGGCUAGAGCCGGCAGCAC | 181-C2 |
| 172 | D-RNA | GUGCUGCGUAGUUAAAAACUACCAACGACUGGCUAGAGCCGGCAGCAC | 178-A6 |
| 173 | D-RNA | GUGCUGCGUAGUUAAAAACUACCAGCGACAGGCUAGAGCCGGCAGCAC | 178-D6 |
| 174 | D-RNA | GUGCUGCGUAGUUAAAAACUACCAGCGACUGGCUAGAGCCGGCAGCAC | 178-D5 |
| 175 | D-RNA | GUGCUGCGUAGUGAGAAACUACCAACGACUGGCUAGAGCCGGCAGCAC | 181-A2 |
| 176 | D-RNA | GGCUGCGUAGUUAAAAACUACCAGCGACUGGCUAGAGCCGGCAGCC | 178-D5-020 |
| 177 | D-RNA | GGCGCGUAGUUAAAAACUACCAGCGACUGGCUAGAGCCGGCGCC | 178-D5-027 |
| 178 | D-RNA | GUGCGCGUAGUUAAAAACUACCAGCGACUGGCUAGAGCCGGCGCAC | 178-D5-030 |
| 179 | D-RNA | GUGCGCGUAGUGAGAAACUACCAACGACUGGCUAGAGCCGGCGCAC | 181-A2-002 |
| 180 | D-RNA | GUGCCGUAGUGAGAAACUACCAACGACUGGCUAGAGCCGGGCAC | 181-A2-004 |
| 181 | D-RNA | GUGGCGUAGUGAGAAACUACCAACGACUGGCUAGAGCCGGCCAC | 181-A2-005 |
| 182 | D-RNA | GUCGCGUAGUGAGAAACUACCAACGACUGGCUAGAGCCGGCGAC | 181-A2-006 |
| 183 | D-RNA | UGCGCGUAGUGAGAAACUACCAACGACUGGCUAGAGCCGGCGCA | 181-A2-007 |
| 184 | D-RNA | GCUGCGUAGUGAGAAACUACCAACGACUGGCUAGAGCCGGCAGC | 181-A2-008 |
| 185 | D-RNA | GCUGCGUAGUGAGAAACUACCAACGACUGGCUAGAGCCGGCAGC | 181-A2-011 |
| 186 | D-RNA | GGUGCGUAGUGAGAAACUACCAACGACUGGCUAGAGCCGGCACC | 181-A2-012 |
| 187 | D-RNA | UGGCGUAGUGAGAAACUACCAACGACUGGCUAGAGCCGGC-CA | 181-A2-015 |
| 188 | D-RNA | GCGCGUAGUGAGAAACUACCAACGACUGGCUAGAGCCGGCGC | 181-A2-016 |
| 189 | D-RNA | GUGCGUAGUGAGAAACUACCAACGACUGGCUAGAGCCGGCAC | 181-A2-017 |
| 190 | D-RNA | GG-GCGUAGUGAGAAACUACCAACGACUGGCUAGAGCCGGCCC | 181-A2-018 |
| 191 | D-RNA | GAGCGUAGUGAGAAACUACCAACGACUGGCUAGAGCCGGCUC | 181-A2-019 |
| 192 | D-RNA | CGGCGUAGUGAGAAACUACCAACGACUGGCUAGAGCCGGCCG | 181-A2-020 |
| 193 | D-RNA | CCGCGUAGUGAGAAACUACCAACGACUGGCUAGAGCCGGCGG | 181-A2-021 |
| 194 | D-RNA | CAGCGUAGUGAGAAACUACCAACGACUGGCUAGAGCCGGCUG | 181-A2-022 |
| 195 | D-RNA | CUGCGUAGUGAGAAACUACCAACGACUGGCUAGAGCCGGCAG | 181-A2-023 |
| 196 | D-RNA | AGCGUGUUAGUGAAGUGGGUGGCAGGUAAAGGACACGCU | 184-B8trc |
| 197 | D-RNA | AGCGUGGUAGCGGUGUGGGUGGUAGGUAAAGGCCACGCU | 184-C6trc |
| 198 | D-RNA | AGCGUGAUAGAAGAGCGGGUGGUAGGUAAAGGUCAGGCU | 184-H5trc |
| 199 | D-RNA | AGCGUGUUAGGUAGGGUGGUAGUAAGUAAAGGACACGCU | 184-A7trc |
| 200 | D-RNA | AGCGUGUUAGGUGGGUGGUAGUAAGUAAAGGACACGCU | 187-A5trc |
| 201 | D-RNA | AGCGUGUUAGGUGGGUGGUAGUAAGUAAAGGGCACGCU | 187-H5trc |
| 202 | D-RNA | CCGCUUAGGUGGGUGGUAGUAAGUAAAGGGGCGG | 174-D4-004 |
| 203 | D-RNA | GCGCGAGCAGGUGGGUGGUAGAAUGUAAAGACUCGCGUC | 166-A4-002 |
| 204 | D-RNA | CGUGUUAGGUGGGUGGUAGUAAGUAAAGGACACG | 187-A5trc-001 |
| 205 | D-RNA | GUGUUAGGUGGGUGGUAGUAAGUAAAGGACAC | 187-A5trc-002 |
| 206 | D-RNA | CGUGUUAGGUGGGUGGUAGUAAGUAAAGGGCACG | 187-H5trc-002 |
| 207 | D-RNA | GUGUUAGGUGGGUGGUAGUAAGUAAAGGGCAC | 187-H5trc-003 |
| 208 | D-RNA | UGUUAGGUGGGUGGUAGUAAGUAAAGGGCA | 187-H5trc-004 |
| 209 | D-RNA | GGACGAGAGUGACAAAUGAUAUAACCUCCUGACUAACGCUGCGGGCGACAGG | 177-B3 |
| 210 | D-RNA | GGACCUAUCGCUAAGACAACGCGCAGUCUACGGGACAUUCUCCGCGGACAGG | 177-C1 |
| 211 | D-RNA | GGACAAUUGUUACCCCCGAGAGAGACAAAUGAGACAACCUCCUGAAGACAGG | 177-C2 |
| 212 | D-RNA | GGACGAAAGUGAGAAAUGAUACAACCUCCUGUUGCUGCGAAUCCGGACAGG | 177-E3 |
| 213 | D-RNA | GGACGUAAAAGACGCUACCCGAAAGAAUGUCAGGAGGGUAGACCGACAGG | 177-D1 |
| 214 | D-RNA | GGACUAGAAACUACAAUAGCGGCCAGUUGCACCGCGUUAUCAACGACAGG | 177-E1 |
| 215 | D-RNA | GGACUAGUCAGCCAGUGUGUAUAUCGGACGCGGGUUUAUUUACUGACAGG | 177-A1 |
| 216 | D-RNA | GGACUGUCCGGAGUGUGAAACUCCCCGAGACCGCCAGAAGCGGGGACAGG | 177-G3 |
| 217 | D-RNA | GGACUUCUAUCCAGGUGGGUGGUAGUAUGUAAAGAGAUAGAAGUGACAGG | 177-C3 |
| 218 | D-RNA | GGACGAGAGCGAACAAUGAUAUAACCUCCUGACGGAAAGAGAUCGACAGG | 177-A2 |
| 219 | D-RNA | CCUGUGCUACACGCAGUAAGAAGUGAACGUUCAGUAUGUGUGCACAGG | 170-E4trc |
| 220 | D-RNA | CGUGAGCCAGGCACCGAGGGCGUUAACUGGCUGAUUGGACACGACACG | 166-D2trc |
| 221 | D-RNA | CGUGAACAUGCAAGCUAAGCGGGGCUGUUGGUUGCUUGGCCCGCCACG | 174-A2trc |
| 222 | D-RNA | CGUGCAGAGAGAGACCAACCACGUAAAAUCAACCUAAUGGGCCGCACG | 174-E2trc |
| 223 | D-RNA | CGUGCAGAGAGAGACCAACCACGUAAAAUCAACCUAAUGGGCCGCACG | 183-G3trc |
| 224 | D-RNA | CGUGAACAUUCAAGCUAAGCGGGGCUGUUGGUUGCUUGGCCCGCCACG | 183-B2trc |
| 225 | D-RNA | CGUGCCGAGGCGGCGACCAGCGUUACUUAGAGAGGCUUUGGCACCACG | 166-B2trc |
| 226 | D-RNA | CGUGAUAACAGCCGUCGGUCAAGAAAACAAAGUUCGGGCGGCGCACG | 166-G3trc |
| 227 | D-RNA | CGUGGGUGGCGCACCGAGGGCGAAAAGCCACCAGUAAAGAUAGACCG | 166-D1trc |
| 228 | D-RNA | CGUGUGAUCUCCUUUGGGGUGAUUAGCUUAGAGACUUCCCACACG | 183-H2trc |
| 229 | D-RNA | GCACCUUCGCCUAAUACACGUGCCGGCUAGCUAAUACUCGUCCGC | 167-A7trc |
| 230 | D-RNA | GCACGACUUGGGCGACCAGUGAUACUUAGAGAGCAAGUCGUCGGC | 167-C7trc |
| 231 | D-RNA | GCGCGCGCUCAGUAAGAAAUUGAAAGUUCAGAAUGUCGUCGCGC | 167-B5trc |
| 232 | D-RNA | AGUGUGUGGCAGGCUAAGGAGAUAUUCCGAGACCACGCU | 184-D7trc |
| 233 | D-RNA | AGUGUGUGGCAGACUAUGGAUAGACUCCGAGACCACGCU | 184-D6trc |
| 234 | D-RNA | AGCGUGAGGCGACCAGCGGAUUACUUAGAGAGUCACGCU | 184-E5trc |
| 235 | D-RNA | AGCGUGAAGGGGACCAGCGUUACUUACAGAGUUCACGCU | 184-G6trc |
| 236 | D-RNA | AGCGUGUGAUGUAUGUAGCACCGUAUCAGAGGACACGCU | 184-B7trc |
| 237 | D-RNA | AGCGUGAGGCGACCCGUGUUUCGUAGAGAGUCACGCU | 184-B6trc |
| 238 | D-RNA | 5' PEG-GCACGUCCCUCACCGGUGCAAGUGAAGCCGUGGCUCUGCG | NOX-E36-5' PEG |
| 239 | D-RNA | GCACGUCCCUCACCGGUGCAAGUGAAGCCGUGGCUCUGCG-3'PEG | NOX-E36-3' PEG |
| 240 | D-RNA | GAGAUGGCGACAUUGGUUGGGCAUGAGGCGAGGCCCUUUGAUGAAUCCGCGGCCAUUC | 188-A3-001 |
| 241 | D-RNA | GAUGGCGACAUUGGUUGGGCAUGAGGCGAGGCCCUUUGAUGAAUCCGCGGCCAUUC | 188-A3-004 |
| 242 | D-RNA | GGCGACAUUGGUUGGGCAUGAGGCGAGGCCCUUUGAUGAAUCCGCGGCCAUUC | 188-A3-005 |
| 243 | D-RNA | GGCGACAUUGGUUGGGCAUGAGGCGAGGCCCUUUGAUGAAUCCGCGGCCAUU | 188-A3-006 |
| 244 | D-RNA | GGCGACAUUGGUUGGGCAUGAGGCGAGGCCCUUUGAUGAAUCCGCGGCCA | (D-)188-A3-007 = (D-) mNOX-E36 |
| 245 | D-RNA | GCUGGUUACCGAGGGGGCGUCGUUGGAGUUUGGUUGGUUGUCACCAGC | 189-G7-001 |
| 246 | D-RNA | CUGGUUACCGAGGGGGCGUCGUUGGAGUUUGGUUGGUUGUCACCAG | 189-G7-002 |
| 247 | D-RNA | UGGUUACCGAGGGGGCGUCGUUGGAGUUUGGUUGGUUGUCACCA | 189-G7-003 |
| 248 | D-RNA | GCCGGUUACCGAGGGGGCGUCGUUGGAGUUUGGUUGGUUGUCACCGGC | 189-G7-007 |
| 249 | D-RNA | GCCGGCUACCGAGGGGGCGUCGUUGGAGUUUGGUUGGUUGUCGCCGGC | 189-G7-008 |
| 250 | D-RNA | GCGCGUACCGAGGGGGCGUCGUUGGAGUUUGGUUGGUUGUCCGCGC | 189-G7-010 |
| 251 | D-RNA | GGGCCUACCGAGGGGGCGUCGUUGGAGUUUGGUUGGUUGUCGGCCC | 189-G7-012 |
| 252 | L-protein | QPDAVNAPLTCCYSFTGKMIPMSRLENYKRITSSRCPKEAVVFVTKLKREICADPNKEWVQ KYIRKLDQNQVRSET | rat MCP-1 |
| 253 | L-RNA | 5' PEG-GGCGACAUUGGUUGGGCAUGAGGCGAGGCCCUUUGAUGAAUCCGCGGCCA | mNOX-E36-5'PEG |
| 254 | L-RNA | GGCGACAUUGGUUGGGCAUGAGGCGAGGCCCUUUGAUGAAUCCGCGGCCA-3'PEG | mNOX-E36-3'PEG |
| 255 | L-DNA | 5'-GAGGGACGTGC-(Spacer18)₂-NH4⁺ -3' | NOX-E36 Capture probe |
| 256 | L-DNA | 5'- Biotin-(Spacer18)₂-CGCAGAGCC | NOX-E36 Detect (-ion) probe |
| 257 | L-Protein | KSMQVPFSRCCFSFAEQEIPLRAILCYRNTSSICSNEGLIFKLKRGKEACALDTVGWVQRH RKMLRHCPSKRK | CCL1/I-309 |
| 258 | L-Protein | SLAADTPTACCFSYTSRQIPQNFIADYFETSSQCSKPGVIFLTKRSRQVCADPSEEWVQKY VSDLELSA | CCL3/MIP-1α |
| 259 | L-Protein | APMGSDPPTACCFSYTARKLPRNFVVDYYETSSLCSQPAVVFQTKRSKQVCADPSESWVQE YVYDLELN | CCL4/MIP-1β |
| 260 | L-Protein | SPYSSDTTPCCFAYIARPLPRAHIKEYFYTSGKCSNPAVVFVTRKNRQVCANPEKKWVREY INSLEMS | CCL5/RANTES |
| 261 | L-Protein | FNPQGLAQPDALNVPSTCCFTFSSKKISLQRLKSYVITTSRCPQKAVIFRTKLGKEICADP KEKWVQNYMKHLGRKAHTLKT | CCL13/MCP-4 |
| 262 | L-Protein | TKTESSSRGPYHPSECCFTYTTYKIPRQRIMDYYETNSQCSKPGIVFITKRGHSVCTNPSD KWVQDYIKDMKEN | CCL14/HCC-1 |
| 263 | L-Protein | ASVATELRCQCLQTLQGIHPKNIQSVNVKSPGPHCAQTEVIATLKNGRKACLNPASPIVKK IIEKMLNSDKSN | CXCL1/GROα |
| 264 | L-Protein | APLATELRCQCLQTLQGIHLKNIQSVKVKSPGPHCAQTEVIATLKNGQKACLNPASPMVKK IIEKMLKNGKSN | CXCL2/GROβ |
| 265 | L-Protein | ASVVTELRCQCLQTLQGIHLKNIQSVNVRSPGPHCAQTEVIATLKNGKKACLNPASPMVQK IIEKILNKGSTN | CXCL3/GROγ |
| 266 | L-Protein | EAEEDGDLQCLCVKTTSQVRPRHITSLEVIKAGPHCPTAQLIATLKNGRKICLDLQAPLYK KIIKKLLES | CXCL4/PF4 |
| 267 | L-Protein | GPAAAVLRELRCVCLQTTQGVHPKMISNLQVFAIGPQCSKVEVVASLKNGKEICLDPEAPFL KKVIQKILDGGNKEN | CXCL5/ENA-78 |
| 268 | L-Protein | GPVSAVLTELRCTCLRVTLRVNPKTIGKLQVFPAGPQCSKVEVVASLKNGKQVCLDPEAPFL KKVIQKILDSGNKKN | CXCL6/GCP-2 |
| 269 | L-Protein | SSTKGQTKRNLAKGKEESLDSDLYAELRCMCIKTTSGIHPKNIQSLEVIGKGTHCNQVEVIA TLKDGRKICLDPDAPRIKKIVQKKLAGDESAD | CXCL7/NAP-2 |
| 270 | L-Protein | EGAVLPRSAKELRCQCIKTYSKPFHPKFIKELRVIESGPHCANTEIIVKLSDGRELCLDPKE NWVQRVVEKFLKRAENS | CXCL8/IL-8 |
| 271 | L-Protein | TPVVRKGRCSCISTNQGTIHLQSLKDLKQFAPSPSCEKIEIIATLKNGVQTCLNPDSADVKE | CXCL9/MIG |
| | | LIKKWEKQVSQKKKQKNGKKHQKKKVLKVRKSQRSRQKKTT | |
| 272 | L-Protein | VPLSRTVRCTCISISNQPVNPRSLEKLEIIPASQFCPRVEIIATMKKKGEKRCLNPESKAIK NLLKAVSKERSKRSP | CXCL10/IP-10 |
| 273 | L-Protein | FPMFKRGRCLCIGPGVKAVKVADIEKASIMYPSNNCDKIEVIITLKENKGQRCLNPKSKQAR LIIKKVERKNF | CXCL11/I-TAC |
| 274 | L-Protein | KPVSLSYRCPCRFFESHVARANVKHLKILNTPNCALQIVARLKNNNRQVCIDPKLKWIQEYL EKALNKRFKM | CXCL12α/SDF-1α |
| 275 | L-Protein | KPVSLSYRCPCRFFESHVARANVKHLKILNTPNCALQIVARLKNNNRQVCIDPKLKWIQEYL EKALNKRFKM | CXCL12β/SDF-1β |
| 276 | L-Protein | QHHGVTKCNITCSKMTSKIPVALLIHYQQNQASCGKRAIILETRQHRLFCADPKEQWVKDAM QHLDRQAAALTRNG | CX₃CL1/Fractalkine |
| 277 | L-Protein | VGSEVSDKRTCVSLTTQRLPVSRIKTYTITEGSLRAVIFITKRGLKVCADPQATWVRDVVRS MDRKSNTRNNMIQTKPTGTQQSTNTAVTLTG | XCL1/Lymphotactin |
| 278 | L-RNA | 5'-Biotin-GCACGUCCCUCACCGGUGCAAGUGAAGCCGUGGCUCUGCG | biotinylated NOX-E36 |
| 279 | L-RNA | 5'-UAAGGAAACUCGGUCUGAUGCGGUAGCGCUGUGCAGAGCU | POC |
| 280 | L-RNA | 5'-PEG-UAAGGAAACUCGGUCUGAUGCGGUAGCGCUGUGCAGAGCU-3' | POC-PEG |
| 281 | L-DNA | 5'-CCAATGTCGCC-(Spacer18)₂-NH4⁺ -3' | mNOX-E36 Capture probe |
| 282 | L-DNA | 5'- Biotin-(Spacer18)₂-CGCAGAGCC | mNOX-E36 Detect (-ion) probe |
| 283 | L-protein | QPDAINSPVTCCYTFTGKKISSQRLGSYKRVTSSKCPKEAVIFKTILAKEICADPEQKWVQ DAVKQLDKKAQTPKP | horse MCP-1 (Equus caballus) |
| 284 | L-protein | QPDAINSQVACCYTFNSKKISMQRLMNYRRVTSSKCPKEAVIFKTILGKELCADPKQKWVQ DSINYLNKKNQTPKP | bovine MCP-1 (Bos Taurus) |
| 285 | L-protein | QPDAVNAPLTCCYSFTGKMIPMSRLENYKRITSSRCPKEAVVFVTKLKREICADPNKEWVQ KYIRKLDQNQVRSETTVFYKIASTLRTSAPLNVNLTHKSEANASTLFSTTTSSTSVEVTSM TEN | rat MCP-1 (Rattus norvegicus) |
| 286 | L-protein | MLSTSRSRFIRNTNESGEEVTTFFDYDYGAPCHKFDVKQIGAQLLPPLYSLVFIFGFVGNM LVVLILINCKKLKCLTDIYLLNLAISDLLFLITLPLWAHSAANEWVFGNAMCKLFTGLYHI GYFGGIFFIILLTIDRYLAIVHAVFALKARTVTFGVVTSVITWLVAVFASVPGIIFTKCQK EDSVYVCGPYFPRGWNNFHTIMRNILGLVLPLLIMVICYSGILKTLLRCRNEKKRHRAVRV IFTIMIVYFLFWTPYNIVILLNTFQEFFGLSNCESTSQLDQATQVTETLGMTHCCINPIIY AFVGEKFRSLFHIALGCRIAPLQKPVCGGPGVRPGKNVKVTTQGLLDGRGKGKSIGRAPEA SLQDKEGA | Human C-C chemokine receptor type 2 |
| 287 | D-DNA | CACCCGCGAGCACAGCTTCT | mβ-actin forward primer |
| 288 | D-DNA | CGTTGTCGACGACCAGCGCA | mβ-actin reverse primer |
| 289 | D-DNA | TGGAGCAACATGTGGAACTC | mTGFb1 forward primer |
| 290 | D-DNA | GTCAGCAGCCGGTTACCA | mTGFb1 reverse primer |
| 291 | D-DNA | CCACATCTCCTGCTAATGTTG | mTGFb2 forward primer |
| 292 | D-DNA | AGTAGGCAGCATCCAAAGC | mTGFb2 reverse primer |

The invention is described in the following Figures and Examples in more detail from which further embodiment, features and advantages may be taken.

In connection therewith,
Fig. 1 shows a Western blot of fibrotic proteins in mouse conjunctival bleb tissue 7 days post GFS;
Fig. 2 shows two bar diagrams illustrating densitometric quantification of fibrotic proteins in mouse conjunctival bleb tissue 7 days post GFS; data are presented as the mean ± s.e.m. Significance was determined by one-way ANOVA followed by Dunnett's post-hoc test of n≥3 pooled mice tissues; *, p < 0.05, **, p< 0.01; density was determined either as FN1/GAPDH (Fig. 2A) or as COL1A1/GAPDH (Fig. 2B) for control, subconjunctival mNOX-E36 and MMC;
Fig. 3 shows two bar diagrams illustrating the result of qRT-PCR analysis of mRNA expression of TGFβ genes in mouse conjunctival bleb tissue 7 days post GFS. Data are presented as the mean ± s.e.m, significance was determined by one-way ANOVA followed by Dunnett's post-hoc test of n≥3 pooled mice tissues; *, p < 0.05, ***, p< 0.001; relative mRNA expression was determined based on Tgfb2 (Fig. 3A) or Tgfb1 (Fig. 3B) for control, subconjunctival mNOX-E36 and MMC;
Fig. 4 shows representative slit lamp images (Fig. 4A), and a bar diagram indicating relative vascularity expressed as % area for subconjunctival mNOX-E36 and MMC (Fig. 4B), whereby the bar diagram illustrates the result of quantitative analysis of vascular bleb area (indicated by dotted boundary) in subconjunctival mNOX-E36 or MMC treated groups 7 days post-GFS.; data are presented as the mean ± s.e.m. Significance was determined by unpaired, two-tailed Student's t-test of n ≥ 3 pooled mice tissues; *, p < 0.05;
Fig. 5 shows a Western blot of fibrotic proteins in mouse conjunctival bleb tissue 7 days post GFS.
Fig. 6 shows two bar diagrams illustrating densitometric quantification of fibrotic proteins in mouse conjunctival bleb tissue 7 days post GFS; density was determined either as FN1/GAPDH (Fig. 6A) or as COL1/GAPDH (Fig. 6B) for subconjunctival administration of NOX-E36 plus low MMC, subconjunctival and subcutaneous administration of NOX-E36 plus low MMC, subconjunctival and two times subcutaneous administration of NOX-E36 plus low MMC, and administration of standard MMC;

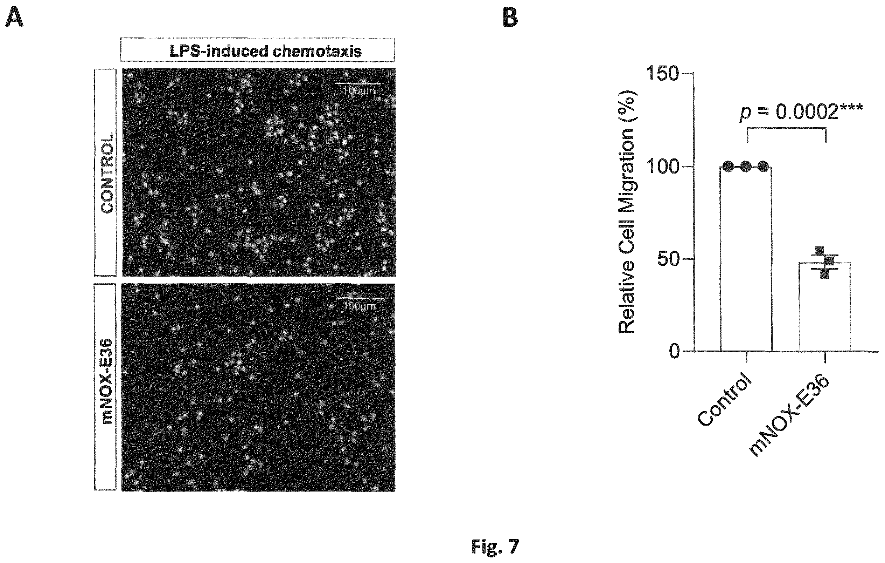

Fig. 7 shows a microphotograph (Fig. 7A) and a bar diagram (Fig. 7B) presenting the result of an analysis of LPS-induced migrated RAW 264.7 macrophages upon mNOX-E36 treatment; Data are presented as the mean ± s.e.m; n≥3. Significance was determined by one-way ANOVA followed by Sidak's post-hoc test. ***, p > 0.001; the upper part of the microphotograph in Fig. 7A shows LPS-induced chemotaxis for the control i.e. with NOX-E36, whereas the lower part of the microphotograph in Fig. 7B shows LPS-induced chemotaxis upon exposure of the cells to NOX-E36; the bar diagram shows relative cell migration in percent for cells exposed to mNOX-E36 ("mNOX-E36") and cells not exposed to mNOX-E36 ("Control");
Fig. 8 shows a Western blot of fibrotic proteins in mouse conjunctival fibroblasts following treatment with conditioned media from mNOX-E36 and/or LPS-treated RAW 264.7 macrophages;
Fig. 9 shows three bar diagrams illustrating densitometric quantification of fibrotic proteins for fibronectin 1 (Fig. 9A), αSMA (Fig. 9B) and CTGF (Fig. 9C) in mouse conjunctival fibroblasts following treatment with conditioned media from mNOX-E36 and/or LPS-treated RAW 264.7; Data are presented as the mean ± s.e.m; n≥3. Significance was determined by one-way ANOVA followed by Sidak's post-hoc test. *, p < 0.05; **, p < 0.01; ***, p < 0.001;
Fig. 10 shows a Western blot showing levels of fibrotic protein COL1A1 and CTGF in mouse conjunctival fibroblasts following treatment with CCL2 (MCP-1) and/or mNOX-E36;
Fig. 11 shows two diagrams illustrating the result of densitometry analysis of Western blot showing levels of fibrotic protein CTGF (Fig. 11A) and COL1A1 (Fig. 11B) in mouse conjunctival fibroblasts following treatment with CCL2 (MCP-1) and/or mNOX-E36; data are presented as the mean ± s.e.m; n≥3. Significance was determined by one-way ANOVA followed by Sidak's post-hoc test. *, p < 0.05; **, p < 0.01; ***, p < 0.001;
Fig. 12 shows a Western blot and densitometry analysis quantifying levels of fibrotic protein COL1A1 and CTGF in mouse conjunctival fibroblasts following treatment with TGFβ2 and/or mNOX-E36;
Fig. 13 shows two diagrams illustrating the result of densitometry analysis of Western blot showing levels of fibrotic protein COL1A1 (Fig. 13A) and CTGF (Fig. 13B) in mouse conjunctival fibroblasts following treatment with TGFβ2 and/or mNOX-E36; data are presented as the mean ± s.e.m; n≥3. Significance was determined by one-way ANOVA followed by Sidak's post-hoc test. *, p < 0.05; **, p < 0.01; ***, p < 0.001;
Fig. 14 shows an alignment of sequences of related RNA ligands binding to human MCP-1 indicating the sequence motif ("Type 1A") that is in a preferred embodiment in its entirety essential for binding to human MCP-1;
Fig. 15 shows an alignment of sequences of related RNA ligands binding to human MCP-1 indicating the sequence motif ("Type 1B") that is in a preferred embodiment in its entirety essential for binding to human MCP-1 and derivatives of RNA ligands 180-D1-002;
Fig. 16 shows an alignment of sequences of related RNA ligands binding to human MCP-1 indicating the sequence motif ("Type 2") that is in a preferred embodiment in its entirety essential for binding to human MCP-1;
Fig. 17 shows an alignment of sequences of related RNA ligands binding to human MCP-1 indicating the sequence motif ("Type 3") that is in a preferred embodiment in its entirety essential for binding to human MCP-1;
Fig. 18 shows derivatives of RNA ligands 178-D5 and 181-A2 (human MCP-1 RNA ligands of sequence motif "Type 3");
Fig. 19 shows an alignment of sequences of related RNA ligands binding to human MCP-1 indicating the sequence motif ("Type 4") that is in a preferred embodiment in its entirety essential for binding to human MCP-1 (other sequences);
Fig. 20 shows a table of sequences of several different RNA ligands binding to human MCP-1 which cannot be related to the MCP-1 binding sequence motifs "Type 1A", "Type 1B"; "Type 2", "Type 3" or "Type 4";
Fig. 21 shows alignments of derivatives of RNA ligand 188-A3-001 and of 189-G7-001 that bind to murine MCP-1;
Fig. 22 shows the result of a binding analysis of the aptamer D-NOX-E36 to biotinylated human D-MCP-1 at room temperature and 37°C, represented as binding of the aptamer over concentration of biotinylated human D-MCP-1 ;
Fig. 23 shows the result of a binding analysis of the aptamer D-mNOX-E36 to biotinylated murine D-MCP-1 at 37°C, represented as binding of the aptamer over concentration of biotinylated murine D-MCP-1;
Fig. 24 shows MCP-1-induced Ca⁺⁺-release in THP-1 cells, whereas a dose-response curve for human MCP-1 was obtained, indicating a half effective concentration (EC₅₀) of approximately 3 nM, represented as difference in fluorescence to blank over concentration of human MCP-1;
Fig. 25 shows the efficacy of Spiegelmer NOX-E36 in a calcium release assay; cells were stimulated with 3 nM human MCP-1 preincubated at 37°C with various amounts of Spiegelmer NOX-E36, represented as percentage of control over concentration of NOX-E36;
Fig. 26 shows the efficacy of Spiegelmer mNOX-E36 in a calcium release assay; cells were stimulated with 5 nM murine MCP-1 preincubated at 37°C with various amounts of Spiegelmer mNOX-E36, represented as percentage of control over concentration of mNOX-E36;
Fig. 27 shows the human MCP-1-induced chemotaxis of THP-1 cells whereas after 3 hours migration of THP-1 cells towards various MCP-1 concentrations a dose-response curve for MCP-1 was obtained, represented as X-fold increase compared to control over concentration of human MCP-1;
Fig. 28 shows the efficacy of Spiegelmer NOX-E36 in a chemotaxis assay; cells were allowed to migrate towards 0.5 nM human MCP-1 preincubated at 37°C with various amounts of Spiegelmer NOX-E36, represented as percentage of control over concentration of Spiegelmer NOX-E36;
Fig. 29 shows the efficacy of Spiegelmer mNOX-E36 in a chemotaxis assay; cells were allowed to migrate towards 0.5 nM murine MCP-1 preincubated at 37°C with various amounts of Spiegelmer NOX-E36, represented as percentage of control over concentration of Spiegelmer mNOX-E36;
Fig. 30 shows the Biacore 2000 sensorgram indicating the K_{D} value of Spiegelmer NOX-E-36 binding to human MCP-1 which was immobilized on a PioneerF1 sensor chip by amine coupling procedure, represented as response (RU) over time;
Fig. 31 shows the Biacore 2000 sensorgram indicating binding of Spiegelmer NOX-E36 to human MCP-family proteins (huMCP-1, huMCP-2, huMCP-3) and human eotaxin, which were immobilized by amine coupling procedure on a PioneerF1 and a CM4 sensor chip, respectively, represented as response (RU) over time;
Fig. 32 shows the Biacore 2000 sensorgram indicating binding of Spiegelmer NOX-E36 to MCP-1 from different species (canine MCP-1, monkey MCP-1, human MCP-1, porcine MCP-1, rabbit MCP-1, mouse MCP-1, rat MCP-1) whereas different forms of MCP-1 were immobilized by amine coupling procedure on PioneerF1 and a CM4 sensor chips, respectively, represented as response (RU) over time;
Fig. 33 shows the Biacore 2000 sensorgram indicating the K_{D} value of Spiegelmer 181-A2-018 binding to to human MCP-1 which was immobilized on a CM4 sensor Chip by amine coupling procedure, represented as response (RU) over time;
Fig. 34 shows the Biacore 2000 sensorgram indicating binding of Spiegelmer 181-A2-018 to human MCP-family proteins (huMCP-1, huMCP-2, huMCP-3) and human eotaxin which were immobilized by amine coupling procedure on a PioneerF1 and a CM4 sensor chip, respectively, represented as response (RU) over time;
Fig. 35 shows the Biacore 2000 sensorgram indicating binding of Spiegelmer 181-A2-018 to MCP-1 from different species (canine MCP-1, monkey MCP-1, human MCP-1, porcine MCP-1, rabbit MCP-1, mouse MCP-1, rat MCP-1) whereas different forms of MCP-1 were immobilized by amine coupling procedure on PioneerF1 and a CM4 sensor chips, respectively, represented as response (RU) over time;
Fig. 36 shows a Clustal W alignment of MCP-1 from different mammalian species as well as human MCP-2, MCP-3, and eotaxin (Positions 1-76 only);
Fig. 37A shows a table summarizing the binding specificity of NOX-E36 and 181-A2-018 regarding MCP-1 from different mammalian species as well as human MCP-2, MCP-3, and eotaxin;
Fig. 37B shows a table summarizing the selectivity of NOX-E36 as determined by Biacore analysis whereby biotinylated NOX-E36 was immobilized on a sensor chip surface and binding of a panel of various CC and CXC chemokines to NOX-E36 was analyzed;
Fig. 37C shows the kinetic analysis of NOX-E36 interacting with chemokines as determined by Biacore analysis whereby the chemokines were immobilized covalently on a CM5 sensor chip surface and various concentrations of the NOX-E36 were injected and NOX-E36s binding behaviour was analyzed using the BiaEvaluation software;
Fig. 37D shows the chemotaxis dose-response curve of THP-1 cell stimulation with MIP-1α with a half- effective concentration of about 0.2 nM;
Fig. 37E shows the Inhibition of MIP-1α induced chemotaxis by NOX-E36. NOX-E36 had no influence on the MIPla induced chemotaxis of THP-1 cells;
Fig. 38 shows the efficacy of Spiegelmer NOX-E36-3'-PEG in a calcium release assay; cells were stimulated with 3 nM human MCP-1 preincubated at 37°C with various amounts of Spiegelmer NOX-E36-3'-PEG, represented as percentage of control over concentration of Spiegelmer NOX-E36-3'-PEG;
Fig. 39 shows the efficacy of Spiegelmer NOX-E36-3'-PEG in a chemotaxis assay; cells were allowed to migrate towards 0.5 nM human MCP-1 preincubated at 37°C with various amounts of Spiegelmer NOX-E36-3'-PEG, represented as percentage of control over concentration of NOX-E36-3'-PEG;
Fig. 40A shows the efficacy of Spiegelmer NOX-E36-5'-PEG in a calcium release assay; cells were stimulated with 3 nM human MCP-1 preincubated at 37°C with various amounts of Spiegelmer NOX-E36-5'-PEG, represented as percentage of control over concentration of Spiegelmer NOX-E36-5'-PEG;
Fig. 40B shows the efficacy of Spiegelmer NOX-E36-5'-PEG in a chemotaxis assay; cells were allowed to migrate towards 0.5 nM human MCP-1 preincubated at 37°C with various amounts of Spiegelmer NOX-E36-5'-PEG, represented as percentage of control over concentration of Spiegelmer NOX-E36-5'-PEG;
Fig. 41 shows murine MCP-1-induced Ca⁺⁺-release in THP-1 cells, whereas a dose-response curve for murine MCP-1 was obtained, indicating a half effective concentration (EC₅₀) of approximately 5 nM, represented as difference in fluorescence to blank over concentration of murine MCP-1;
Fig. 42 shows the efficacy of anti-murine MCP-1 Spiegelmer mNOX-E36-3'-PEG in a calcium release assay; cells were stimulated with 3 nM murine MCP-1 preincubated at 37°C with various amounts of Spiegelmer mNOX-E36-3'-PEG, represented as percentage of control over concentration of Spiegelmer mNOX-E36-3'-PEG;
Fig. 43 shows the murine MCP-1-induced chemotaxis of THP-1 cells whereas after 3 hours migration of THP-1 cells towards various mMCP-1 concentrations a dose-response curve for mMCP-1 was obtained, represented as X-fold increase compared to control over concentration of murine MCP-1;
Fig. 44 shows the efficacy of anti-murine MCP-1 Spiegelmer mNOX-E36-3'-PEG in a chemotaxis assay; cells were allowed to migrate towards 0.5 nM murine MCP-1 preincubated at 37°C with various amounts of Spiegelmer mNOX-E36-3'-PEG, represented as percentage of control over concentration of anti-murine Spiegelmer mNOX-E36-3'-PEG;
Fig. 45 shows the Biacore 2000 sensorgram indicating the K_{D} value of aptamer D-mNOX-E36 binding to murine D-MCP-1 which was immobilized on a PioneerF1 sensor chip by amine coupling procedure, represented as response (RU) over time; and
Fig. 46 shows the Biacore 2000 sensorgram indicating binding of aptamer D-mNOX-E36 to human D-MCP-1 and murine D-MCP-1 whereas the two different forms of D-MCP-1 were immobilized by amine coupling procedure on PioneerF1 and a CM4 sensor chips, respectively, represented as response (RU) over time.

### Example 1: Effect of pharmacological inhibition of chemokine CCL2 with pegylated Spiegelmer mNOX-E36 in a mouse model of glaucoma filtration surgery in comparison with mitomycin C

**Summary:** CCL2 (monocyte chemoattractant protein-1, MCP-1), a potent recruiter of monocytes, is increased in tear fluid of glaucoma patients with greater propensity to scar. NOX-E36 is an anti-CCL2 L-RNA aptamer that has completed a Phase 2 clinical trial in patients with diabetes mellitus and albuminuria. Its murine-specific analogue, the L-RNA aptamer mNOX-E36, was previously shown to attenuate glomerulosclerosis and liver fibrosis in mice. Bleb scarring represents the biggest risk of the failure of glaucoma filtration surgery (GFS), a commonly procedure to reduce ocular pressure in patients with glaucoma. The advent of anti-fibrotic agents, such as mitomycin C (MMC) are often used to prolong the longevity of the bleb. However, the cytotoxic effect of MMC raise significant concerns. This set of experiments compared the efficacy of mNOX-E36 against the anti-metabolite MMC on post-operative fibrosis in a murine model of GFS.

GFS was performed on C57BL/6 mice, followed by subconjunctival mNOX-E36 administration or MMC treatment. The blebs were harvested for immunofluorescence staining, Western blotting and real-time polymerase chain reaction analysis. It was found that subconjunctival mNOX-E36 treatment exhibits comparable anti-fibrotic activity to MMC in inhibiting fibrosis following GFS while preserving bleb health as assessed by the presence of conjunctival vasculature. The results demonstrate the efficacy of mNOX-E36 compared to clinical standard of care, MMC, in attenuating post-operative inflammation and fibrosis following GFS. NOX-E36 shows a promise as a non-toxic anti-fibrotic adjunctive treatment in GFS, thus an alternative to MMC.

### MATERIALS AND METHODS

***In vivo* study design - treatment arms:** Administration of mNOX-E36 and the current gold standard antifibrotic agent MMC was compared, with experiments comprising three treatment arms:
1. Control group: Subconjunctival application of surgical sponges soaked in sterile water and 5 µL subconjunctival injection of 10 mg/mL inactive control revmNOX-E36
2. mNOX-E36 group: Subconjunctival application of surgical sponges soaked in sterile water and 5 µL subconjunctival injection of 10 mg/mL anti-CCL2 Spiegelmer mNOX-E36
3. MMC group: Subconjunctival application of surgical sponges soaked in 0.4 mg/mL MMC (Kyowa Hakko Kirin Co. Ltd, Japan) and 5 µL subconjunctival injection of 10 mg/mL inactive control revmNOX-E36
For all three treatment arms, application of surgical sponges was performed during GFS surgery prior to subconjunctival closure, and subconjunctival injections were performed immediately after the GFS and 24 hours post-operation.

**Mouse model of Glaucoma Filtration Surgery:** Ethical approval was obtained from the SingHealth Institutional Animal Care and Use Committee (IACUC). C57BL/6 mice were bred and treated in accordance with the Association for Research in Vision and Ophthalmology (ARVO) Statement on the Use of Animals in Ophthalmic and Vision Research. The mouse model of GFS is described in Seet et al. 2011 (Seet, Lee et al. 2011) - in brief, mice were anaesthetised via intraperitoneal injection of a ketamine/xylazine cocktail consisting 20 mg/mL ketamine (Troy Laboratories, Australia) and 2 mg/mL xylazine (Parnell Laboratories, Australia). The conjunctiva in one eye was dissected superiorly to expose bare sclera, and a 30-gauge sterile needle was passed into the anterior chamber to form a fistula, allowing aqueous egress into the subconjunctival space. Surgical sponges (Inami, Japan) soaked with the appropriate treatment groups were applied to the subconjunctival space for 2 minutes, then the dissected conjunctiva was closed over the fistula with 10-0 nylon (Ethicon Inc, USA). Following which, subconjunctival and subcutaneous injections of experimental drugs were performed and local fucithalmic ointment (Leo Pharmaceutical Products, Denmark) was instilled at the end of the surgery. The contralateral unoperated eye was used as a baseline for comparison. Animals were euthanized on Day 7 (D7) post-surgery for downstream analysis.

**Histology:** Mouse eyes were enucleated, fixed in 4% paraformaldehyde and and then placed in a slurry of optimal cutting temperature (OCT) compound in cryomold before freezing in dry ice and storage in a -80 °C freezer until ready for sectioning using the Microm HM550 (Carl Zeiss Ltd) (Seet, Chu et al. 2020). 5 µm thick sections were prepared for immunofluorescent microscopy to visualize tissue morphology. Antibodies specific to fibronectin (FN1, Abcam, UK) and collagen 1α1 (COL1A1, Abnova Corp, USA) were used and identified with AlexaFluro-488 or AlexaFluro-594 (Invitrogen, USA) conjugated secondary antibodies. Sections were counterstained with DAPI (Invitrogen, USA) and visualized using Zeiss Imager.Z1 microscope (Carl Zeiss Microimaging GmbH, Germany) or Nikon N-STORM with Andor CSU-W1 spinning disk (Nikon, Japan).

**Western blotting:** Western blot analysis was performed to assess the extent of fibrosis. Bleb tissues from 5 operated eyes were harvested D7 post-surgery and pooled (i.e., taken as n=1). Tissues or cells were processed in lysis buffer complete with protease inhibitors and the resulting lysate loaded onto SDS-polyacrylamide gel for electrophoresis and immunoblotting. The following antibodies were used for detection: anti-fibronectin (Abcam, UK), anti-collagen 1α1 (Abnova Corp, USA), and anti-GAPDH (Santa Cruz Biotechnology, USA) and corresponding horseradish peroxidase (HRP)-conjugated secondary antibodies (Jackson ImmunoResearch Laboratories, USA). Densitometric quantitation was performed, with variations in loading corrected to levels of housekeeping protein GAPDH.

**Real-time quantitative Polymerase Chain Reaction (RT-qPCR):** Bleb tissues were harvested D7 post-surgery and tissues from 3 operated eyes were pooled per treatment arm (i.e., taken as n=1), while contralateral unoperated conjunctiva were harvested as control. Tissues were stored in RNAlater solution (Thermo Fisher Scientific, USA) before being processed for qPCR. All reactions were performed in triplicate and measured as CT threshold levels normalized to housekeeping gene.

The following primers were used:
- mβ-actin forward - CACCCGCGAGCACAGCTTCT;
- mβ-actin reverse - CGTTGTCGACGACCAGCGCA;
- mTGFb1 forward - TGGAGCAACATGTGGAACTC;
- mTGFb1 reverse - GTCAGCAGCCGGTTACCA;
- mTGFb2 forward - CCACATCTCCTGCTAATGTTG;
- mTGFb2 reverse - AGTAGGCAGCATCCAAAGC

**Assessment of bleb vascularity:** Vascularity of blebs was quantified using ImageJ software (USA). In brief, the area of conjunctival vascularization around the surgical site was determined and normalized to total area of conjunctival tissue superior to the limbus.

Statistical analysis: Data are presented as mean ± standard error of mean (s.e.m.). Statistical analyses were performed by an unpaired, two-tailed Student's t-test or one-way ANOVA followed by appropriate post-hoc test using GraphPad Prism. Statistical significance was defined as p-values < 0.05.

### RESULTS

The anti-fibrotic effect of subconjunctival injection of mNOX-E36 was compared to the standard of care, mitomycin C (MMC). MMC administration prolongs bleb survival in part by inhibiting fibroblast proliferation and/or causing fibroblast toxicity, thereby reducing post-operative scarring (Fan Gaskin, Nguyen et al. 2014). Due to its non-specific nature, MMC also targets other cell types including microvascular endothelial cells, thereby inhibiting angiogenesis (Sherwood 2016). Slit-lamp and anterior segment optical coherence tomography imaging at D7 post-GFS revealed that compared to the control group, both subconjunctival mNOX-E36 and MMC treated groups maintained raised, diffuse blebs.

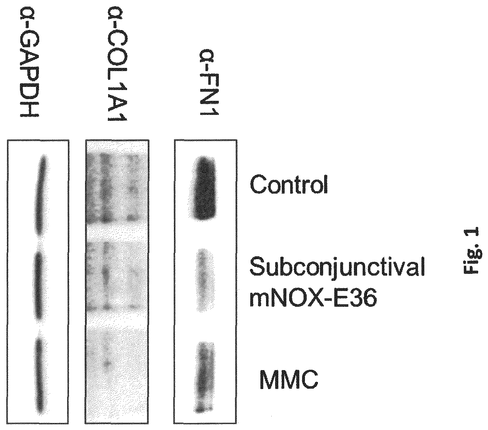

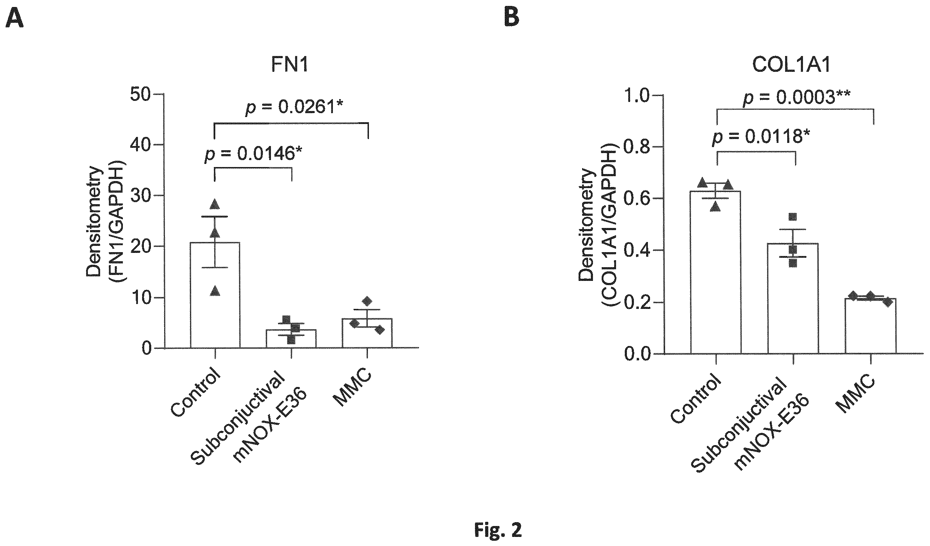

Analysis of bleb histology via immunofluorescence staining demonstrated a reduction in FN1 and COL1A1 deposition in both subconjunctival mNOX-E36 and MMC-treated groups compared to controls. Quantification of levels of fibrotic proteins FN1 and COL1A1 in mouse conjunctival bleb tissues on Day 7 post-GFS by Western blot and densitometry showed that both proteins were significantly downregulated in both arms compared to control **(****Figs. 1** **and** **2****)**.

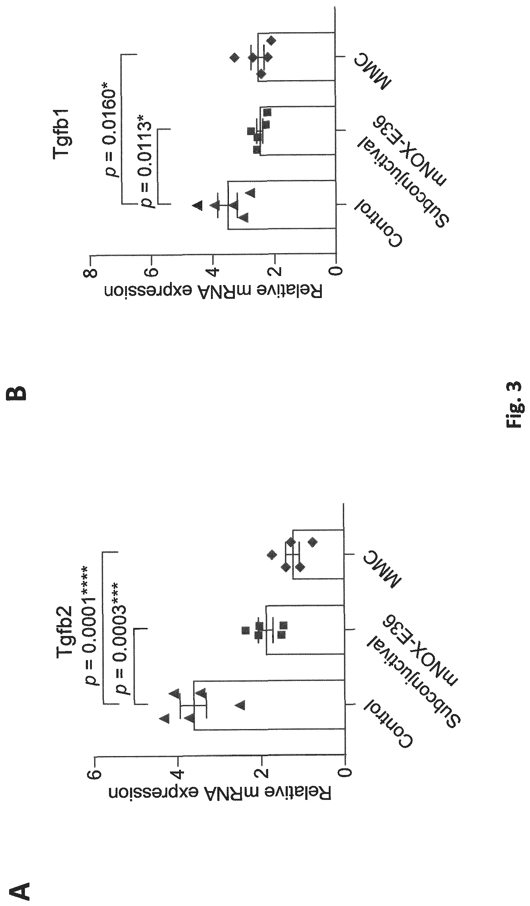

Transforming growth factor-beta (TGFβ) is a well-known pro-fibrotic cytokine and potent inducer of fibroblast activation and ECM production. TGFβ-2, the predominant isoform present in the eye, and TGFβ-1, both contribute to post-operative conjunctiva scarring (Shao, Sinha et al. 2023). To investigate the underlying molecular mechanism for the reduced fibrosis, quantitative PCR was performed on conjunctival bleb tissues D7 post-GFS. Results indicate a substantial reduction in gene expression of both *Tgfb2* and *Tgfb1* following treatment with either mNOX-E36 or MMC **(****Fig. 3****)**. Taken together, the data provides compelling evidence that subconjunctival mNOX-E36 is comparable to MMC in modulating fibrosis after GFS in this model.

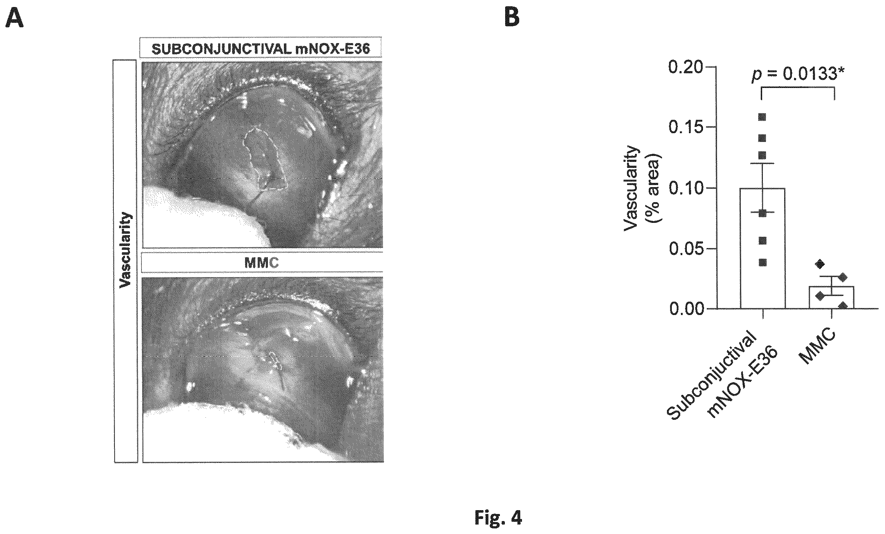

MMC administration prolongs bleb survival in part by inhibiting fibroblast proliferation and/or causing fibroblast toxicity, thereby reducing post-operative scarring. However, due to its non-specific nature, MMC also targets other cell types including microvascular endothelial cells, thereby inhibiting angiogenesis and increasing the risk of avascular bleb formation and subsequent bleb failure. Importantly, quantitative analysis of vascularity around the surgical site suggested that MMC-treated blebs had an avascular appearance, which was not noted in mNOX-E36 treated blebs **(****Fig. 4****)**.

### CONCLUSION

Results suggest a comparable inhibitory effect of mNOX-E36 and standard of care, MMC on post-operative fibrotic cytokine production and ECM deposition. Eyes treated with subconjunctival mNOX-E36 demonstrated noticeably more healthy blebs with normal conjunctival vascularity compared to MMC treated blebs. Development of large avascular blebs following GFS is associated with adverse events such as spontaneous leakage and transconjunctival oozing (Anand, Arora et al. 2006). Therefore, a healthy bleb morphology should preferably maintain some vascularity while avoiding excessive pathological neovascularization (Grover, Kornmann et al. 2020). Previous *in-vitro* studies have shown that blockade of the CCL2/CCR2 axis is less cytotoxic compared to MMC (Chong, Lee et al. 2017). This points to the potential of CCL2 inhibition via NOX-E36 in reducing post-operative inflammation and fibrosis in human patients following GFS, while avoiding the pitfalls associated with MMC.

### Example 2: Combination of systemic and local administration of CCL2 inhibiting Spiegelmer mNOX-E36 in a mouse model of glaucoma filtration surgery

**Summary:** CCL2 (monocyte-chemoattractant protein 1, MCP-1), a potent recruiter of monocytes, is increased in tear fluid of glaucoma patients with greater propensity to scar. NOX-E36 is an anti-CCL2 L-RNA aptamer that has completed a Phase 2 clinical trial in patients with diabetes mellitus and albuminuria. Its murine-specific analogue, the L-RNA aptamer mNOX-E36, was previously shown to attenuate glomerulosclerosis and liver fibrosis in mice. Bleb scarring represents the biggest risk of the failure of glaucoma filtration surgery (GFS), a commonly procedure to reduce ocular pressure in patients with glaucoma. The advent of anti-fibrotic agents, such as mitomycin C (MMC) are often used to prolong the longevity of the bleb. However, the cytotoxic effect of MMC raise significant concerns. This study compared the efficacy of combined systemic (subcutaneous, SC) and local (subconjunctival) administration of mNOX-E36 and low-dose MMC against only local administration of mNOX-E36 and low-dose MMC on post-operative fibrosis in a murine model of GFS.

GFS was performed on C57BL/6 mice, followed by subcutaneous + subconjunctival or only subconjunctival mNOX-E36 administration in addition to low-dose MMC treatment. The blebs were harvested for Western blotting analysis. It was found that coadministration of local (subconjunctival) and systemic (subcutaneous) mNOX-E36 in combination with low dose MMC is more effective than subconjunctival mNOX-E36 with low dose MMC alone in attenuating fibrotic protein deposition following GFS. The results demonstrate that the combination of local and systemic administration of NOX-E36 is a preferred option to inhibit post-operative fibrosis following GFS.

### MATERIALS AND METHODS

***In vivo* study design - treatment arms:** Administration of mNOX-E36 via different routes of administration in combination with low-dose MMC and standard MMC alone as the current gold standard for antifibrotic treatment was compared, with experiments comprising four treatment arms:

### 1. Subconjunctival mNOX-E36 + low-dose MMC group

SC injection of control (revmNOX-E36, 20 mg/kg body weight) pre-operatively;
Subconjunctival injection of 5 µL of 0.1 mg/mL MMC (Kyowa Hakko Kirin Co. Ltd, Japan) at the end of surgery;
Subconjunctival injection of 5 µL mNOX-E36 (10 mg/mL) at end of surgery and on D1 post-op

### 2. Subconjunctival mNOX-E36 + 2 x SC mNOX-E36 + low-dose MMC group

SC injection of mNOX-E36 (20 mg/kg body weight) pre-op and on D1;
Subconjunctival injection of 5 µL of 0.1 mg/mL MMC at the end of surgery;
Subconjunctival injection of 5 µL mNOX-E36 (10mg/mL) at end of surgery and on D1 post-op

### 3. Subconjunctival mNOX-E36 + SC mNOX-E36 + low-dose MMC group

SC injection of mNOX-E36 (20 mg/kg body weight) pre-op;
Subconjunctival injection of 5 µL of 0.1 mg/mL MMC at the end of surgery;
Subconjunctival injection of 5 µL mNOX-E36 (10mg/mL) at end of surgery and on D1 post-op

### 4. Standard MMC group

SC injection of control (revmNOX-E36, 20 mg/kg body weight) pre-op;
Subconjunctival injection of 5 µL of 0.2 mg/mL MMC at the end of surgery;
Subconjunctival injection of 5 µL revmNOX-E36 (10 mg/mL) at end of surgery and on D1 post-op
For all four treatment arms, subconjunctival injections were performed immediately after the GFS, and an additional subconjunctival injection of mNOX-E36 was performed24 hours post-operation.

**Mouse model of Glaucoma Filtration Surgery:** Ethical approval was obtained from the SingHealth Institutional Animal Care and Use Committee (IACUC). C57BL/6 mice were bred and treated in accordance with the Association for Research in Vision and Ophthalmology (ARVO) Statement on the Use of Animals in Ophthalmic and Vision Research. The mouse model of GFS is described in Seet et al. 2011 (Seet, Lee et al. 2011) - in brief, mice were anaesthetised via intraperitoneal injection of a ketamine/xylazine cocktail consisting 20 mg/mL ketamine (Troy Laboratories, Australia) and 2 mg/mL xylazine (Parnell Laboratories, Australia). The conjunctiva in one eye was dissected superiorly to expose bare sclera, and a 30-gauge sterile needle was passed into the anterior chamber to form a fistula, allowing aqueous egress into the subconjunctival space. The dissected conjunctiva was then closed over the fistula with 10-0 nylon (Ethicon Inc, USA). Following which, subconjunctival and subcutaneous injections of experimental drugs were performed and local fucithalmic ointment (Leo Pharmaceutical Products, Denmark) was instilled at the end of the surgery. The contralateral unoperated eye was used as a baseline for comparison. Animals were euthanized on Day 7 (D7) post-surgery for downstream analysis.

**Western blotting:** Western blot analysis was performed to assess the extent of fibrosis. Bleb tissues from 5 operated eyes were harvested D7 post-surgery and pooled (i.e., taken as n=1). Tissues or cells were processed in lysis buffer complete with protease inhibitors and the resulting lysate loaded onto SDS-polyacrylamide gel for electrophoresis and immunoblotting. The following antibodies were used for detection: anti-fibronectin (Abcam, UK), anti-collagen 1α1 (Abnova Corp, USA), and anti-GAPDH (Santa Cruz Biotechnology, USA) and corresponding horseradish peroxidase (HRP)-conjugated secondary antibodies (Jackson ImmunoResearch Laboratories, USA). Densitometric quantitation was performed, with variations in loading corrected to levels of housekeeping protein GAPDH.

### RESULTS

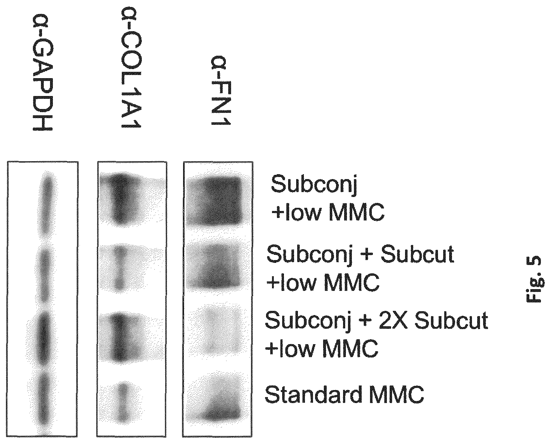

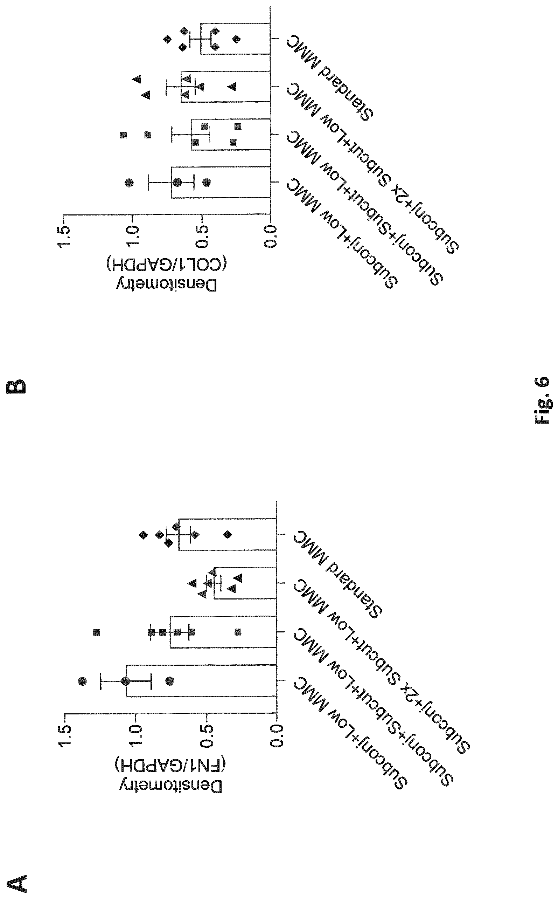

Co-administration of subconjunctival and subcutaneous mNOX-E36 with a sub-therapeutic dose of MMC ("low dose") is more effective than subconjunctival mNOX-E36 with low dose MMC alone in attenuating fibrotic protein, collagen I (19.5% reduction) and fibronectin (28.8% reduction) deposition following GFS, as assessed by Western blot and densitometry analysis of fibrotic proteins FN1 and COL1A1 in mouse conjunctival bleb tissues on day 7 post-GFS. An additional dose of SC mNOX-E36 one day post-GFS had an even stronger effect on further reducing fibronectin protein deposition (40.8% reduction). A combination of subconjunctival and subcutaneous mNOX-E36 with low dose MMC demonstrated comparable anti-fibrotic activity to standard dose MMC in inhibiting fibrosis following GFS **(****Figs 5** **and** **6**).

### CONCLUSION

Results demonstrate that the combination of local and systemic administration of (m)NOX-E36 is a preferred option to inhibit post-operative fibrosis following GFS, underlining the potential of CCL2 inhibition via NOX-E36 in reducing post-operative inflammation and fibrosis in human patients following GFS, while avoiding the pitfalls associated with MMC.

### Example 3: Multi-functional role of CCL2 inhibiting Spiegelmer mNOX-E36 on both macrophage and conjunctival fibroblast activity

**Summary:** CCL2 (monocyte-chemoattractant protein 1, MCP-1), a potent recruiter of monocytes, is increased in tear fluid of glaucoma patients with greater propensity to scar. NOX-E36 is an anti-CCL2 L-RNA aptamer that has completed a Phase 2 clinical trial in patients with diabetes mellitus and albuminuria. Its murine-specific analogue, the L-RNA aptamer mNOX-E36, was previously shown to attenuate glomerulosclerosis and liver fibrosis in mice. Bleb scarring represents the biggest risk of the failure of glaucoma filtration surgery (GFS), a commonly procedure to reduce ocular pressure in patients with glaucoma. The advent of anti-fibrotic agents, such as mitomycin C (MMC) are often used to prolong the longevity of the bleb. However, the cytotoxic effect of MMC raise significant concerns. This set of *in vitro* studies investigated the mechanism of action of mNOX-E36 in attenuating inflammation and fibrosis following GFS and revealed a bifunctional role of mNOX-E36 in inhibiting both macrophage and conjunctival fibroblast activity.

### MATERIALS AND METHODS

**Cell Culture:** Primary conjunctival fibroblasts isolated from C57BL6/J mice were cultured as described previously (Seet, Su et al. 2010). RAW 264.7 (ATCC, USA) macrophage cell line was maintained in Dulbecco's modified Eagle's medium (DMEM) supplemented with 10% fetal bovine serum (FBS) and 1% Penicillin-Streptomycin. Lipopolysaccharide (LPS) was used at a concentration 0.5 µg/mL. Anti-CCL2 Spiegelmer mNOX-E36 and inactive control revmNOX-E36 were used at a concentration of 200 µg/mL. Cytokines, TGFβ2 and CCL2 were used at a dose of 10 ng/mL and 100 ng/mL, respectively.

**Transwell migration:** Transwell migration assay was carried out in 24-well, 8 µm pore size transwell permeable support inserts (Corning, USA) coated overnight with 100 µg/mL rat tail collagen I. 10% FBS with LPS was used as a chemoattractant. Post-migration, cells were fixed and stained with DAPI and visualized using EVOS M5000 Imaging System (Life Technologies, USA). ImageJ software (National Institutes of Health, USA) was used to determine the total number of migrated cells.

**Western blotting:** Western blot analysis was performed to assess the extent of fibrosis. Cells were processed in lysis buffer complete with protease inhibitors and the resulting lysate loaded onto SDS-polyacrylamide gel for electrophoresis and immunoblotting. The following antibodies were used for detection: anti-fibronectin (Abcam, UK), anti-collagen 1α1 (Abnova Corp, USA), anti-connective tissue growth factor (Santa Cruz Biotechnology, USA), anti-alpha smooth muscle actin (Abcam, UK) and anti-GAPDH (Santa Cruz Biotechnology, USA) and corresponding horseradish peroxidase (HRP)-conjugated secondary antibodies (Jackson ImmunoResearch Laboratories, USA). Densitometric quantitation was performed, with variations in loading corrected to levels of housekeeping protein GAPDH.

**Statistical analysis:** Data are presented as mean ± standard error of mean (s.e.m.). Statistical analyses were performed by an unpaired, two-tailed Student's t-test or one-way ANOVA followed by appropriate post-hoc test using GraphPad Prism. Statistical significance was defined as p-values < 0.05.

### RESULTS

To determine the mechanism of action of mNOX-E36 in attenuating inflammation and fibrosis following GFS, a series of *in vitro* experiments was conducted involving RAW264.7 macrophages and mouse conjunctival fibroblasts (MCFs). First it was demonstrated that mNOX-E36 is able to inhibit LPS-induced RAW264.7 migration **(****Fig. 7****).**

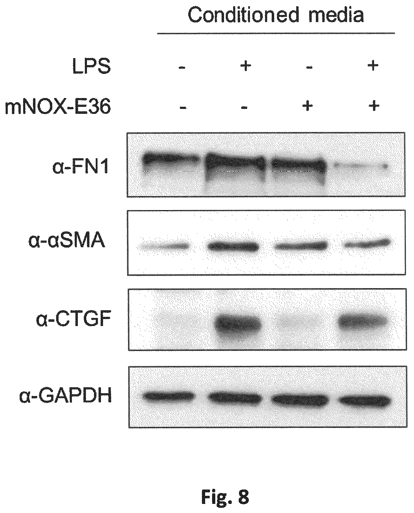

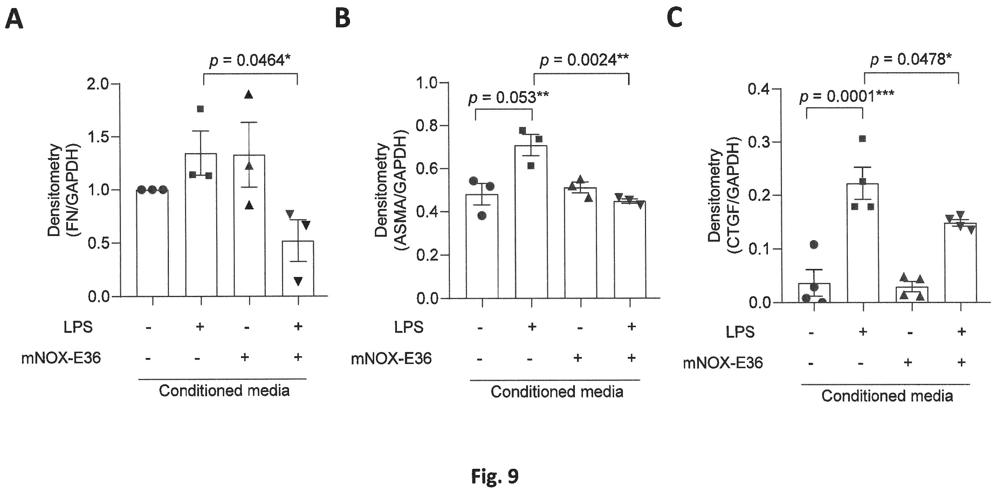

To understand the impact of mNOX-E36 on macrophage-fibroblast interaction, mouse conjunctival fibroblasts (MCFs) were exposed to conditioned media from LPS and/or mNOX-E36 treated RAW 264.7 cells. MCFs subjected to conditioned media from LPS-stimulated RAW 264.7 cells demonstrated an increased expression of pro-fibrotic proteins FN1, α-SMA and CTGF. However, fibrotic protein deposition was significantly reduced when MCFs were exposed to conditioned media derived from mNOX-E36 and LPS cotreated RAW 264.7 cells instead **(****Figs. 8****,** **9****).**

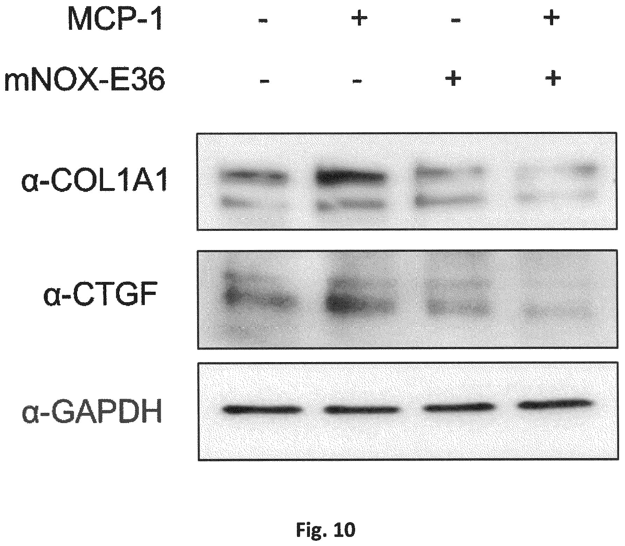

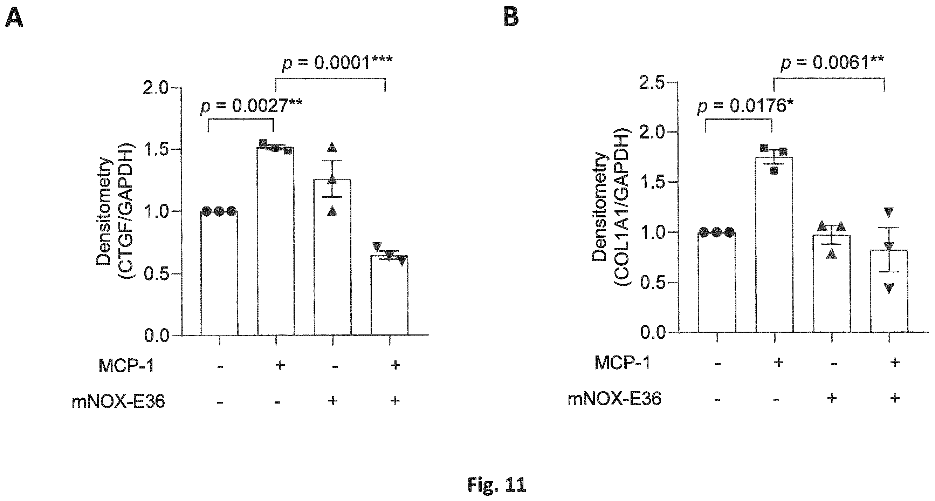

Next, it was investigated whether CCL2 (MCP-1) could directly induce conjunctival fibrosis. Our data showed that CCL2 robustly induced deposition of pro-fibrotic proteins COL1A1 and CTGF in MCFs, and this could be inhibited with mNOX-E36 **(****Figs. 10****,** **11****).**

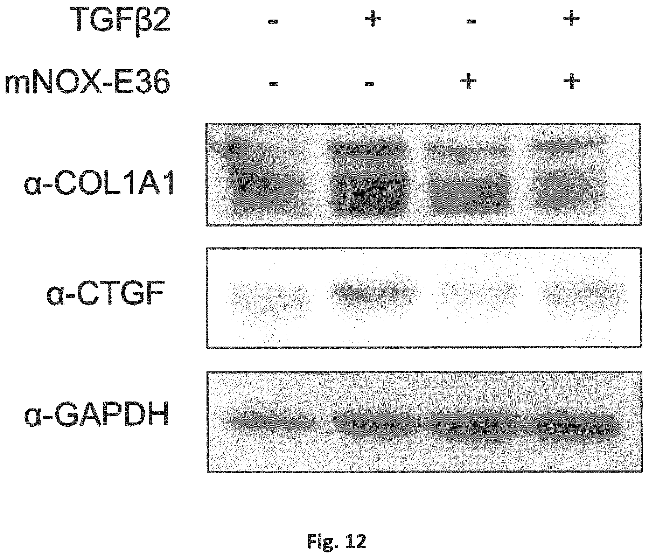

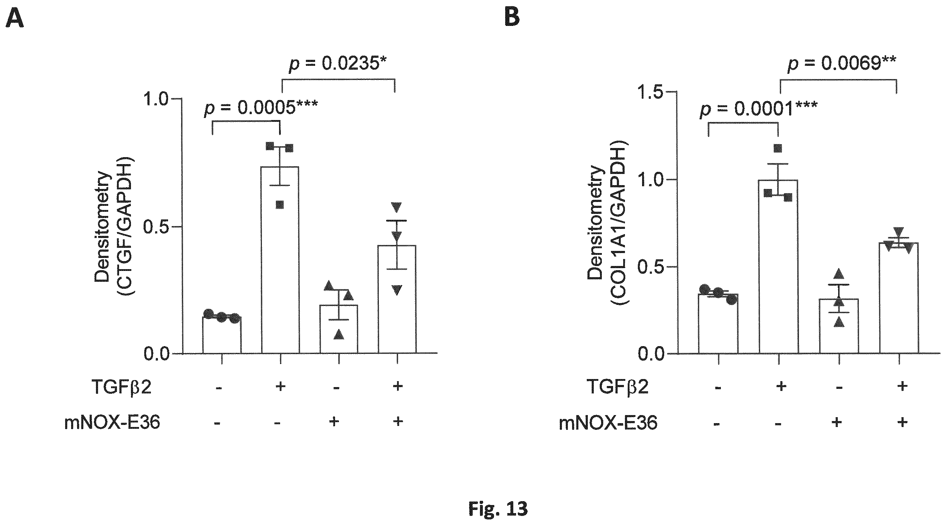

Lastly, to determine whether mNOX-E36 may have a direct effect on conjunctival fibroblasts besides attenuating the activity of macrophages and macrophage-fibroblast crosstalk, MCFs were subjected to direct treatment with mNOX-E36 in the presence of a pro-fibrotic cytokine, TGFβ2. The results demonstrate that mNOX-E36 treatment inhibited the TGFβ2-induced COL1A1 and CTGF fibrotic protein expression in MCFs **(****Figs. 12****,** **13****).**

### CONCLUSION

mNOX-E36 inhibits both macrophage activation and fibrotic protein deposition in conjunctival fibroblasts.

### Example 4: Nucleic acids that bind human MCP-1

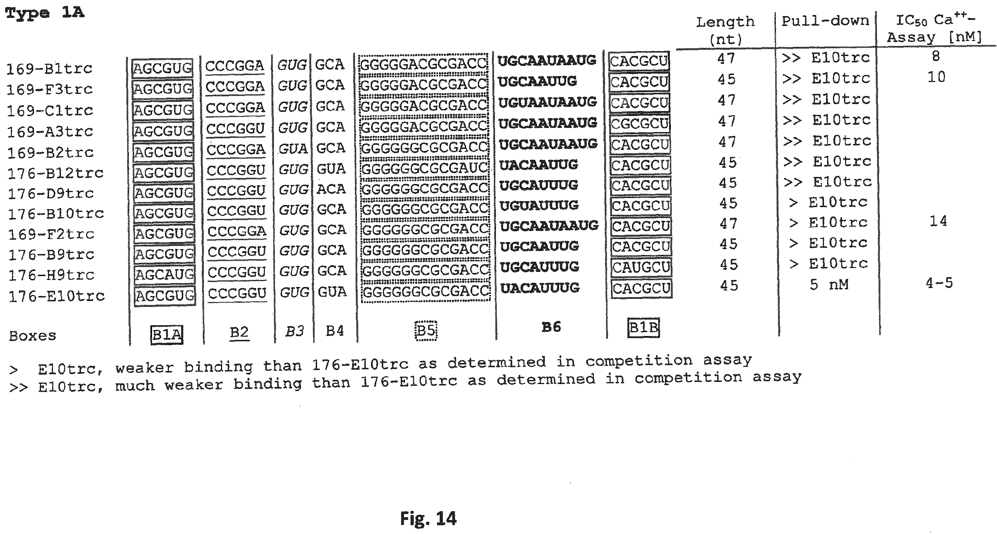

Using biotinylated human D-MCP-1 as a target, several nucleic acids that bind to human MCP-1 could be generated the nucleotide sequences of which are depicted in Figures 14 through 20. The nucleic acids were characterized on the aptamer, i. e. D-nucleic acid level using competitive or direct pull-down assays with biotinylated human D-MCP-1 (Example 7) or on the Spiegelmer level, i. e. L-nucleic acid with the natural configuration of MCP-1 (L-MCP) by surface plasmon resonance measurement using a Biacore 2000 instrument (Example 10), an *in vitro* cell culture Ca⁺⁺-release assay (Example 8), or an *in vitro* chemotaxis assay (Example 9).

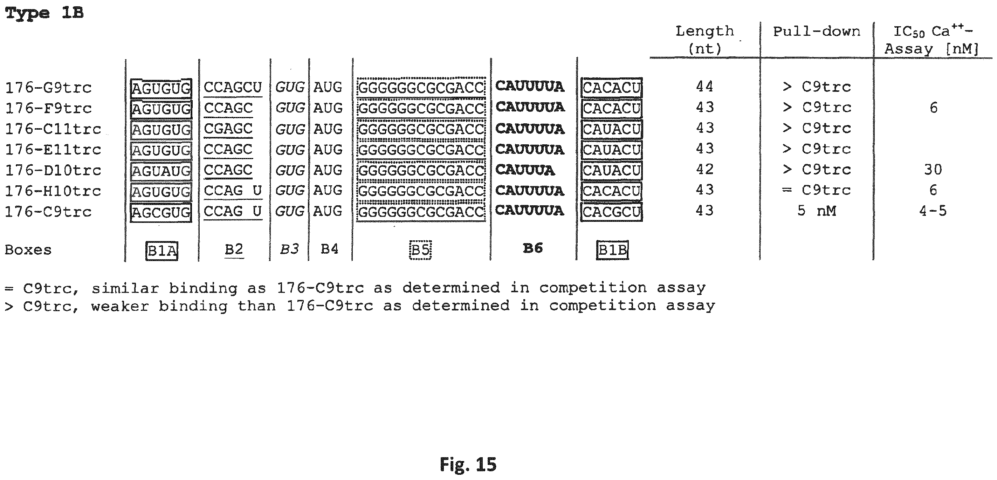

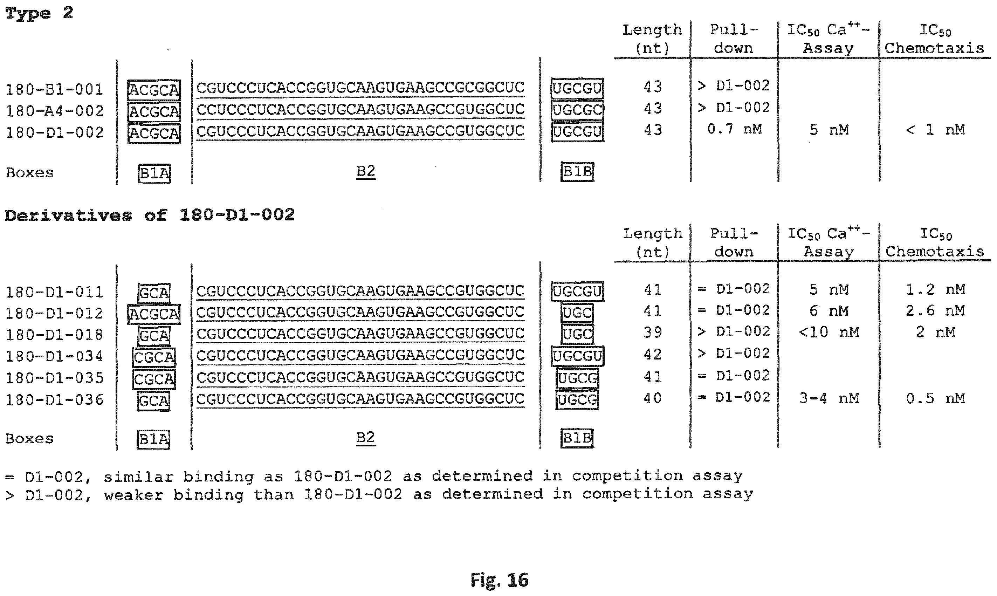

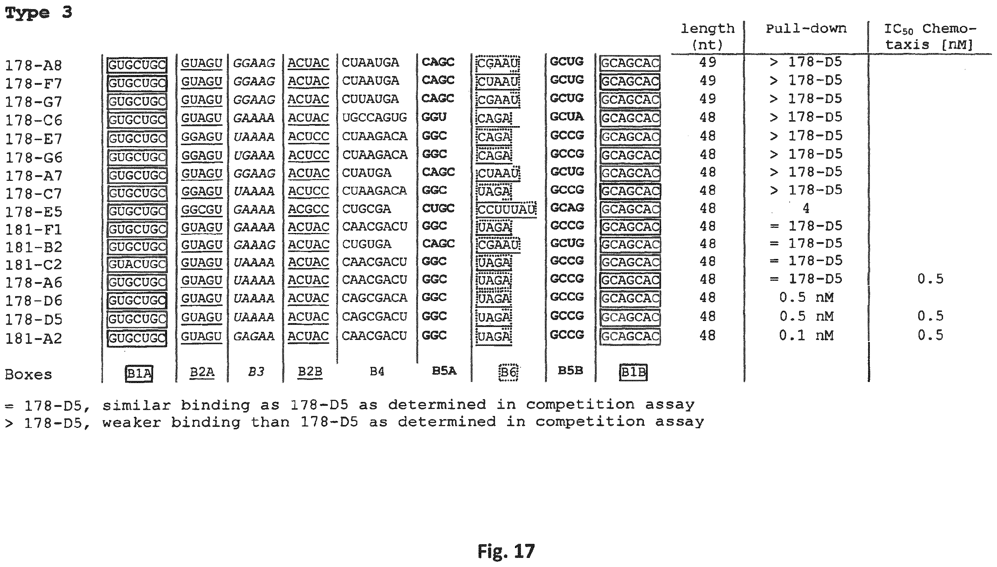

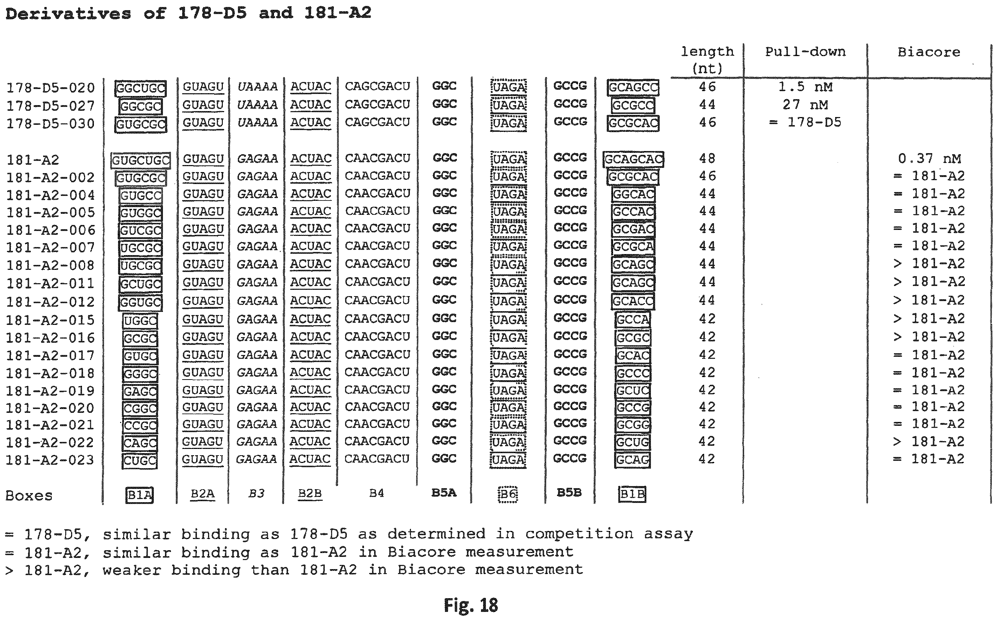

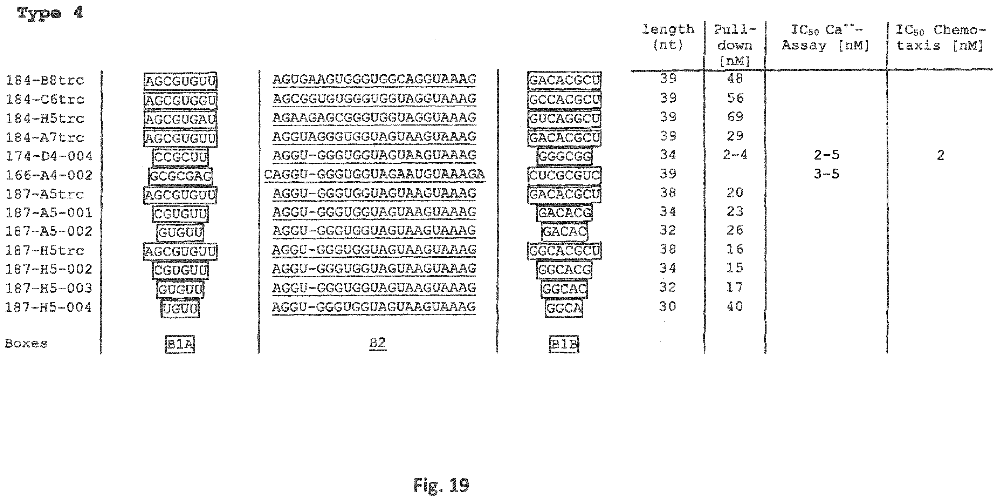

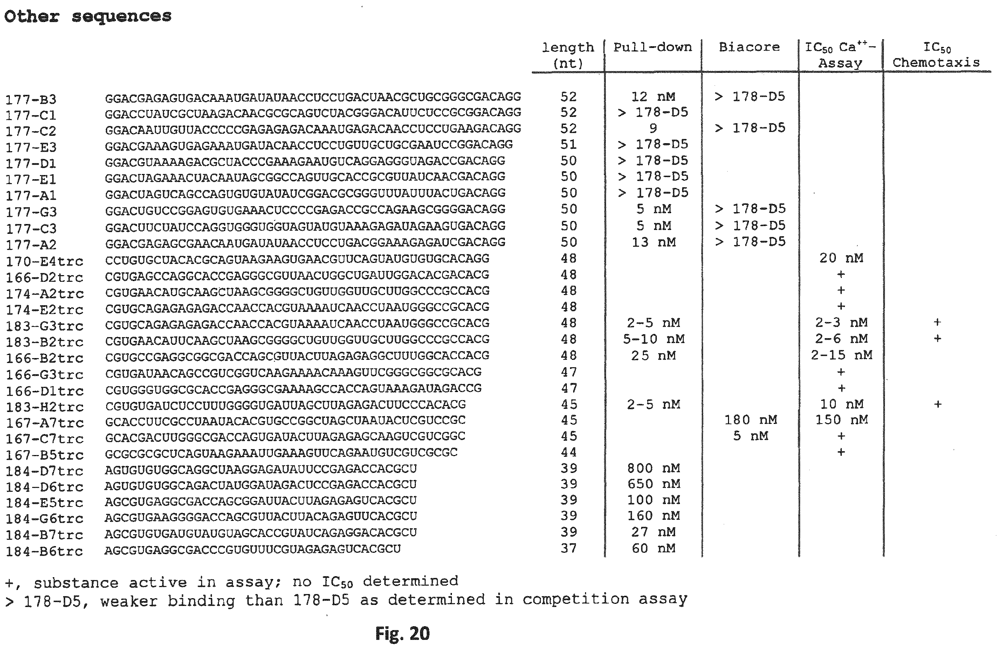

The nucleic acid molecules thus generated exhibit different sequence motifs, four main types are defined in Figs. 1 and 15 (Type 1A / 1B), Fig. 16 (Type 2), Figs. 17 and 18 (Type 3), and Fig. 19 (Type 4). Additional MCP-1 binding nucleic acids which cannot be related to each other and to the different sequence motifs described herein, are listed in Fig. 20. For definition of nucleotide sequence motifs, the IUPAC abbreviations for ambiguous nucleotides is used:

| | | |
|---|---|---|
| S | strong | G or C; |
| W | weak | A or U; |
| R | purine | G or A; |
| Y | pyrimidine | C or U; |
| K | keto | G or U; |
| M | imino | A or C; |
| B | not A | C or U or G; |
| D | not C | A or G or U; |
| H | not G | A or C or U; |
| V | not U | A or C or G; |
| N | all | A or G or C or U |

If not indicated to the contrary, any nucleic acid sequence or sequence of stretches and boxes, respectively, is indicated in the 5' → 3' direction.

### Type 1A MCP-1 binding nucleic acids (Fig. 14)

As depicted in Fig. 1 all sequences of MCP-1 binding nucleic acids of Type 1A comprise several sequences stretches or boxes whereby boxes 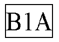 and 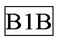 are the 5'- and 3' terminal stretches that can hybridize with each other. However, such hybridization is not necessarily given in the molecule as actually present under physiological conditions. Boxes B2, *B3*, B4, 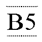 and box **B6** are flanked by box 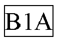 and box 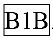 .

The nucleic acids were characterized on the aptamer level using direct and competitive pull-down assays with biotinylated human D-MCP-1 in order to rank them with respect to their binding behaviour (Example 7). Selected sequences were synthesized as Spiegelmer (Example 6) and were tested using the natural configuration of MCP-1 (L-MCP) in an *in vitro* cell culture Ca⁺⁺-release assay (Example 8).

The sequences of the defined boxes may be different between the MCP-1 binding nucleic acids of Type 1A which influences the binding affinity to MCP-1. Based on binding analysis of the different MCP-1 binding nucleic acids summarized as ***Type 1A MCP-1 binding nucleic acids,*** the boxes 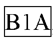 , B2, *B3*, B4, 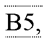 **B6** and 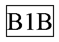 and their nucleotide sequences as described in the following are individually and more preferably in their entirety essential for binding to MCP-1:
- boxes 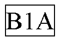 and 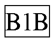 are the 5'- and 3' terminal stretches can hybridize with each other; where 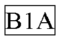 is 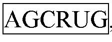 , preferably 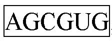 ; and where 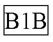 is 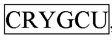 , preferably 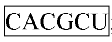 ;
- box B2, which is CCCGGW, preferably CCCGGU;
- box *B3*, which is *GUR*, preferably *GUG*;
- box B4, which is RYA, preferably GUA;
- box B5, which is GGGGGRCGCGAYC, preferably 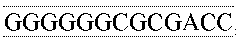 ;
- box **B6,** which is **UGCAAUAAUG** or **URYAWUUG,** preferably **UACAUUUG;**

As depicted in Fig. 14, the nucleic acid molecule referred to as 176-E10trc has the best binding affinity to MCP-1 (as aptamer in the pull-assay with a K_{D} of 5 nM as well as as Spiegelmer with an IC₅₀ of 4 - 5 nM in *in vitro* cell culture Ca⁺⁺-release assay) and therefore may constitute the optimal sequence and the optimal combination of sequence elements B1A 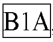 , B2, *B3*, B4, B5, **B6** and 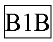 .

### Type 1B MCP-1 binding nucleic acids (Fig. 15)

As depicted in Fig. 15, all sequences of Type 1B comprise several sequences stretches or boxes whereby boxes 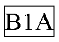 and 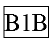 are the 5'- and 3' terminal stretches that can hybridize with each other and boxes B2, *B3*, B4, 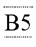 and box **B6** are flanked by box 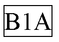 and box 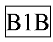 . However, such hybridization is not necessarily given in the molecule as actually present under physiological conditions.

The nucleic acids were characterized on the aptamer level using direct and competitive pull-down assays with biotinylated human D-MCP-1 in order to rank them with respect to their binding behavior (Example 4). Selected sequences were synthesized as Spiegelmer (Example 6) and were tested using the natural configuration of MCP-1 (L-MCP) in an *in vitro* cell culture Ca⁺⁺-release assay (Example 8).

The sequences of the defined boxes may be different between the MCP-1 binding nucleic acids of Type 1B which influences the binding affinity to MCP-1. Based on binding analysis of the different MCP-1 binding nucleic acids summarized as ***Type 1B MCP-1 binding nucleic acids,*** the boxes 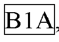 , B2, *B3*, B4, 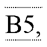 **B6** and 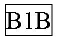 and their nucleotide sequences as described in the following are individually and more preferably in their entirety essential for binding to MCP-1:
- boxes 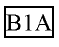 and 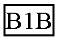 that can hybridize with each other; where 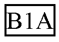 is 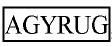 , preferably 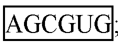 ; and where 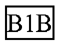 is 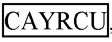 , preferably 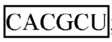 ;
- box B2, which is CCAGCU or CCAGY, preferably CCAGU;
- box *B3*, which is *GUG*;
- box B4, which is AUG;
- box B5, which is GGGGGGCGCGACC;
- box **B6**, which is **CAUUUUA** or **CAUUUA,** preferably **CAUUUUA;**
As depicted in Fig. 15, the nucleic acid referred to as 176-C9trc has the best binding affinity to MCP-1 (as aptamer in the pull-down assay with a K_{D} of 5 nM as well as as Spiegelmer with an IC₅₀ of 4 - 5 nM in *in vitro* cell culture Ca⁺⁺-release assay) and therefore may constitute the optimal sequence and the optimal combination of sequence elements 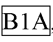 , B2, *B3*n B4, B5, **B6** and 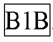 .

### Type 2 MCP-1 binding nucleic acids (Fig. 16)

As depicted in Fig. 16, all sequences of Type 2 comprise several sequences stretches or boxes whereby boxes 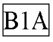 and 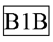 are the 5'- and 3' terminal stretches that can hybridize with each other and box B2 is the central sequence element. However, such hybridization is not necessarily given in the molecule as actually present under physiological conditions.

The nucleic acids were characterized on the aptamer level using direct and competitive pull-down assays with biotinylated human D-MCP-1 in order to rank them with respect to their binding behaviour (Example 4). Selected sequences were synthesized as Spiegelmer (Example 6) and were tested tested using the natural configuration of MCP-1 (L-MCP) in *in vitro* cell culture Ca⁺⁺-release (Example 8) or *in vitro* chemotaxis assays (Example 9).

The sequences of the defined boxes may be different between the MCP-1 binding nucleic acids of Type 3 which influences the binding affinity to MCP-1. Based on binding analysis of the different MCP-1 binding nucleic acids summarized as ***Type 2 MCP-1 binding nucleic acids,*** the boxes 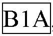 , B2, and 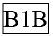 and their nucleotide sequences as described in the following are individually and more preferably in their entirety essential for binding to MCP-1:
- boxes 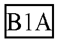 and 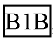 , 5'- and 3' terminal stretches that can hybridize with each other; where 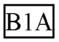 is 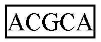 and 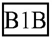 is 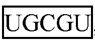 , of 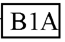 is 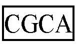 and 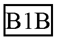 is 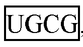 , or 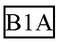 is 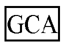 and 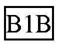 is 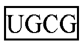 or 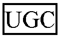 ; preferably 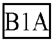 is 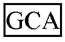 and 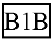 is 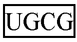 ;
- box B2, CSUCCCUCACCGGUGCAAGUGAAGCCGYGGCUC, preferably CGUCCCUCACCGGUGCAAGUGAAGCCGUGGCUC

As depicted in Fig. 19, the nucleic acid referred to as 180-D1-002 as well as the derivatives of 180-D1-002 like 180-D1-011, 180-D1-012, 180-D1-035, and 180-D1-036 (= NOX-E36) have the best binding affinity to MCP-1 as aptamer in the pull-down or competitive pull-down assay with an K_{D} of < 1 nM and therefore may constitute the optimal sequence and the optimal combination of sequence elements 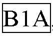 , B2, and 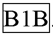 .

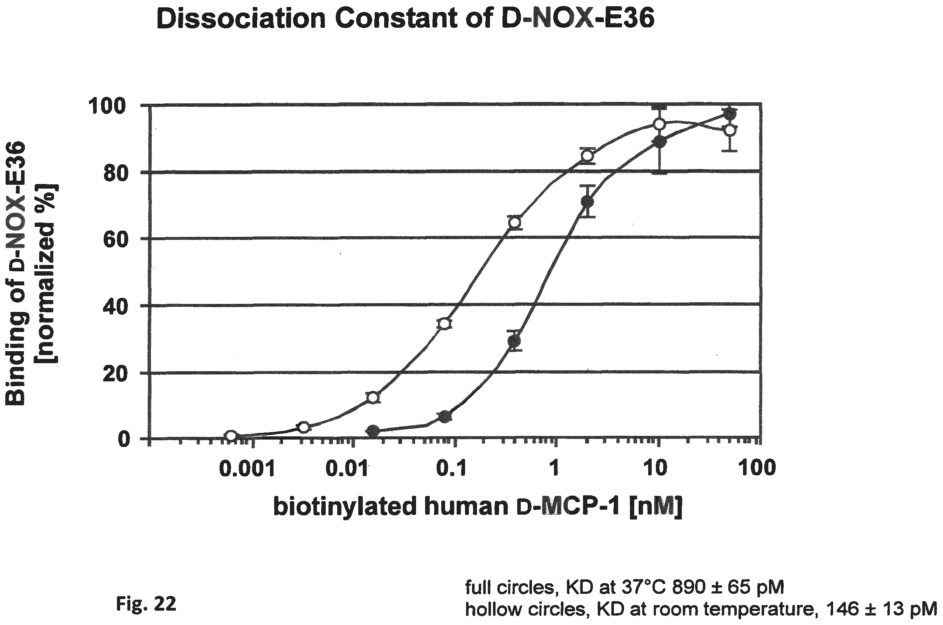

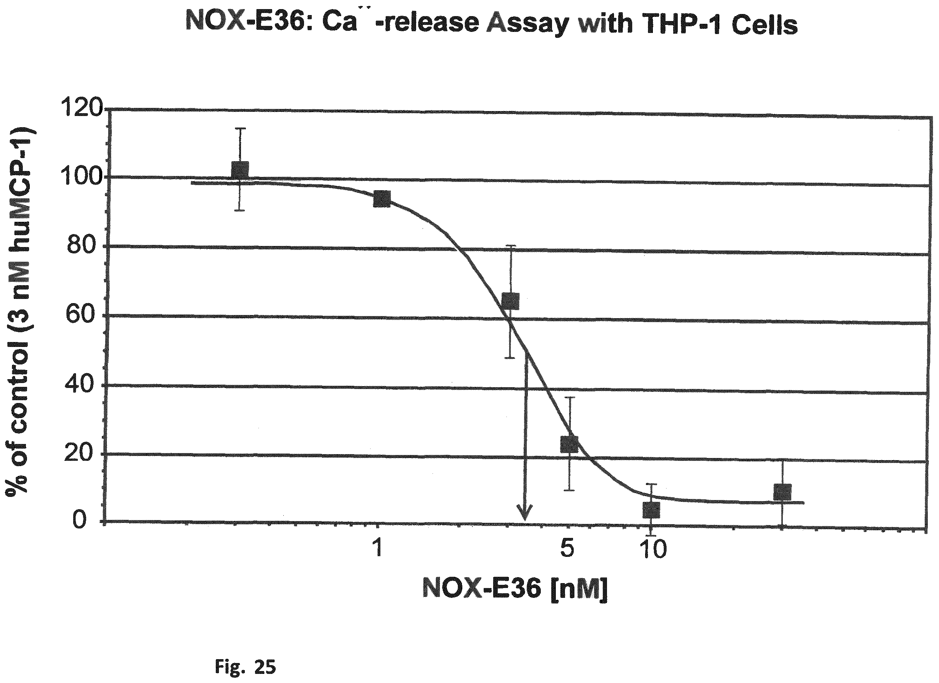

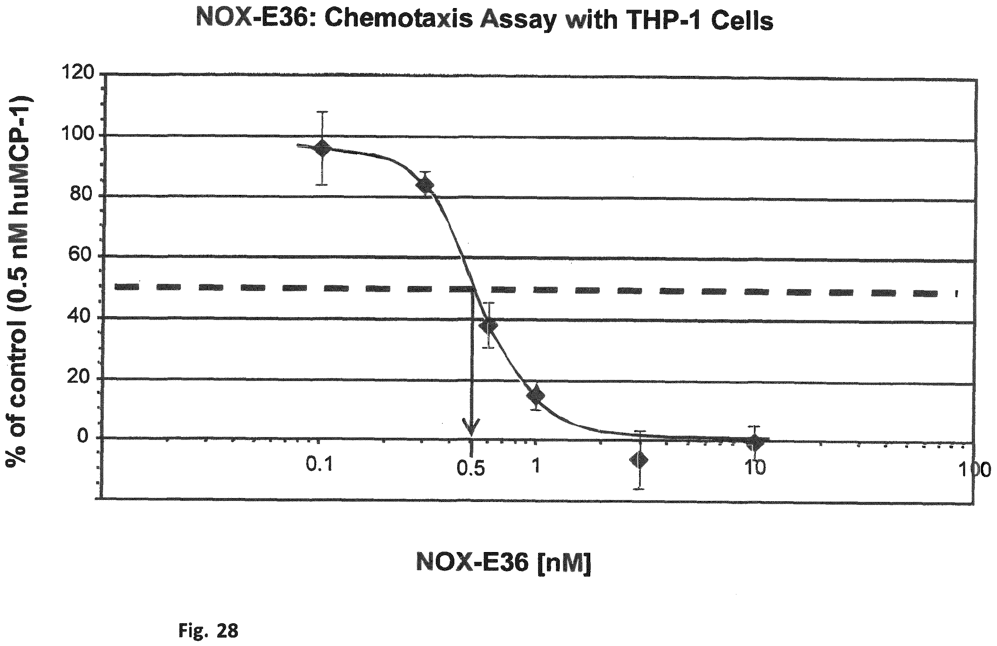

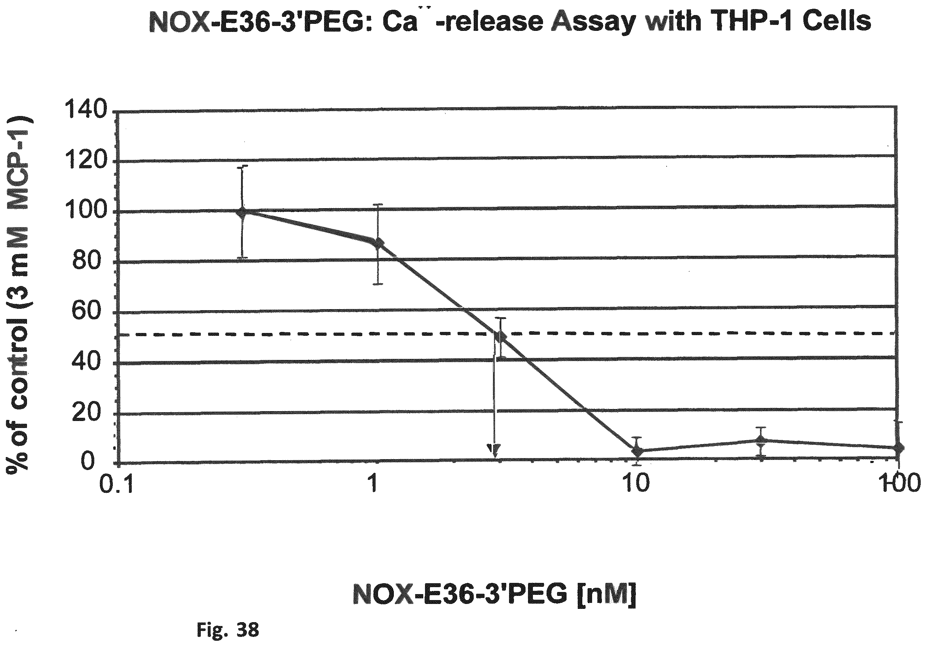

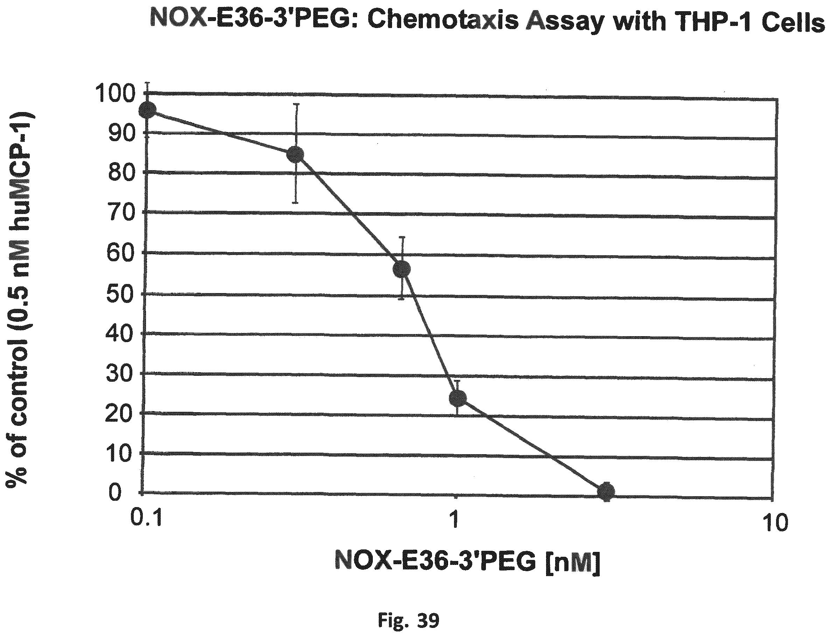

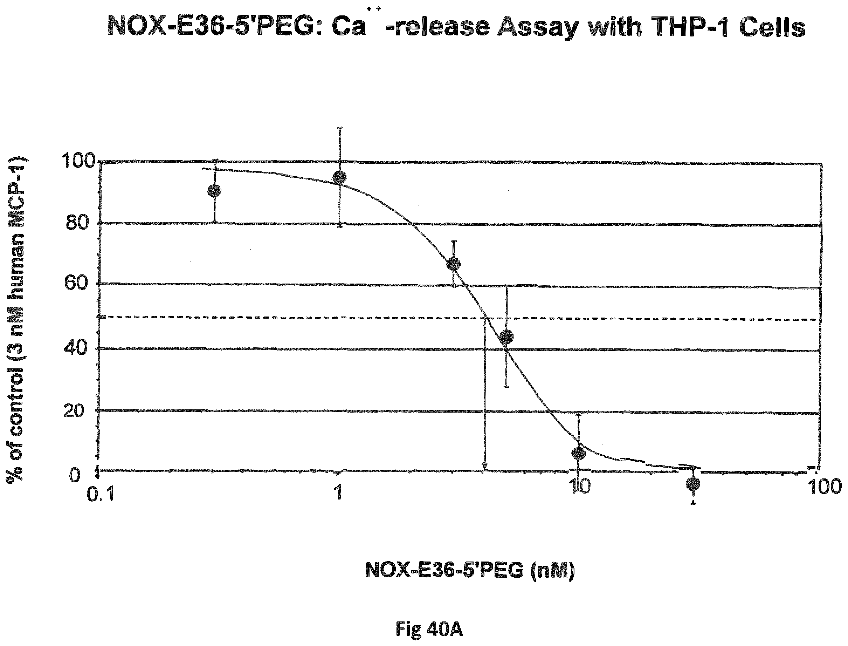

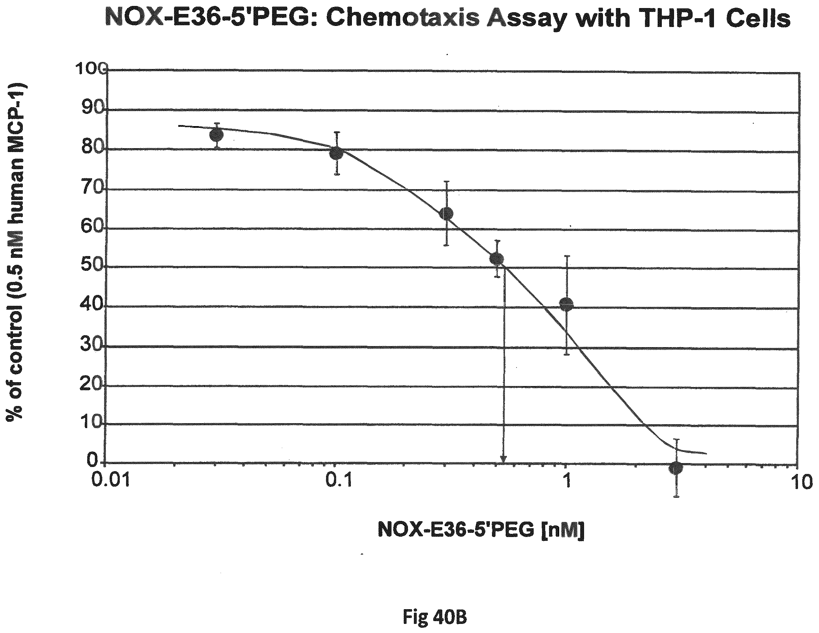

For nucleic acid molecule D-NOX-E36 (D-180-D1-036; SEQ.ID No. 159), a dissociation constant (K_{D}) of 890 ± 65 pM at room temperature (RT) and of 146 ± 13 pM at 37°C was determined (Example 7; Fig. 22). The respective Spiegelmer NOX-E36 (180-D1-036; SEQ.ID No. 37) exhibited an inhibitory concentration (IC₅₀) of 3 - 4 nM in an *in vitro* Ca⁺⁺-release assay (Example 8; Fig. 25) and of ca. 0.5 nM in an *in vitro* chemotaxis assay (Example 9; Fig. 28). For the PEGylated derivatives of NOX-E36, NOX-E36-3'PEG and NOX-E36-5'PEG, IC₅₀s of ca. 3 nM were determined in the Ca⁺⁺-release assay (Example 8, Fig. 38 and Fig. 40A) and < 1 nM in the chemotaxis assay (Example 9; Fig. 39 and Fig. 40B).

### Type 3 MCP-1 binding nucleic acids (Figs. 17+18)

As depicted in Figs. 17 and 18, all sequences of Type 3 comprise several sequence stretches or boxes whereby three pairs of boxes are characteristic for ***Type 3 MCP-1 binding nucleic acids.*** Both boxes 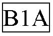 and 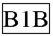 as well as boxes B2A and B2B as well as boxes **B5A** and **B5B** bear the ability to hybridize with each other. However, such hybridization is not necessarily given in the molecule as actually present under physiological conditions. Between these potentially hybridized sequence elements, non-hybridizing nucleotides are located, defined as box *B3*, box B4 and box 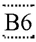 .

The nucleic acids were characterized on the aptamer level using direct and competitive pull-down assays with biotinylated human D-MCP-1 in order to rank them with respect to their binding behavior (Example 7). Selected sequences were synthesized as Spiegelmer (Example 6) and were tested using the natural configuration of MCP-1 (L-MCP) in *in vitro* chemotaxis assays (Example 9) or via Biacore measurements (Example 10).

The sequences of the defined boxes may be different between the MCP-1 binding nucleic acids of Type 3 which influences the binding affinity to MCP-1. Based on binding analysis of the different MCP-1 binding nucleic acids summarized as ***Type 3 MCP-1 binding nucleic acids,*** the boxes 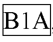 , B2A, *B3*, B2B, B4, **B5A,** 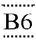 , **B5B,** 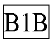 and their nucleotide sequences as described in the following are individually and more preferably in their entirety essential for binding to MCP-1:
- boxes 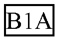 and 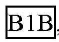 , 5'- and 3' terminal stretches that can hybridize with each other;
   where 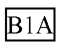 is 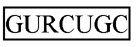 and 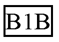 is 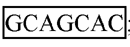 ; preferably 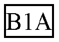 is 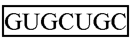 and 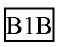 is 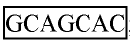 ;
   or 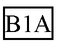 is 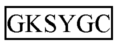 and 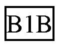 is 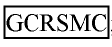 ; preferably 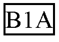 is 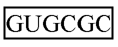 and 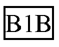 is 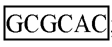 ;
   or 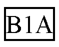 is 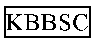 and 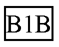 is 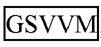 ; preferably 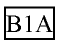 is 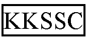 and 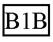 is 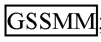 ;
   or 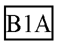 is 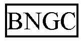 and 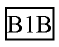 is 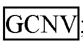 ; preferably 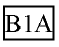 is 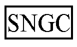 and 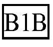 is 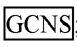 ; most preferably 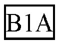 is 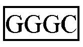 and 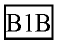 is 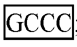 ;
- boxes B2A and B2B, stretches that can hybridize with each other; where B2A is GKMGU and B2B is ACKMC; preferably B2A is GUAGU and B2B is ACUAC;
- box *B3*, which is *KRRAR*, preferably *UAAAA* or *GAGAA*;
- box B4, which is CURYGA or CUWAUGA or CWRMGACW or UGCCAGUG, preferably CAGCGACU or CAACGACU;
- **B5A** and **B5B,** stretches that can hybridize with each other; where **B5A** is **GGY** and **B5B** is **GCYR** whereas **GCY** can hybridize with the nucleotides of **B5A**; or **B5A** is **CWGC** and **B5B** is **GCWG;** preferably **B5A** is **GGC** and **B5B** is **GCCG;**
- box B6, which is: YAGA or CKAAU or CCUUUAU, preferably UAGA.

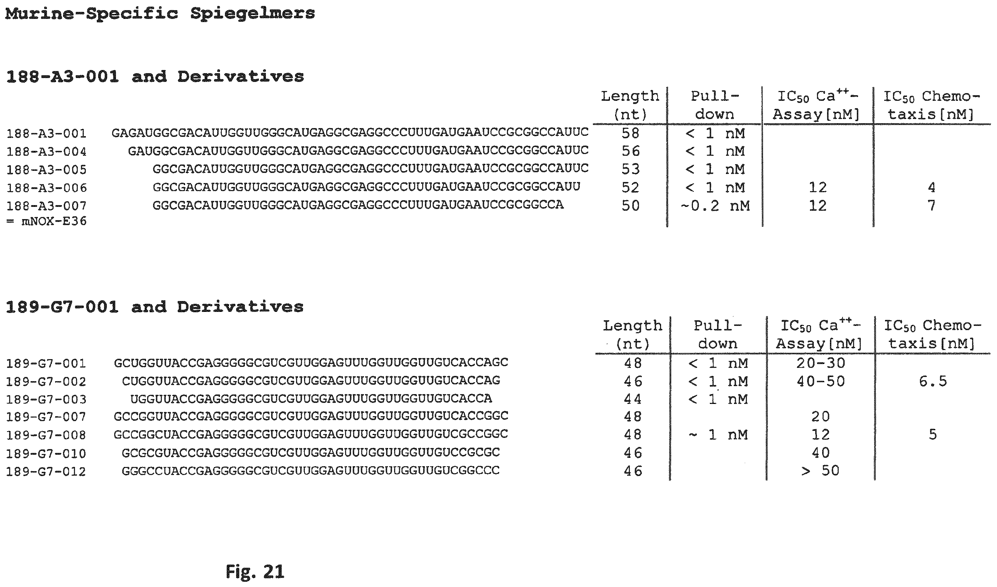

As depicted in Figs. 20 and 21, the nucleic acid referred to as 178-D5 and its derivative 178-D5-030 as well as 181-A2 with its derivatives 181-A2-002, 181-A2-004, 181-A2-005, 181-A2-006, 181-A2-007, 181-A2-017, 181-A2-018, 181-A2-019, 181-A2-020, 181-A2-021, and 181-A2-023 have the best binding affinity to MCP-1. 178-D5 and 178-D5-030 were evaluated as aptamers in direct or competitive pull-down assays (Example 7) with an K_{D} of approx. 500 pM. In the same experimental set-up, 181-A2 was determined with an K_{D} of approx. 100 pM. By Biacore analysis (Example 10), the K_{D} of 181-A2 and its derivatives towards MCP-1 was determined to be 200 - 300 pM. In Ca⁺⁺ release and chemotaxis assays with cultured cells (Example 8 and 9, respectively), for both 178-D5 and 181-A2, an IC₅₀ of approx. 500 pM was measured. Therefore, 178-D5 as well as 181-A2 and their derivatives may constitute the optimal sequence and the optimal combination of sequence elements 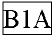 , B2A, *B3*, B2B, B4, **B5A,** 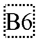 , **B5B** and 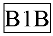 .

### Type 4 MCP-1 binding nucleic acids (Fig. 22)

As depicted in Fig. 22, all sequences of Type 4 comprise several sequences, stretches or boxes whereby boxes 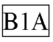 and 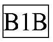 are the 5'- and 3' terminal stretches that can hybridize with each other and box B2 is the central sequence element.

The nucleic acids were characterized on the aptamer level using direct pull-down assays with biotinylated human D-MCP-1 in order to rank them with respect to their binding behavior (Example 7). Selected sequences were synthesized as Spiegelmer (Example 6) and were tested using the natural configuration of MCP-1 (L-MCP) in an *in vitro* cell culture Ca⁺⁺-release (Example 8) and/or chemotaxis assay (Example 9).

The sequences of the defined boxes may differ among the MCP-1 binding nucleic acids of Type 4 which influences the binding affinity to MCP-1. Based on binding analysis of the different MCP-1 binding nucleic acids summarized as ***Type 4 MCP-1 binding nucleic acids,*** the boxes 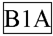 , B2, and 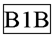 and their nucleotide sequences as described in the following are individually and more preferably in their entirety essential for binding to MCP-1:
- boxes 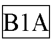 and 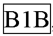 , 5'- and 3' terminal stretches that can hybridize with each other; where 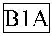 is 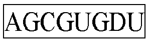 and 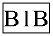 is 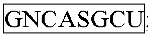 ; or 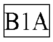 is 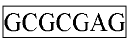 and 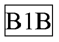 is 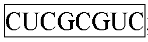 ; or 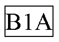 is 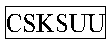 and 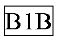 is 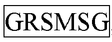 ; or 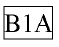 is 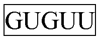 and 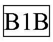 is 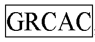 ; or 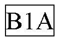 is 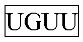 and 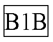 is 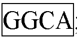 ; preferably 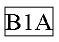 is 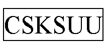 and 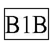 is 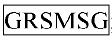 ; mostly preferred B1A is 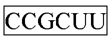 and 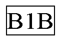 is 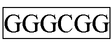 ; and

- box B2, which is AGNDRDGBKGGURGYARGUAAAG or
   AGGUGGGUGGUAGUAAGUAAAG or CAGGUGGGUGGUAGAAUGUAAAGA, preferably AGGUGGGUGGUAGUAAGUAAAG

As depicted in Fig. 19, the nucleic acid referred to as 174-D4-004 and 166-A4-002 have the best binding affinity to MCP-1 (as Spiegelmer with an IC₅₀ of 2 - 5 nM in *in vitro* cell culture Ca⁺⁺ release assay) and may, therefore, constitute the optimal sequence and the optimal combination of sequence elements 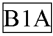 , B2, and 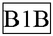 .

Additionally, 29 other MCP-1 binding nucleic acids were identified which cannot be described by a combination of nucleotide sequence elements as has been shown for Types 1 - 4 of MCP-1 binding nucleic acids. These sequences are listed in Fig. 20.

It is to be understood that any of the sequences shown in Figs. 14 through 20 are nucleic acids suitable for use in the present invention, in particular the invention as defined in the claims, the aspects and any embodiment, including those truncated forms thereof but also including those extended forms thereof under the proviso, however, that the thus truncated and extended, respectively, nucleic acid molecules are still capable of binding to the target.

### Example 5: Nucleic acids that bind murine MCP-1

Using biotinylated murine D-MCP-1 as a target, several nucleic acid molecules binding thereto could be generated. The result of a sequence analysis of these nucleic acid molecules can be taken from Fig. 21.

The nucleic acids were characterized on the aptamer level using a pull-down assay using biotinylated murine D-MCP-1 in order to in order to rank them with respect to their binding behavior (Example 7). Selected sequences were synthesized as Spiegelmer (Example 6) and were tested using the natural configuration of MCP-1 (L-MCP) in an *in vitro* cell culture Ca⁺⁺-release (Example 8) and chemotaxis assay (Example 9).

As depicted in Fig. 21, D-188-A3-001 and D-189-G7-001 and their derivatives bind D-MCP-1 with sub-nanomolar K_{D} in the pull-down assay (Fig. 21).

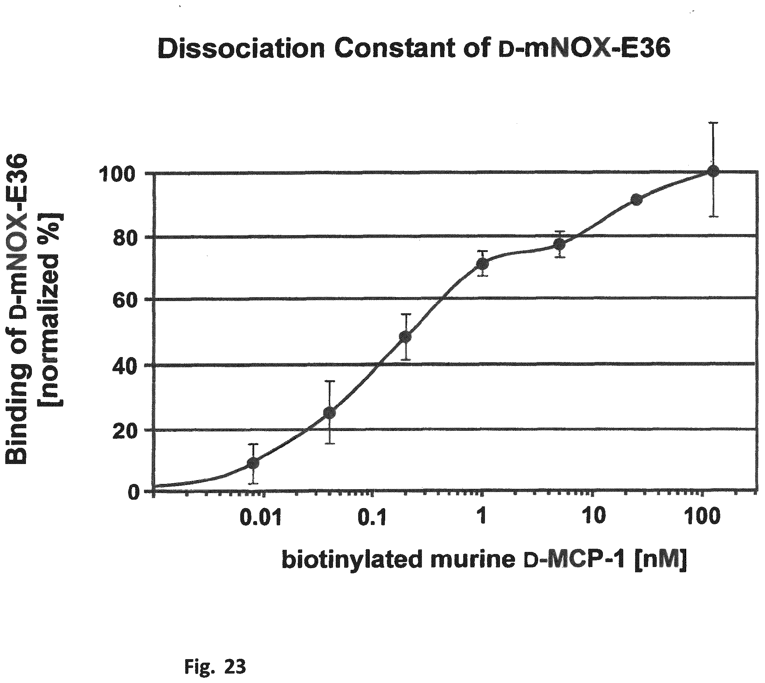

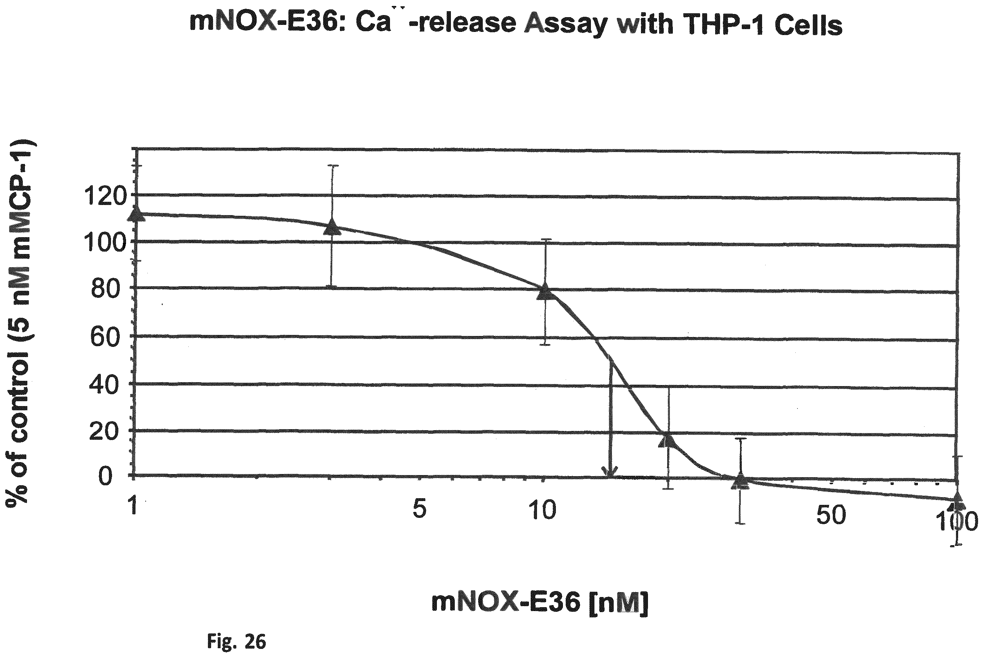

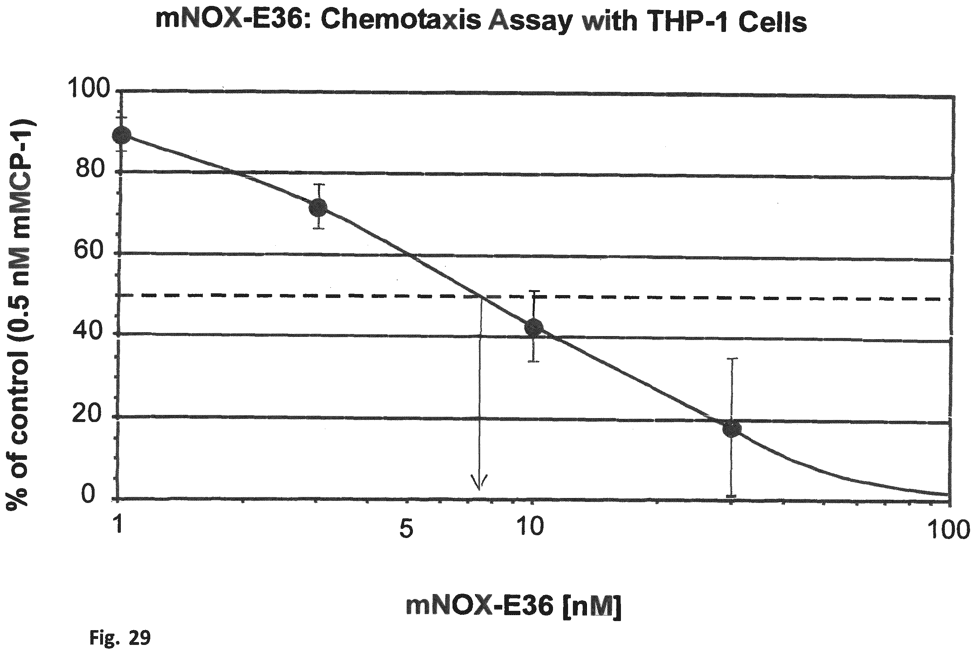

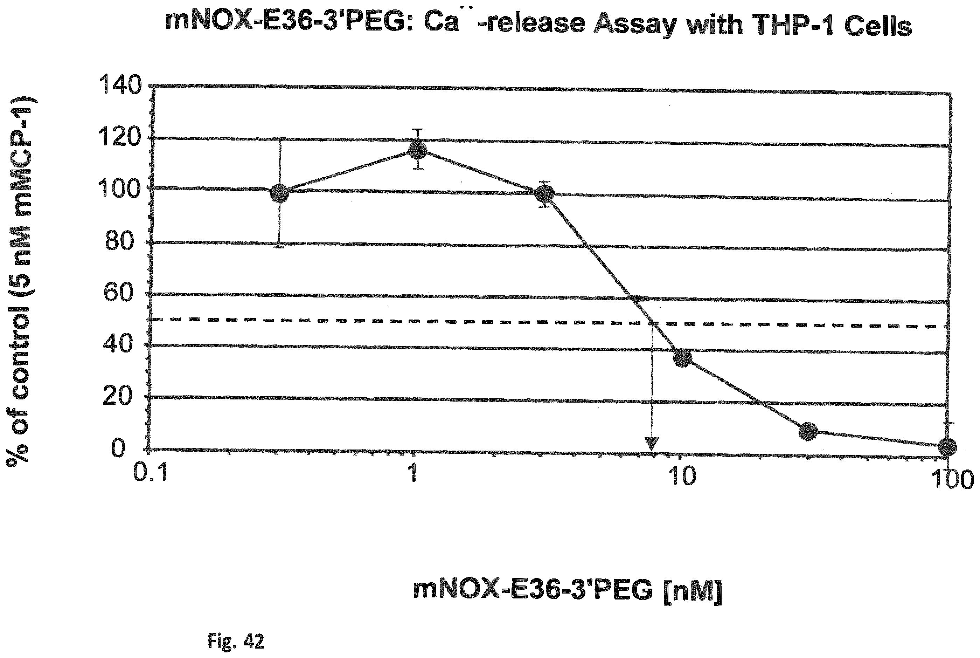

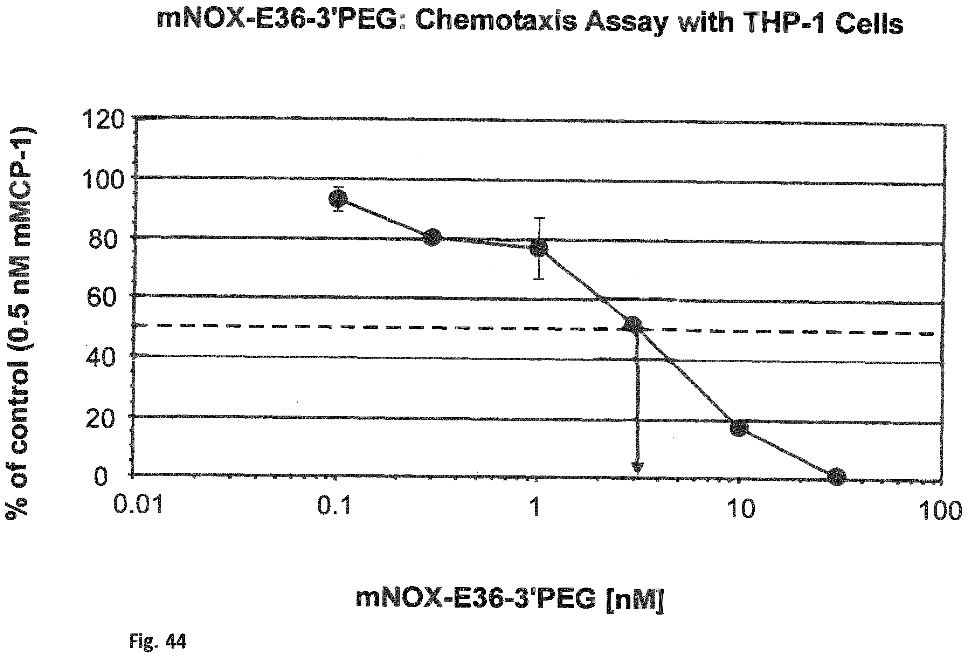

For D-mNOX-E36 (= D-188-A3-007; SEQ.ID No. 244), a dissociation constant (K_{D}) of 0.1 - 0.2 nM at 37°C was determined (Example 7; Fig. 23). The respective Spiegelmer mNOX-E36 (188-A3-007; SEQ.ID No. 122) exhibited an inhibitory concentration (IC₅₀) of approx. 12 nM in an *in vitro* Ca⁺⁺-release assay (Example 8; Fig. 26) and of approx. 7 nM in an *in vitro* chemotaxis assay (Example 9; Fig. 29). For the PEGylated derivative of mNOX-E36, mNOX-E36-3'PEG (SEQ.ID No. 254), IC₅₀'s of approx. 8 nM were determined in the Ca⁺⁺-release assay (Example 8, Fig. 42) and approx. 3 nM in the chemotaxis assay (Example 9; Fig. 44).

It is to be understood that any of the sequences shown in Fig. 21 are nucleic acids suitable for use in the present invention, in particular the invention as defined in the claims, the aspects and any embodiment, including those truncated forms thereof but also including those extended forms thereof under the proviso, however, that the thus truncated and extended, respectively, nucleic acid molecules are still capable of binding to the target.

### Example 6: Synthesis and derivatization of Aptamers and Spiegelmers

### Small scale synthesis

Aptamers and Spiegelmers were produced by solid-phase synthesis with an ABI 394 synthesizer (Applied Biosystems, Foster City, CA, USA) using 2'TBDMS RNA phosphoramidite chemistry (M.J. Damha, K.K. Ogilvie, Methods in Molecular Biology, Vol. 20 Protocols for oligonucleotides and analogs, ed. S. Agrawal, p. 81-114, Humana Press Inc. 1993). rA(N-Bz)-, rC(Ac)-, rG(N-ibu)-, and rU- phosphoramidites in the D- and L-configuration were purchased from ChemGenes, Wilmington, MA. Aptamers and Spiegelmers were purified by gel electrophoresis.

### Large scale synthesis plus modification

Spiegelmer NOX-E36 was produced by solid-phase synthesis with an ÄktaPilot100 synthesizer (Amersham Biosciences; General Electric Healthcare, Freiburg) using 2'TBDMS RNA phosphoramidite chemistry (M.J. Damha, K.K. Ogilvie, Methods in Molecular Biology, Vol. 20 Protocols for oligonucleotides and analogs, ed. S. Agrawal, p. 81-114, Humana Press Inc. 1993). L-rA(N-Bz)-, L-rC(Ac)-, L-rG(N-ibu)-, and L-rU-phosphoramidites were purchased from ChemGenes, Wilmington, MA. The 5'-amino-modifier was purchased from American International Chemicals Inc. (Framingham, MA, USA). Synthesis of the unmodified Spiegelmer was started on L-riboG modified CPG pore size 1000 Å (Link Technology, Glasgow, UK); for the 3'-NH₂-modified Spiegelmer, 3'-Aminomodifier-CPG, 1000 Å (ChemGenes, Wilmington, MA) was used. For coupling (15 min per cycle), 0.3 M benzylthiotetrazole (CMS-Chemicals, Abingdon, UK) in acetonitrile, and 3.5 equivalents of the respective 0.1 M phosphoramidite solution in acetonitrile was used. An oxidation-capping cycle was used. Further standard solvents and reagents for oligonucleotide synthesis were purchased from Biosolve (Valkenswaard, NL). The Spiegelmer was synthesized DMT-ON; after deprotection, it was purified via preparative RP-HPLC (Wincott F. et al. (1995) Nucleic Acids Res 23:2677) using Source15RPC medium (Amersham). The 5'DMT-group was removed with 80% acetic acid (30 min at RT). Subsequently, aqueous 2 M NaOAc solution was added and the Spiegelmer was desalted by tangential-flow filtration using a 5 K regenerated cellulose membrane (Millipore, Bedford, MA).

### PEGylation of NOX-E36

In order to prolong the Spiegelmer's plasma residence time in vivo, Spiegelmer NOX-E36 was covalently coupled to a 40 kDa polyethylene glycol (PEG) moiety at the 3'-end or 5'-end.

### 3'-PEGylation of NOX-E36

For PEGylation (for technical details of the method for PEGylation see European patent application EP 1 306 382), the purified 3'-amino modified Spiegelmer was dissolved in a mixture of H₂O (2.5 ml), DMF (5 ml), and buffer A (5 ml; prepared by mixing citric acid • H₂O [7 g], boric acid [3.54 g], phosphoric acid [2.26 ml], and 1 M NaOH [343 ml] and adding H2O to a final volume of 1 l; pH = 8.4 was adjusted with 1 M HCl).

The pH of the Spiegelmer solution was brought to 8.4 with 1 M NaOH. Then, 40 kDa PEG-NHS ester (Nektar Therapeutics, Huntsville, AL) was added at 37°C every 30 min in four portions of 0.6 equivalents until a maximal yield of 75 to 85% was reached. The pH of the reaction mixture was kept at 8 - 8.5 with 1 M NaOH during addition of the PEG-NHS ester.

The reaction mixture was blended with 4 ml urea solution (8 M), 4 ml buffer A, and 4 ml buffer B (0.1 M triethylammonium acetate in H₂O) and heated to 95°C for 15 min. The PEGylated Spiegelmer was then purified by RP-HPLC with Source 15RPC medium (Amersham), using an acetonitrile gradient (buffer B; buffer C: 0.1 M triethylammonium acetate in acetonitrile). Excess PEG eluted at 5% buffer C, PEGylated Spiegelmer at 10 - 15% buffer C. Product fractions with a purity of >95% (as assessed by HPLC) were combined and mixed with 40 ml 3 M NaOAC. The PEGylated Spiegelmer was desalted by tangential-flow filtration (5 K regenerated cellulose membrane, Millipore, Bedford MA).

### 5'-PEGylation of NOX-E36

For PEGylation (for technical details of the method for PEGylation see European patent application EP 1 306 382), the purified 5'-amino modified Spiegelmer was dissolved in a mixture of H₂O (2.5 ml), DMF (5 ml), and buffer A (5 ml; prepared by mixing citric acid • H₂O [7 g], boric acid [3.54 g], phosphoric acid [2.26 ml], and 1 M NaOH [343 ml] and adding water to a final volume of 1 l; pH = 8.4 was adjusted with 1 M HCl).

The pH of the Spiegelmer solution was brought to 8.4 with 1 M NaOH. Then, 40 kDa PEG-NHS ester (Nektar Therapeutics, Huntsville, AL) was added at 37°C every 30 min in six portions of 0.25 equivalents until a maximal yield of 75 to 85% was reached. The pH of the reaction mixture was kept at 8 - 8.5 with 1 M NaOH during addition of the PEG-NHS ester.

The reaction mixture was blended with 4 ml urea solution (8 M), and 4 ml buffer B (0.1 M triethylammonium acetate in H₂O) and heated to 95°C for 15 min. The PEGylated Spiegelmer was then purified by RP-HPLC with Source 15RPC medium (Amersham), using an acetonitrile gradient (buffer B; buffer C: 0.1 M triethylammonium acetate in acetonitrile). Excess PEG eluted at 5% buffer C, PEGylated Spiegelmer at 10 - 15% buffer C. Product fractions with a purity of >95% (as assessed by HPLC) were combined and mixed with 40 ml 3 M NaOAC. The PEGylated Spiegelmer was desalted by tangential-flow filtration (5 K regenerated cellulose membrane, Millipore, Bedford MA).

### Example 7: Determination of Binding Constants (Pull-Down Assay)

### Direct pull-down assay

The affinity of aptamers to D-MCP-1 was measured in a pull-down assay format at 20 or 37°C, respectively. Aptamers were 5'-phosphate labelled by T4 polynucleotide kinase (Invitrogen, Karlsruhe, Germany) using [γ-³²P]-labelled ATP (Hartmann Analytic, Braunschweig, Germany). The specific radioactivity of labeled aptamers was 200,000 - 800,000 cpm/pmol. Aptamers were incubated after de- and renaturation at 20 pM concentration at 37°C in selection buffer (20 mM Tris-HCl pH 7.4; 137 mM NaCl; 5 mM KCl; 1 mM MgCl₂; 1 mM CaCl₂; 0.1% [w/vol] Tween-20) together with varying amounts of biotinylated D-MCP-1 for 4 - 12 hours in order to reach equilibrium at low concentrations. Selection buffer was supplemented with 10 µg/ml human serum albumin (Sigma-Aldrich, Steinheim, Germany), and 10 µg/ml yeast RNA (Ambion, Austin, USA) in order to prevent adsorption of binding partners with surfaces of used plasticware or the immobilization matrix. The concentration range of biotinylated D-MCP-1 was set from 8 pM to 100 nM; total reaction volume was 1 ml. Peptide and peptide-aptamer complexes were immobilized on 1.5 µl Streptavidin Ultralink Plus particles (Pierce Biotechnology, Rockford, USA) which had been preequilibrated with selection buffer and resuspended in a total volume of 6 µl. Particles were kept in suspension for 30 min at the respective temperature in a thermomixer. Immobilized radioactivity was quantitated in a scintillation counter after detaching the supernatant and appropriate washing. The percentage of binding was plotted against the concentration of biotinylated D-MCP-1 and dissociation constants were obtained by using software algorithms (GRAFIT; Erithacus Software; Surrey U.K.) assuming a 1:1 stoichiometry.

### Competitive pull-down assay

In order to compare different D-MCP-1 binding aptamers, a competitive ranking assay was performed. For this purpose, the most affine aptamer available was radioactively labelled (see above) and served as reference. After de- and renaturation it was incubated at 37°C with biotinylated D-MCP-1 in 1 ml selection buffer at conditions that resulted in around 5 - 10 % binding to the peptide after immobilization and washing on NeutrAvidin agarose or Streptavidin Ultralink Plus (both from Pierce) without competition. An excess of de- and renatured non-labelled D-RNA aptamer variants was added to different concentrations (e.g. 2, 10, and 50 nM) with the labelled reference aptamer to parallel binding reactions. The aptamers to be tested competed with the reference aptamer for target binding, thus decreasing the binding signal in dependence of their binding characteristics. The aptamer that was found most active in this assay could then serve as a new reference for comparative analysis of further aptamer variants.

### Example 8: Determination of Inhibitory Concentration in a Ca⁺⁺-Release Assay

THP-1-cells (DSMZ, Braunschweig) were cultivated overnight at a cell density of 0.3 x 10⁶/ml at 37°C and 5% CO₂ in RPMI 1640 medium with GlutaMAX (Invitrogen) which contained in addition 10% fetal calf serum, 50 units/ml penicillin, 50 µg/ml streptomycin and 50 µM β-mercaptoethanol.

The Spiegelmers were incubated together with recombinant human MCP-1 (Bachem) in Hanks balanced salt solution (HBSS), containing 1 mg/ml bovine serum albumin, 5 mM probenecid and 20 mM HEPES (HBSS+) for 15 to 60 min at 37°C in a 0.2 ml low profile 96-tube plate ("stimulation solution").

For loading with the calcium indicator dye, cells were centrifuged at 300 x g for 5 min, resuspended in 4 ml indicator dye solution (10 µM fluo-4 [Molecular Probes], 0.08% pluronic 127 [Molecular Probes] in HBSS+) and incubated for 60 min at 37°C. Thereafter, 11 ml HBSS+ were added, and the cells were centrifuged as above, washed once with 15 ml HBSS+ and then resuspended in HBSS+ to give a cell density of 1.1 x 10⁶/ml. 90 µl of this cell suspension were added to each well of a black 96-well plate.

Measurement of fluorescence signals was done at an excitation wavelength of 485 nm and an emission wavelength of 520 nm in a Fluostar Optima multidetection plate reader (BMG). For parallel measurement of several samples, wells of one (perpendicular) row of a 96-well plate were recorded together. First three readings with a time lag of 4 sec were done for determination of the base line. Then the recording was interrupted, and the plate was moved from the instrument. Using a multi-channel pipette, 10 µl of the stimulation solution was added to the wells, then the plate was moved into the instrument again and the measurement was continued. In total, 20 recordings with time intervals of 4 seconds were performed.

For each well the difference between maximal fluorescence and base line value was determined and plotted against MCP-1 concentration or, in the experiments on the inhibition of calcium release by Spiegelmers, against concentration of Spiegelmer.

### Determination of half-maximal effective concentration (EC₅₀) for human MCP-1

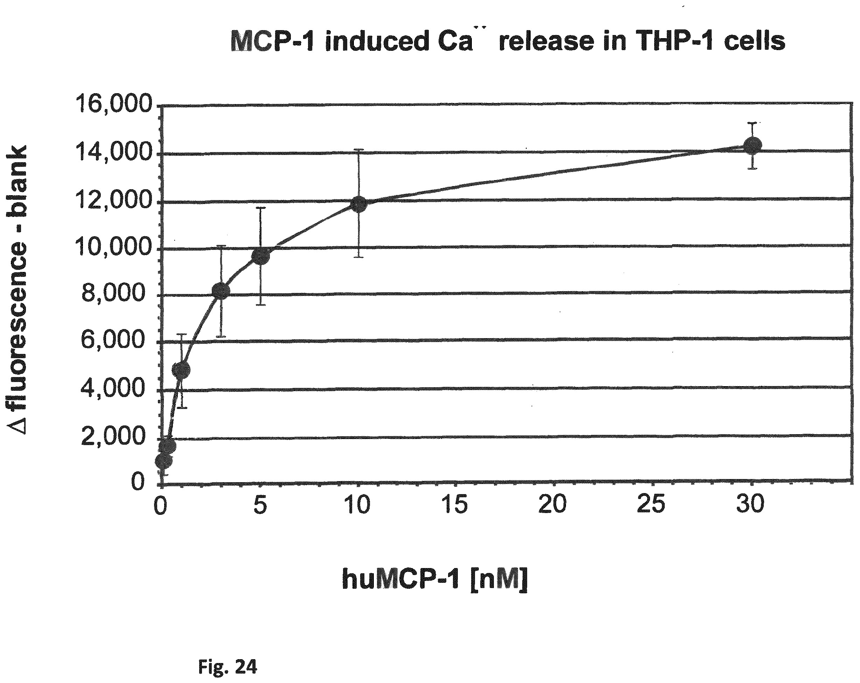

After stimulation of THP-1 cells with various hMCP-1 concentrations and plotting the difference between the maximal and the baseline signals, a dose-response curve for human MCP-1 was obtained, indicating a half effective concentration (EC₅₀) of about 2 - 4 nM (Fig. 24). This concentration was used for the further experiments on inhibition of Ca⁺⁺-release by Spiegelmers.

### Determination of half-maximal effective concentration (EC₅₀) for murine MCP-1

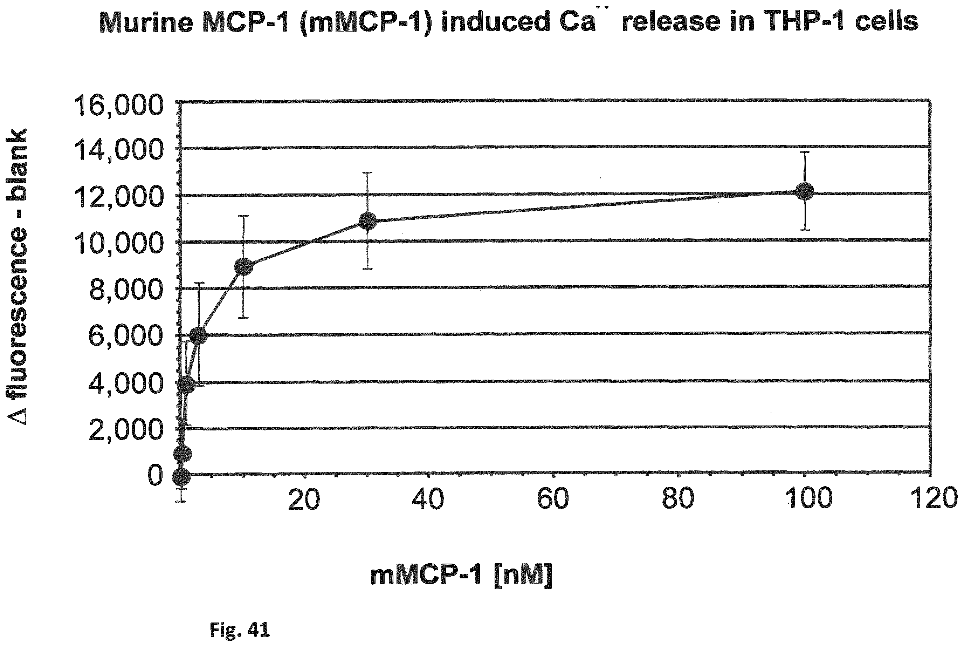

After stimulation of THP-1 cells with various mMCP-1 concentrations and plotting the difference between the maximal and the baseline signals, a dose-response curve for murine MCP-1 was obtained, indicating a half effective concentration (EC₅₀) of about 5 nM (Fig. 41). This concentration was used for the further experiments on inhibition of Ca⁺⁺-release by Spiegelmers.

### Example 9: Determination of Inhibitory Concentration in a Chemotaxis Assay

THP-1 cells grown as described above were centrifuged, washed once in HBH (HBSS, containing 1 mg/ml bovine serum albumin and 20 mM HEPES) and resuspended at 3 x 10⁶ cells/ml. 100 µl of this suspension were added to Transwell inserts with 5 µm pores (Corning, #3421). In the lower compartments MCP-1 was preincubated together with Spiegelmers in various concentrations in 600 µl HBH at 37°C for 20 to 30 min prior to addition of cells. Cells were allowed to migrate at 37°C for 3 hours. Thereafter the inserts were removed and 60 µl of 440 µM resazurin (Sigma) in phosphate buffered saline was added to the lower compartments. After incubation at 37°C for 2.5 hours, fluorescence was measured at an excitation wavelength of 544 nm and an emission wavelength of 590 nm in a Fluostar Optima multidetection plate reader (BMG).

### Determination of half-maximal effective concentration (EC₅₀) for human MCP-1

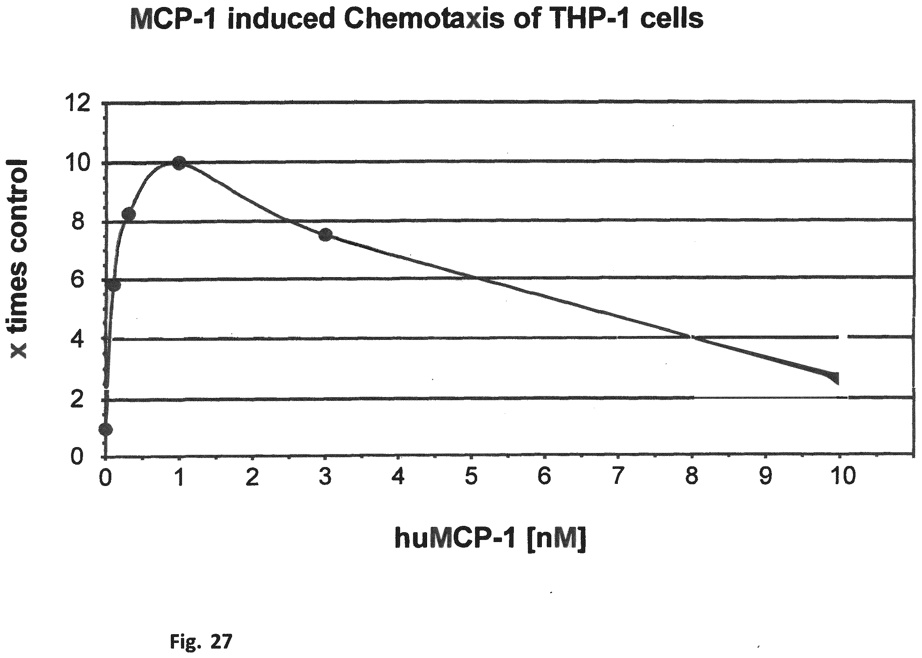

After 3 hours migration of THP-1 cells towards various human MCP-1 concentrations, a dose-response curve for human MCP-1 was obtained, indicating a maximal effective concentration of about 1 nM and reduced activation at higher concentrations (Fig. 27). For the further experiments on inhibition of chemotaxis by Spiegelmers a MCP-1 concentration of 0.5 nM was used.

### Determination of half-maximal effective concentration (EC₅₀) for murine MCP-1

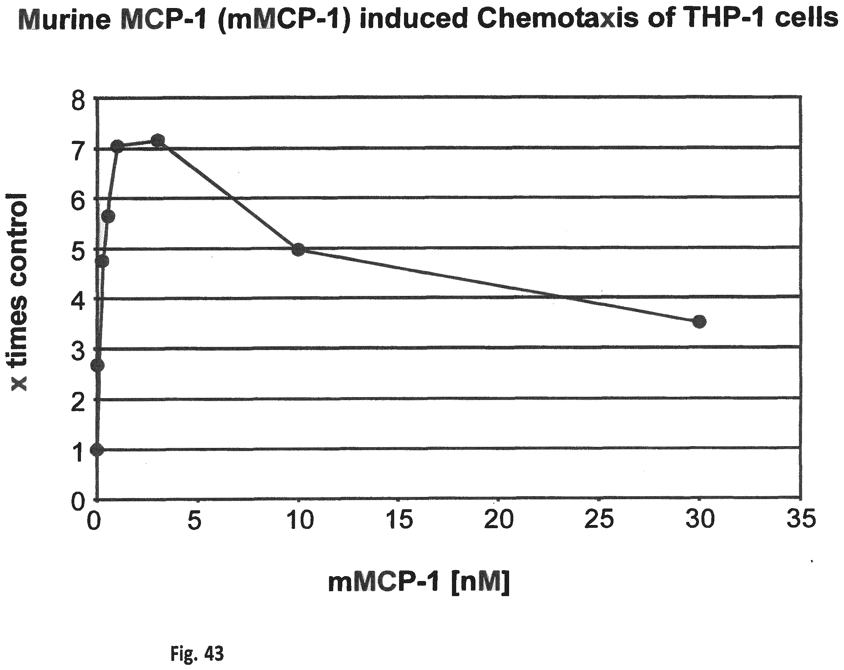

After 3 hours migration of THP-1 cells towards various murine MCP-1 concentrations, a dose-response curve for murine MCP-1 was obtained, indicating a maximal effective concentration of about 1 - 3 nM and reduced activation at higher concentrations (Fig. 43). For the further experiments on inhibition of chemotaxis by Spiegelmers a murine MCP-1 concentration of 0.5 nM was used.

### Example 10: Binding Analysis by Surface Plasmon Resonance Measurement

### 10.1 Specificity assessment of NOX-E36, 181-A2-018 and mNOX-E36

The Biacore 2000 instrument (Biacore AB, Uppsala, Sweden) was used to analyze binding of nucleic acids to human MCP-1 and related proteins. When coupling was to be achieved via amine groups, the proteins were dialyzed against water for 1 - 2 h (Millipore VSWP mixed cellulose esters; pore size, 0.025 µM) to remove interfering amines. PioneerF1 or CM4 sensor chips (Biacore AB) were activated before protein coupling by a 35-µl injection of a 1:1 dilution of 0.4 M NHS and 0.1 M EDC at a flow of 5 µl/min. Chemokine was then injected in concentrations of 0.1 - 1.5 µg/ml at a flow of 2 µl/min until the instrument's response was in the range of 1000 - 2000 RU (relative units). Unreacted NHS esters were deactivated by injection of 35 µl ethanolamine hydrochloride solution (pH 8.5) at a flow of 5 µl/min. The sensor chip was primed twice with binding buffer and equilibrated at 10 µl/min for 1 - 2 hours until the baseline appeared stable. For all proteins, kinetic parameters and dissociation constants were evaluated by a series of Spiegelmer injections at concentrations of 1000, 500, 250, 125, 62.5, 31.25, and 0 nM in selection buffer (Tris-HCl, 20 mM; NaCl, 137 mM; KCl, 5 mM; CaCl₂, 1 mM; MgCl₂, 1 mM; Tween20, 0.1% [w/v]; pH 7.4). In all experiments, the analysis was performed at 37°C using the Kinject command defining an association time of 180 and a dissociation time of 360 seconds at a flow of 10 µl/min. Data analysis and calculation of dissociation constants (K_{D}) was done with the BIAevaluation 3.0 software (BIACORE AB, Uppsala, Sweden) using the Langmuir 1:1 stochiometric fitting algorithm.

### 10.1.1 NOX-E36 and 181-A2-018 (human-MCP-1 specific nucleic acids)

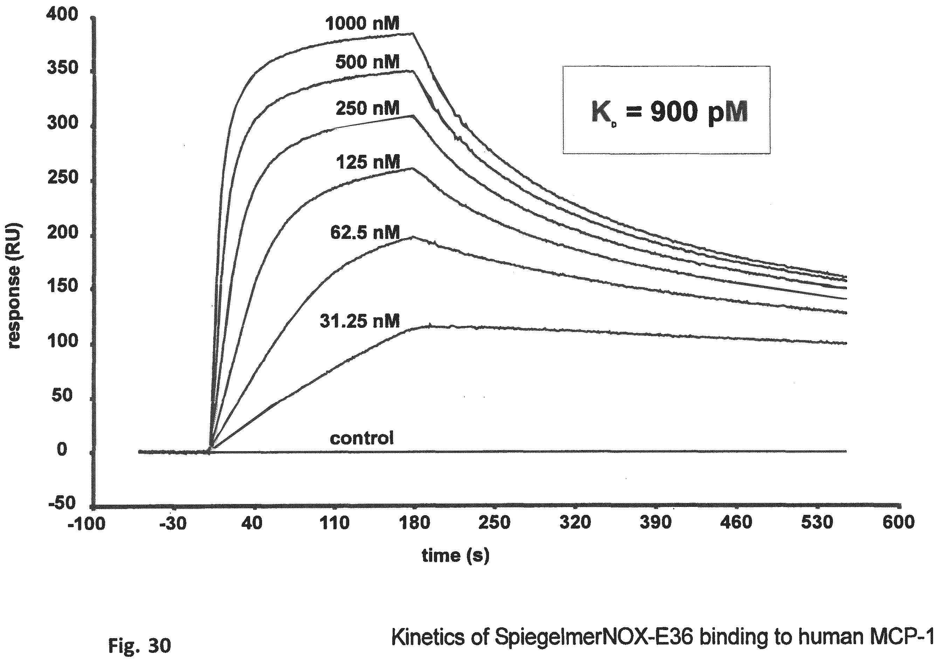

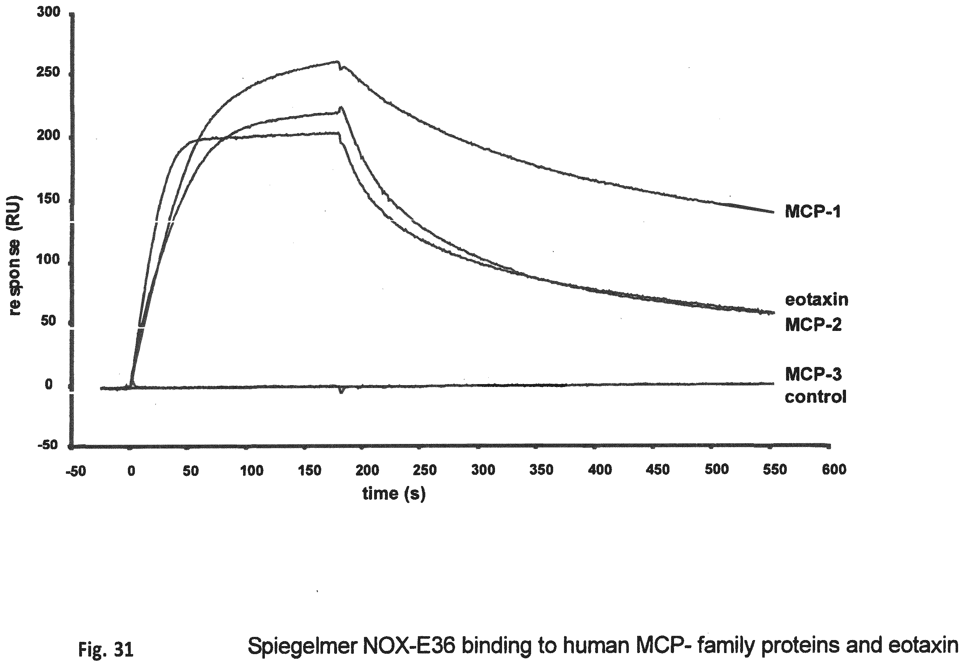

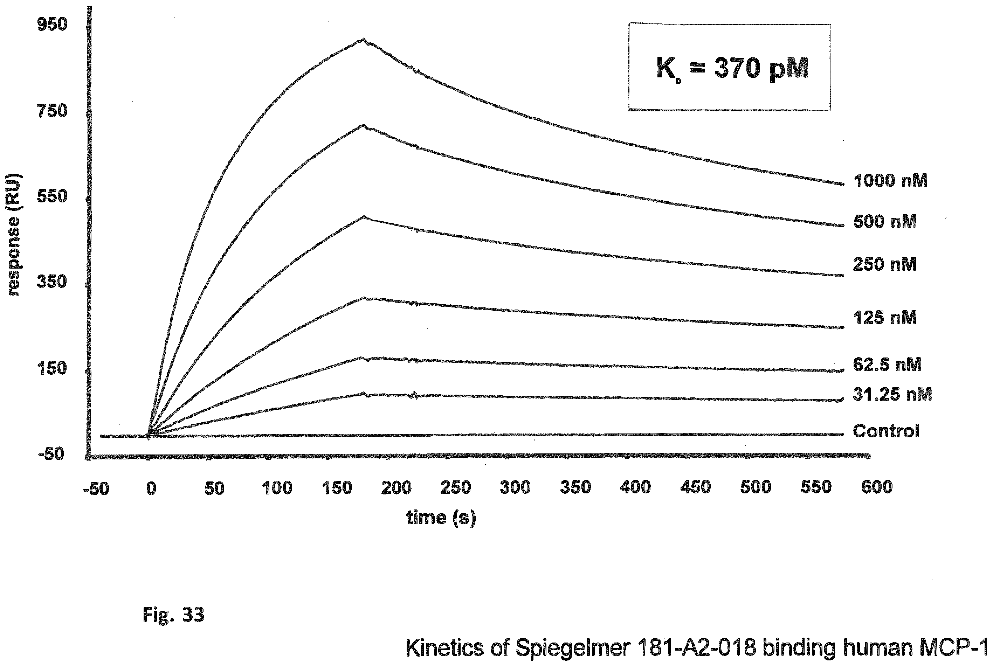

Only for human MCP-1 all sensorgrams are depicted (Figs 30 and 33, respectively); for the other proteins, only the sensorgram obtained with 125 nM Spiegelmer concentration is shown for sake of clarity (Figs. 31/32 and 34/35).

Analysis of the NOX-E36•hMCP-1 interaction: recombinant human MCP-1 was immobilized on a PioneerF1 sensor chip following the manufacturer's recommendations (amine coupling procedure) until an instrument response of 1381 RU (relative units) was established. The determined dissociation constant (K_{D}) for NOX-E36 binding to human MCP-1 was ca. 890 pM (Fig. 30).

Analysis of the 181-A2-018•hMCP-1 interaction: recombinant human MCP-1 was immobilized on a CM4 sensor chip following the manufacturer's recommendations (amine coupling procedure) until an instrument response of 3111 RU (relative units) was established. The determined dissociation constant (K_{D}) for 181-A2-018 binding to human MCP-1 was ca. 370 pM (Fig. 33).

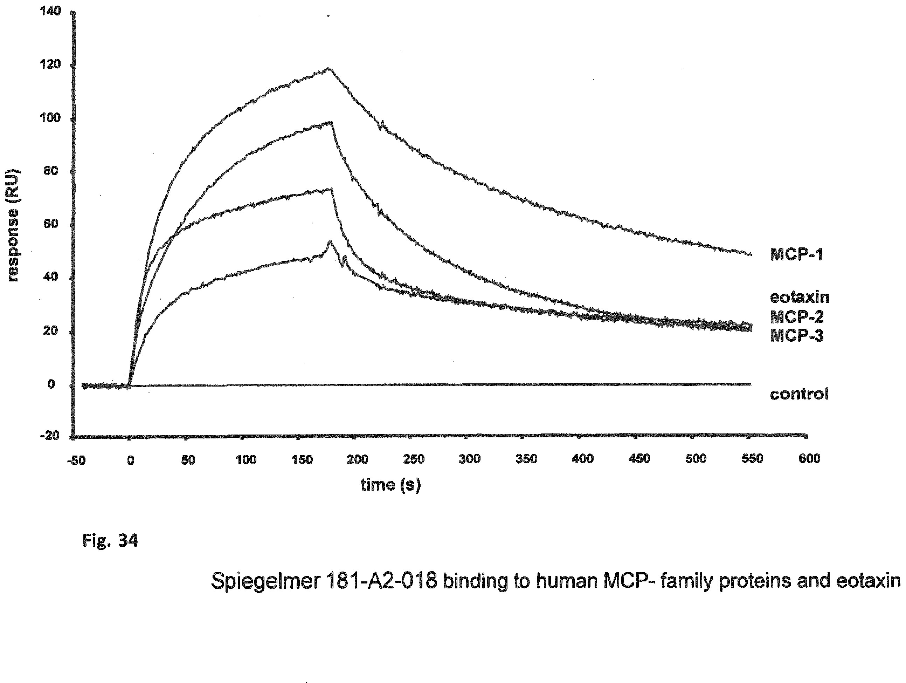

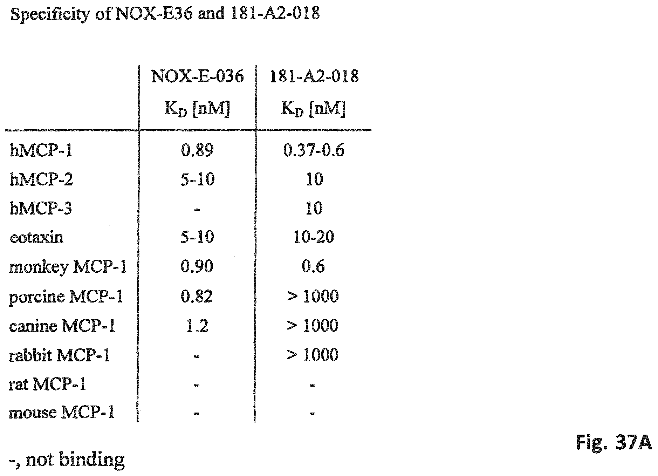

To determine the specificity of NOX-E36 and 181-A2-018, various human MCP-1 family proteins as well as human eotaxin were immobilized on a PioneerF1 and a CM4 sensor chip (hMCP-1, 1754 RU; hMCP-2, 1558 RU; hMCP-3, 1290 RU; eotaxin, 1523 RU). Kinetic analysis revealed that NOX-E36 binds to eotaxin and hMCP-2 with dissociation constants (K_{D}) of 5 - 10 nM; hMCP-3 was not recognized (Figs. 31 and 37A). 181-A2-018, in contrast, binds eotaxin, hMCP-2 and hMCP-3, but with slightly lower affinity (10 - 20 nM; Figs. 34 and 37A).

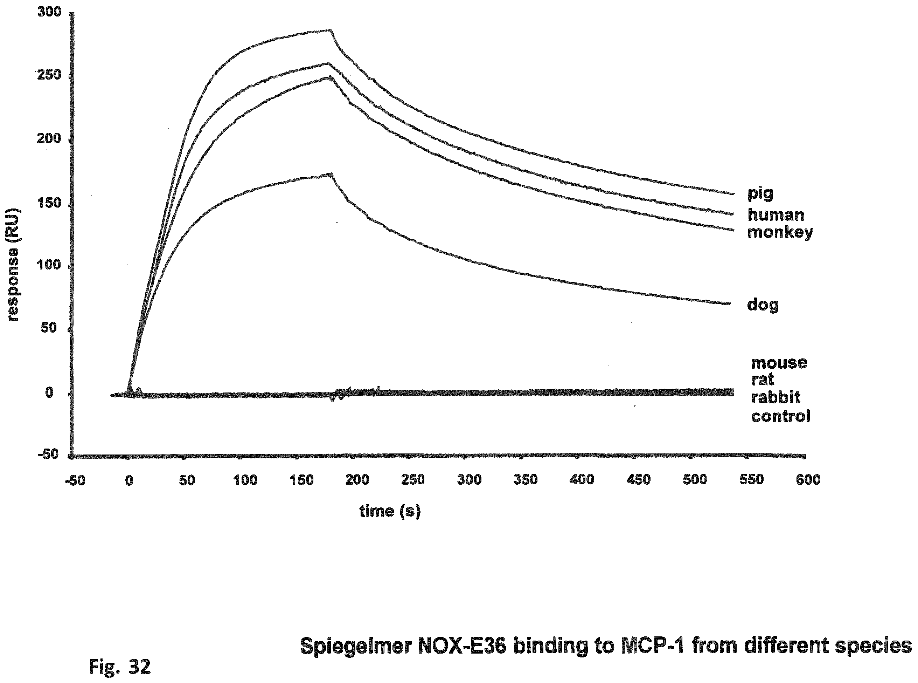

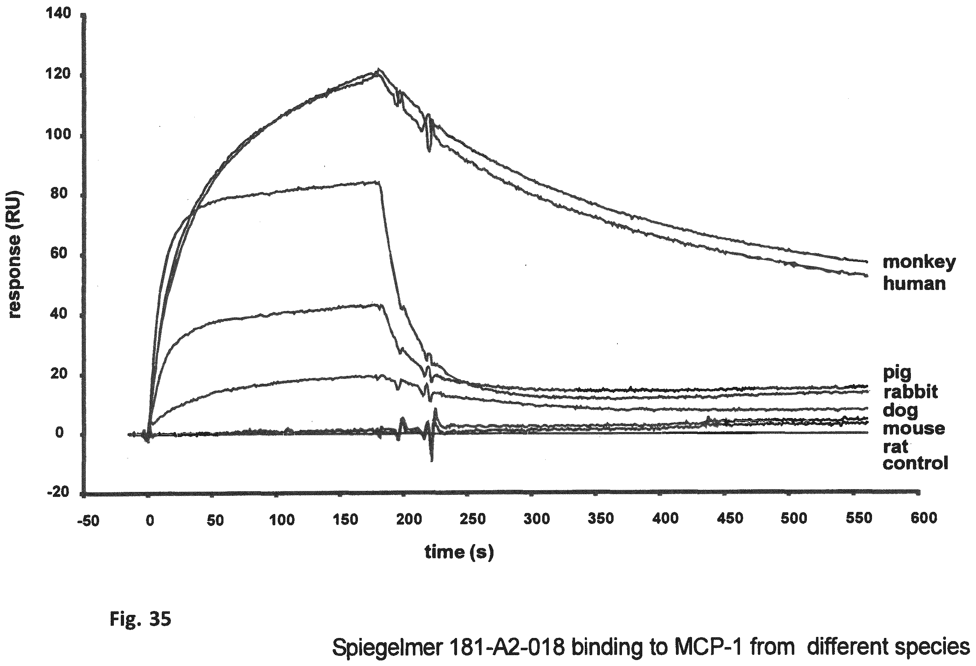

Interspecies cross-reactivity of NOX-E36 and 181-A2-018 was assessed using amino-coupling immobilized MCP-1 from human (1460 RU), monkey (1218 RU), pig (1428 RU), dog (1224 RU), rabbit (1244 RU), rat (1267 RU), and mouse (1361 RU) on a PioneerF1 and a CM4 sensor chip. Kinetic analysis revealed that NOX-E36 binds to human, monkey, porcine, and canine MCP-1 with comparable dissociation constants (K_{D}) of 0.89 - 1.2 nM whereas MCP-1 from mouse, rat and rabbit were not recognized (Figs. 32 and 37A). 181-A2-018 binds to human and monkey MCP-1 with comparable dissociation constants (K_{D}) of 0.5-0.6 nM, whereas porcine, rabbit and canine MCP-1 are bound with much lower affinity. Rat and mouse MCP-1 were not recognized by NOX-A2-018 (Figs. 35 and 37A).

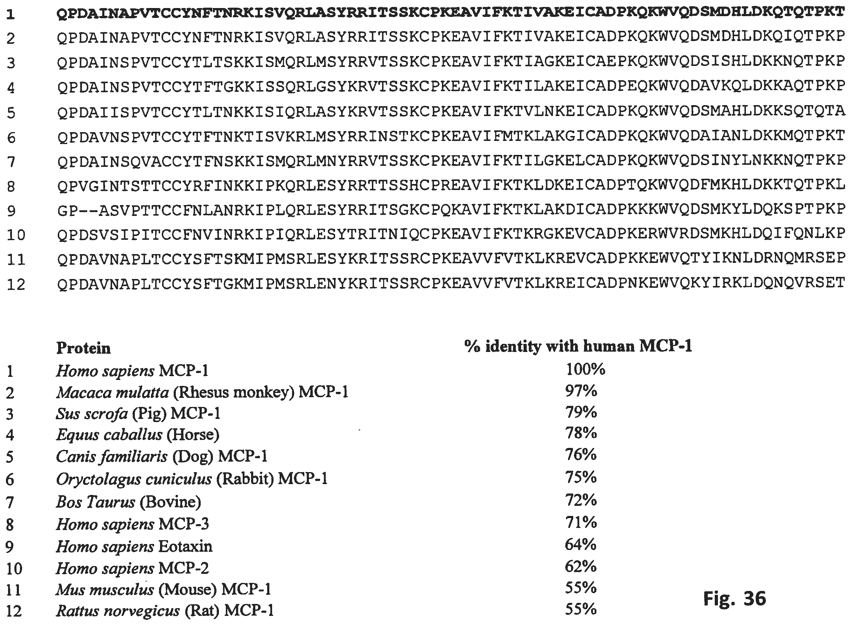

Sequences as well as degree of homology in percent identical amino acids between the MCP-1 protein from different species and closely related human proteins are depicted in Fig. 36; calculated KD values for NOX-E36 and 181-A2-018 are displayed in tabular format in Fig. 37A.

### 10.1.2 mNOX-E36 (murine MCP-1 specific nucleic acid)

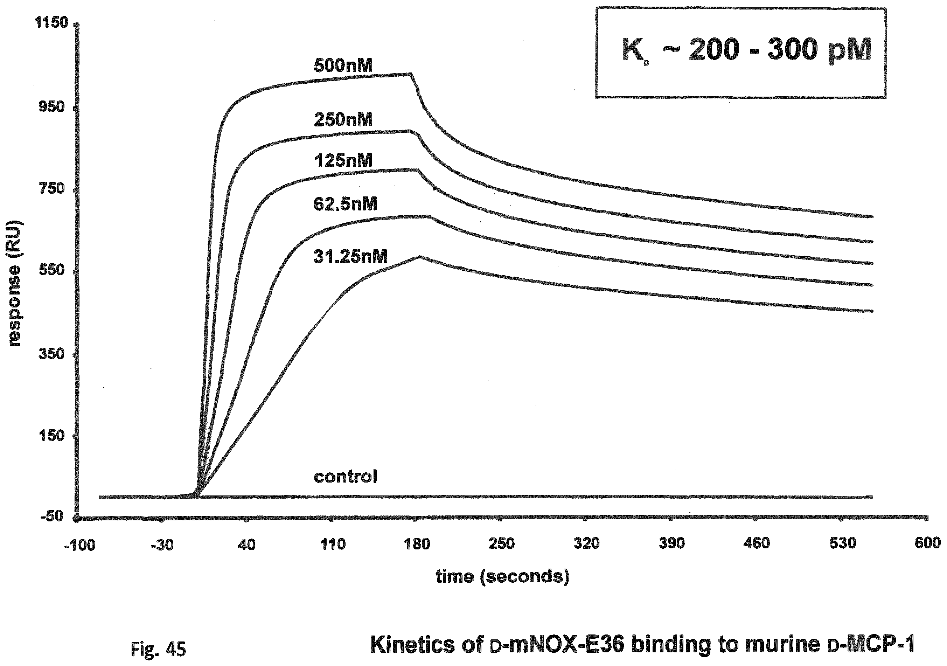

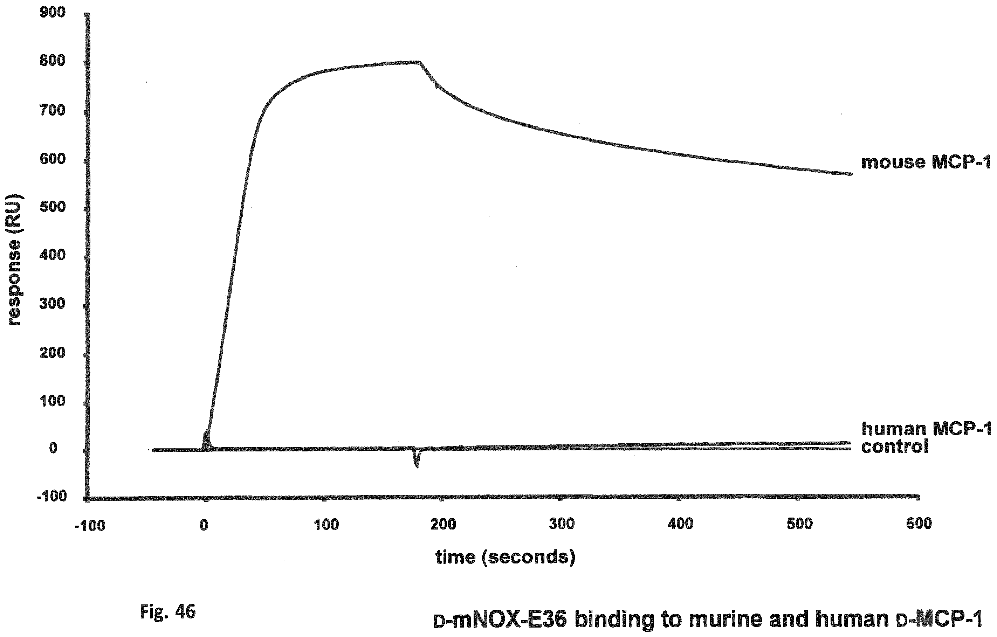

To analyze the binding behaviour of mNOX-E36, 3759 RU of synthetic biotinylated murine D-MCP-1 (flow cell 3) and 3326 RU of biotinylated human D-MCP-1 (flow cell 4) were immobilized on a Streptavidin conjugated sensor chip (Biacore AB, Freiburg, Germany), respectively. mNOX-E36 aptamer (D-RNA) solutions of 500, 250, 125, 62.5, 31.25, and 0 nM were injected using the Kinject command defining an association time of 180 sec and a dissociation time of 360 sec. Flow cell 1 was used as buffer and dextran matrix control (Biacore SA-Chip surface) whereas on flow cell 2, an unspecific D-peptide was immobilized to determine unspecific binding of the aptamer. Fig. 45 shows a sensorgram of the D-NOX-E36 kinetic for binding to murine D-MCP-1 with a calculated dissociation constant (K_{D}) of 200 - 300 pM. mNOX-E36 does not bind human D-MCP-1 (Fig. 46); for sake of clarity, only the sensorgram obtained with 125 nM Spiegelmer is shown.

### 10.2 Selectivity assessment of NOX-E36

Selectivity of NOX-E36 was assessed by surface plasmon resonance analysis by immobilizing 5'biotinylated NOX-E36 on a Streptavidin (SA-Chip). 352 RU of NOX-E36 on flowcell (FC) 1 and equal amount of 5'-terminal biotinylated non-functional control Spiegelmer (POC) on FC 2 were immobilized by streptavidin/biotin binding. FC3 was used as surface control to determine unspecific binding to the dextran-SA sensor surface.

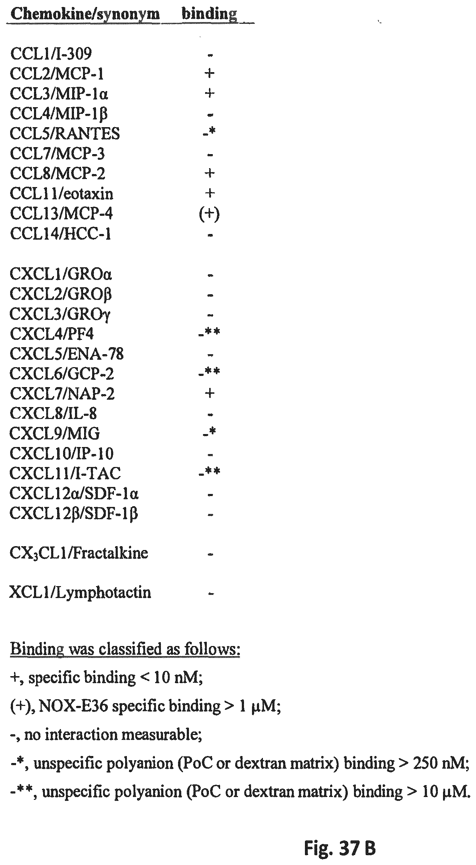

100 nM of a panel of human chemokines from all four subgroups (CC, CXC, CX₃C, and XC) were injected for 360s and complexes were allowed to dissociate for 360s at a flow of 10µl/min and 37°C. Response units after association (Resp.1; degree of interaction) and after dissociation (Resp.2, affinity of interaction) were plotted. After each injection the chip surface was regenerated with a 240s of 1 M sodium chloride with 0,1% Tween; immobilized Spiegelmers were subsequently allowed to refold for 2 minutes at physiological conditions (running buffer). Injection of each chemokine was repeated 3 times. CXCL1, CXCL2, CXCL6 and CXCL9 showed unspecific binding to ribonucleic acids and chip dextran surface. Specific high-affinity binding to immobilized NOX-E36 could only be detected for CCL2/MCP-1, CCL8/MCP-2, CCL11/eotaxin, CCL3/MIP1α, and CXCL7/NAP-2 (Fig. 37B). The finding that MCP-2 and eotaxin are bound by NOX-E36 is not surprising due to the relatively high homology between these chemokines and MCP-1 of 62 and 70 %, for the unexpected positives CCL3/MIP-1α and CXCL7/NAP-2, *in vitro* tests for functional inhibition have been performed or are currently being established, respectively.

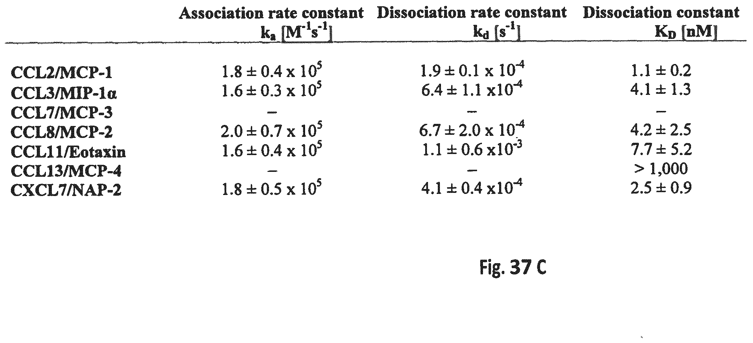

Finally, the kinetic parameters of interaction between NOX-E36 and CCL2/MCP-1, CCL8/MCP-2, CCL11/eotaxin, CCL3/MIP1α, CXCL7/NAP-2, CCL7/MCP-3 and CCL13/MCP-4 were determined in the "inverted" system. Here, the chemokines were immobilized and free NOX-E36 was injected (for the detailed protocol, see 10.1). Kinetic data are summarized in Fig. 37C.

### 10.3 Assessment of anti-MIP-1α Functionality in vitro

Biacore measurements had shown cross reactivity of NOX-E36 with MIP-1α. By employing a functional, cell culture-based *in vitro* assay it should be checked if mere Biacore binding of NOX-E36 to MIP-1α also translates to functionality, e.g. antagonism.

To achieve this, chemotaxis experiments with THP-1 cells were performed that can be stimulated by MIP-1 α. THP-1 cells grown as described above were centrifuged, washed once in HBH (HBSS, containing 1 mg/ml bovine serum albumin and 20 mM HEPES) and resuspended at 3 x 10⁶ cells/ml. 100 µl of this suspension were added to Transwell inserts with 5 µm pores (Corning, #3421). In the lower compartments MIP-1α was preincubated together with Spiegelmers in various concentrations in 600 µl HBH at 37°C for 20 to 30 min prior to addition of cells. Cells were allowed to migrate at 37°C for 3 hours. Thereafter the inserts were removed and 60 µl of 440 µM resazurin (Sigma) in phosphate buffered saline was added to the lower compartments. After incubation at 37°C for 2.5 hours, fluorescence was measured at an excitation wavelength of 544 nm and an emission wavelength of 590 nm in a Fluostar Optima multidetection plate reader (BMG).

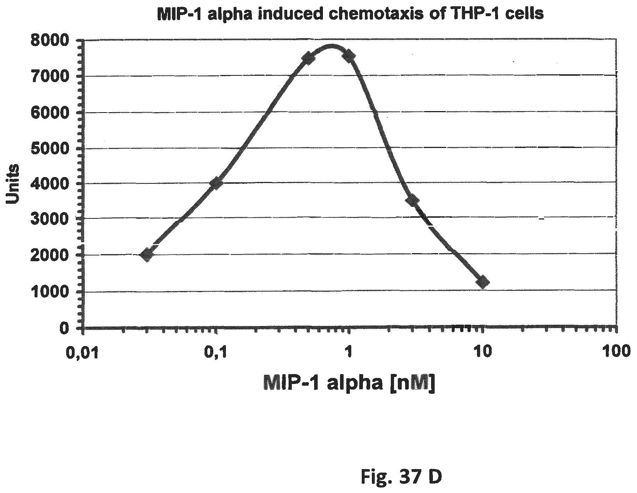

After 3 hours migration of THP-1 cells towards various human MIP-1α concentrations, a dose-response curve for human MIP-1α was obtained, indicating a half-maximal effective concentration of about 1 nM and reduced activation at higher concentrations (Fig. 37D). For the further experiments on inhibition of chemotaxis by Spiegelmers a MIP-1α concentration of 0.5 nM was used.

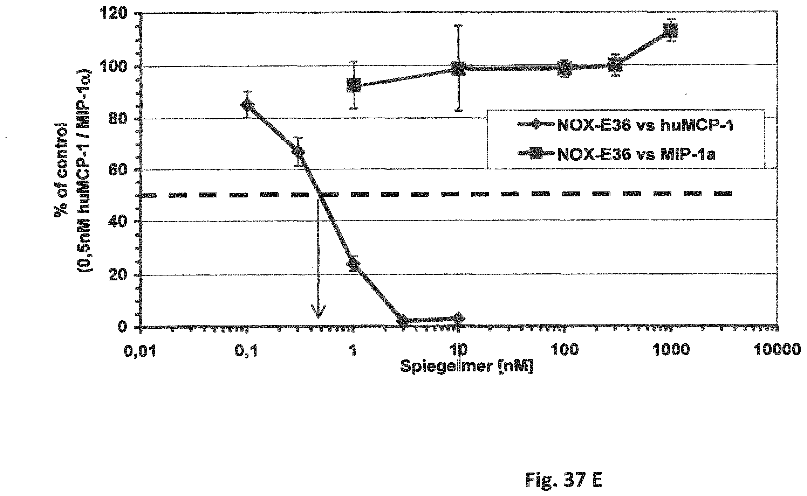

Experiments for determination of chemotaxis inhibition by NOX-E36 were performed with a stimulus of 0.5 nM MIP-1α. It could be clearly shown that NOX-E36 does not inhibit MIP-1α induced chemotaxis up to the highest tested concentration of 1 µM MIP-1α. As positive control, the respective experiment with MCP-1 as stimulus was performed in parallel (Fig. 37E).

### REFERENCES

The complete bibliographic data of the documents recited herein the disclosure of which is incorporated by reference is, if not indicated to the contrary, as follows.
Ahmed, II (2015). "MIGS and the FDA: What's in a Name?" Ophthalmology 122(9): 1737-1739.
Anand, N. and S. Arora (2007). "Surgical revision of failed filtration surgery with mitomycin C augmentation." J Glaucoma 16(5): 456-461.
Anand, N., S. Arora and M. Clowes (2006). "Mitomycin C augmented glaucoma surgery: evolution of filtering bleb avascularity, transconjunctival oozing, and leaks." Br J Ophthalmol 90(2): 175-180.
Armendariz, B. G. and U. Chakravarthy (2024). "Fibrosis in age-related neovascular macular degeneration in the anti-VEGF era." Eye (Lond) 38(17): 3243-3251.
Balas, M. and D. J. Mathew (2023). "Minimally Invasive Glaucoma Surgery: A Review of the Literature." Vision (Basel) 7(3).
Bitrian, E., B. J. Song and J. Caprioli (2014). "Bleb revision for resolution of hypotony maculopathy following primary trabeculectomy." Am J Ophthalmol 158(3): 597-604 e591.
Bloom, P. and L. Au (2018). ""Minimally Invasive Glaucoma Surgery (MIGS) Is a Poor Substitute for Trabeculectomy"-The Great Debate." Ophthalmol Ther 7(2): 203-210.
Boring, L., J. Gosling, S. W. Chensue, S. L. Kunkel, R. V. Farese, Jr., H. E. Broxmeyer and I. F. Charo (1997). "Impaired monocyte migration and reduced type 1 (Th1) cytokine responses in C-C chemokine receptor 2 knockout mice." J Clin Invest 100(10): 2552-2561.
Broadway, D. C., P. A. Bloom, C. Bunce, M. Thiagarajan and P. T. Khaw (2004). "Needle revision of failing and failed trabeculectomy blebs with adjunctive 5-fluorouracil: survival analysis." Ophthalmology 111(4): 665-673.
Capitena Young, C. E., Ammar, D. A., Seibold, L. K., Pantcheva, M. B., SooHoo, J. R., Kahook, M. Y, (2018). "Histopathologic Examination of Trabecular Meshwork Changes After Trabecular Bypass Stent Implantation." L Glaucoma 27(7): 606.
Chawla, A., K. Mercieca, C. Fenerty and N. P. Jones (2013). "Outcomes and complications of trabeculectomy enhanced with 5-fluorouracil in adults with glaucoma secondary to uveitis." J Glaucoma 22(8): 663-666.
Chen, C. W., H. T. Huang, J. S. Bair and C. C. Lee (1990). "Trabeculectomy with simultaneous topical application of mitomycin-C in refractory glaucoma." J Ocul Pharmacol 6(3): 175-182.
Chiu, H. I., H. I. Su, Y. C. Ko and C. J. Liu (2022). "Outcomes and risk factors for failure after trabeculectomy in Taiwanese patients: medical chart reviews from 2006 to 2017." Br J Ophthalmol 106(3): 362-367.
Chong, R. S., Y S. Lee, S. W. L. Chu, L. Z. Toh and T. T. L. Wong (2017). "Inhibition of Monocyte Chemoattractant Protein 1 Prevents Conjunctival Fibrosis in an Experimental Model of Glaucoma Filtration Surgery." Invest Ophthalmol Vis Sci 58(9): 3432-3439.
Conlon, R., H. Saheb and Ahmed, II (2017). "Glaucoma treatment trends: a review." Can J Ophthalmol 52(1): 114-124.
Coote, M. A., V. Gupta, S. Vasudevan and J. G. Crowston (2011). "Posterior revision for failed blebs: long-term outcomes." J Glaucoma 20(6): 377-382.
Craven, E. R., I. P. Singh, T. M. Yu, S. Rhoten, O. R. Sadruddin and A. Sheybani (2022). "Reoperation Rates and Disease Costs for Primary Open-Angle Glaucoma Patients in the United States Treated with Incisional Glaucoma Surgery." Ophthalmol Glaucoma 5(3): 297-305.
de Leon, J. M. S. and C. M. G. Pionela (2021). "Outcomes of primary trabeculectomy with mitomycin-C for primary angle closure glaucoma among supervised trainees in a tertiary eye center in Manila." Int Ophthalmol 41(5): 1643-1650.
Ederer, F., D. A. Gaasterland, L. G. Dally, J. Kim, P. C. VanVeldhuisen, B. Blackwell, B. Prum, G. Shafranov, R. C. Allen, A. Beck and A. Investigators (2004). "The Advanced Glaucoma Intervention Study (AGIS): 13. Comparison of treatment outcomes within race: 10-year results." Ophthalmology 111(4): 651-664.
Edmunds, B., J. R. Thompson, J. F. Salmon and R. P. Wormald (2002). "The National Survey of Trabeculectomy. III. Early and late complications." Eye (Lond) 16(3): 297-303.
Fan Gaskin, J. C., D. Q. Nguyen, G. Soon Ang, J. O'Connor and J. G. Crowston (2014). "Wound Healing Modulation in Glaucoma Filtration Surgery-Conventional Practices and New Perspectives: The Role of Antifibrotic Agents (Part I)." J Curr Glaucoma Pract 8(2): 37-45.
Frade, J. M., M. Mellado, G. del Real, J. C. Gutierrez-Ramos, P. Lind and A. C. Martinez (1997). "Characterization of the CCR2 chemokine receptor: functional CCR2 receptor expression in B cells." J Immunol 159(11): 5576-5584.
Franceschi, C., M. Bonafe, S. Valensin, F. Olivieri, M. De Luca, E. Ottaviani and G. De Benedictis (2000). "Inflamm-aging. An evolutionary perspective on immunosenescence." Ann N Y Acad Sci 908: 244-254.
Gedde, S. J., J. C. Schiffman, W. J. Feuer, L. W. Herndon, J. D. Brandt, D. L. Budenz and G. Tube versus Trabeculectomy Study (2012). "Treatment outcomes in the Tube Versus Trabeculectomy (TVT) study after five years of follow-up." Am J Ophthalmol 153(5): 789-803 e782.
Green, E., M. Wilkins, C. Bunce and R. Wormald (2014). "5-Fluorouracil for glaucoma surgery." Cochrane Database Syst Rev 2014(2): CD001132.
Gressel, M. G., R. K. Parrish, 2nd and R. Folberg (1984). "5-fluorouracil and glaucoma filtering surgery: I. An animal model." Ophthalmology 91(4): 378-383.
Grover, D. S., H. L. Kornmann and R. L. Fellman (2020). "Historical Considerations and Innovations in the Perioperative Use of Mitomycin C for Glaucoma Filtration Surgery and Bleb Revisions." J Glaucoma 29(3): 226-235.
Hattenhauer, M. G., D. H. Johnson, H. H. Ing, D. O. Hodge, L. C. Butterfield, D. C. Herman and D. T. Gray (1999). "Probability of filtration surgery in patients with open-angle glaucoma." Arch Ophthalmol 117(9): 1211-1215.
Heuer, D. K., R. K. Parrish, 2nd, M. G. Gressel, E. Hodapp, P. F. Palmberg and D. R. Anderson (1984). "5-fluorouracil and glaucoma filtering surgery. II. A pilot study." Ophthalmology 91(4): 384-394.
Hollo, G. (2017). "Wound Healing and Glaucoma Surgery: Modulating the Scarring Process with Conventional Antimetabolites and New Molecules." Dev Ophthalmol 59: 80-89.
Hu, W. and S. Y. Wang (2022). "Predicting Glaucoma Progression Requiring Surgery Using Clinical Free-Text Notes and Transfer Learning With Transformers." Transl Vis Sci Technol 11(3): 37.
Hubner, L., U. Schlotzer-Schrehardt, J. M. Weller, B. Hohberger, C. Y. Mardin and R. Lammer (2022). "Ultrastructural analysis of explanted CyPass microstents and correlation with clinical findings." Graefes Arch Clin Exp Ophthalmol 260(8): 2663-2673.
Jamjoom, H., M. Osman and E. A. Osman (2020). "Overfiltering Bleb en Route to Annular Ciliochoroidal Effusion." Middle East Afr J Ophthalmol 27(4): 241-243.
Kaplan, H. J. (2007). "Anatomy and function of the eye." Chem Immunol Allergy 92: 4-10.
Khaw, P. T., M. B. Sherwood, S. L. MacKay, M. J. Rossi and G. Schultz (1992). "Five-minute treatments with fluorouracil, floxuridine, and mitomycin have long-term effects on human Tenon's capsule fibroblasts." Arch Ophthalmol 110(8): 1150-1154.
Koike, K. J. and P. T. Chang (2018). "Trabeculectomy: A Brief History and Review of Current Trends." Int Ophthalmol Clin 58(3): 117-133.
Kurihara, T., G. Warr, J. Loy and R. Bravo (1997). "Defects in macrophage recruitment and host defense in mice lacking the CCR2 chemokine receptor." J Exp Med 186(10): 1757-1762.
Kuziel, W. A., S. J. Morgan, T. C. Dawson, S. Griffin, O. Smithies, K. Ley and N. Maeda (1997). "Severe reduction in leukocyte adhesion and monocyte extravasation in mice deficient in CC chemokine receptor 2." Proc Natl Acad Sci U S A 94(22): 12053-12058.
Landers, J., K. Martin, N. Sarkies, R. Bourne and P. Watson (2012). "A twenty-year follow-up study of trabeculectomy: risk factors and outcomes." Ophthalmology 119(4): 694-702.
Levin, L. L. K., P. L.; Hartnett, M. E. (2024). Adler's Physiology of the Eye, Elsevier.
Lusthaus, J. and I. Goldberg (2019). "Current management of glaucoma." Med J Aust 210(4): 180-187.
Maestrini, H. A., S. Cronemberger, H. D. Matoso, J. R. Reis, R. V. Merula, A. D. Filho, E. Sakurai and G. A. Ferreira (2011). "Late needling of flat filtering blebs with adjunctive mitomycin C: efficacy and safety for the corneal endothelium." Ophthalmology 118(4): 755-762.
Mills, R. P., D. L. Budenz, P. P. Lee, R. J. Noecker, J. G. Walt, L. R. Siegartel, S. J. Evans and J. J. Doyle (2006). "Categorizing the stage of glaucoma from pre-diagnosis to end-stage disease." Am J Ophthalmol 141(1): 24-30.
Musch, D. C., B. W. Gillespie, L. M. Niziol, L. F. Cashwell, P. R. Lichter and G. Collaborative Initial Glaucoma Treatment Study (2008). "Factors associated with intraocular pressure before and during 9 years of treatment in the Collaborative Initial Glaucoma Treatment Study." Ophthalmology 115(6): 927-933.
Nieto, M., F. Navarro, J. J. Perez-Villar, M. A. del Pozo, R. Gonzalez-Amaro, M. Mellado, J. M. Frade, A. C. Martinez, M. Lopez-Botet and F. Sanchez-Madrid (1998). "Roles of chemokines and receptor polarization in NK-target cell interactions." J Immunol 161(7): 3330-3339.
Nikita, E. and I. Murdoch (2018). "Same-site surgical revision of failed trabeculectomy blebs with mitomycin C augmentation: long-term follow-up." Eye (Lond) 32(2): 352-358.
Olali, C., A. P. Rotchford and A. J. King (2011). "Outcome of repeat trabeculectomies." Clin Exp Ophthalmol 39(7): 658-664.
Parrish, R. K., 2nd, J. C. Schiffman, W. J. Feuer, D. K. Heuer and G. Fluorouracil Filtering Surgery Study (2001). "Prognosis and risk factors for early postoperative wound leaks after trabeculectomy with and without 5-fluorouracil." Am J Ophthalmol 132(5): 633-640.
Raj, A. Y., A.; Awad, J., Elahi, B. (2018). "Current Surgical Practice of Trabeculectomy in the United Kingdom (UK National Trabeculectomy Survey 2016)." Int J Ophthalmol Clin Res 5: 094.
Reibaldi, A., M. G. Uva and A. Longo (2008). "Nine-year follow-up of trabeculectomy with or without low-dosage mitomycin-c in primary open-angle glaucoma." Br J Ophthalmol 92(12): 1666-1670.
Salmon, J. F. (2024). Kanski's Clinical Ophthalmology, Elsevier.
Seet, L.-F., R. Su, V. A. Barathi, W. S. Lee, R. Poh, Y. M. Heng, E. Manser, E. N. Vithana, T. Aung, M. Weaver, E. H. Sage and T. T. Wong (2010). "SPARC Deficiency Results in Improved Surgical Survival in a Novel Mouse Model of Glaucoma Filtration Surgery." PLOS ONE 5(2): e9415.
Seet, L. F., S. W. L. Chu, X. Teng, L. Z. Toh and T. T. Wong (2020). "Assessment of progressive alterations in collagen organization in the postoperative conjunctiva by multiphoton microscopy." Biomed Opt Express 11(11): 6495-6515.
Seet, L. F., W. S. Lee, R. Su, S. N. Finger, J. G. Crowston and T. T. Wong (2011). "Validation of the glaucoma filtration surgical mouse model for antifibrotic drug evaluation." Mol Med 17(5-6): 557-567.
Shao, C. G., N. R. Sinha, R. R. Mohan and A. D. Webel (2023). "Novel Therapies for the Prevention of Fibrosis in Glaucoma Filtration Surgery." Biomedicines 11(3).
Sherwood, M. B. (2016) "The Pros and Cons of Using Mitomycin-C." Review of Ophthalmology.
Singer, A. J. and R. A. Clark (1999). "Cutaneous wound healing." N Engl J Med 341(10): 738-746.
Soltau, J. B., R. F. Rothman, D. L. Budenz, D. S. Greenfield, W. Feuer, J. M. Liebmann and R. Ritch (2000). "Risk factors for glaucoma filtering bleb infections." Arch Ophthalmol 118(3): 338-342.
Sugimoto, Y., H. Mochizuki, S. Ohkubo, T. Higashide, K. Sugiyama and Y. Kiuchi (2015). "Intraocular Pressure Outcomes and Risk Factors for Failure in the Collaborative Bleb-Related Infection Incidence and Treatment Study." Ophthalmology 122(11): 2223-2233.
Wagner, F. M., A. K. Schuster, K. Kianusch, J. Stingl, N. Pfeiffer and E. M. Hoffmann (2023). "Long-term success after trabeculectomy in open-angle glaucoma: results of a retrospective cohort study." BMJ Open 13(2): e068403.
Wagner, I. V., M. W. Stewart and S. K. Dorairaj (2022). "Updates on the Diagnosis and Management of Glaucoma." Mayo Clin Proc Innov Qual Outcomes 6(6): 618-635.
Weinreb, R. N., T. Aung and F. A. Medeiros (2014). "The pathophysiology and treatment of glaucoma: a review." JAMA 311(18): 1901-1911.
Weinreb, R. N., C. K. Leung, J. G. Crowston, F. A. Medeiros, D. S. Friedman, J. L. Wiggs and K. R. Martin (2016). "Primary open-angle glaucoma." Nat Rev Dis Primers 2: 16067.
Wilkins, M., A. Indar and R. Wormald (2005). "Intra-operative mitomycin C for glaucoma surgery." Cochrane Database Syst Rev 2005(4): CD002897.
Yadgari, M., Hassanpour, K. (2018). "Avascular blebs and late bleb leakage: A review of causes and management." J Ophthalm Optometr Sci 2: 31.
Akahoshi T, Wada C, Endo H, Hirota K, Hosaka S, Takagishi K, Kondo H, Kashiwazaki S, Matsushima K (1993). Expression of monocyte chemotactic and activating factor in rheumatoid arthritis. Regulation of its production in synovial cells by interleukin-1 and tumor necrosis factor. Arthritis Rheum. 36:762
Alam R, York J, Moyars M, Stafford S, Grant JA, Lee J, Forsythe P, Sim T, Ida N (1996). Increased MCP-1, RANTES, and MIP-1α in bronchoalveolar lavage fluid of allergic asthmatic patients. Am. J. Respir. Crit. Care Med. 153:1398
Altschul SF, Gish W, Miller W, Myers EW, Lipman DJ (1990), Basic local alignment search tool. J Mol Biol. 215(3):403-10.
Altschul SF, Madden TL, Schaffer AA, Zhang J, Zhang Z, Miller W, Lipman DJ (1997). Gapped BLAST and PSI-BLAST: a new generation of protein database search programs. Nucleic Acids Res. Sep 1;25(17):3389-402.
Amann B, Tinzmann R, Angelkort B (2003). ACE inhibitors improve diabetic nephropathy through suppression of renal MCP-1. Diabetes Care 26:2421
Anders HJ, Vielhauer V, Frink M, Linde Y, Cohen CD, Blattner SM, Kretzler M, Strutz F, Mack M, Grone HJ, Onuffer J, Horuk R, Nelson PJ, Schlöndorff D (2002). A chemokine receptor CCR-1 antagonist reduces renal fibrosis after unilateral ureter ligation. J. Clin. Invest. 109:251
Anders HJ, Vielhauer V, Schlöndorff D (2003). Chemokines and chemokine receptors are involved in the resolution or progression of renal disease. Kidney Int. 63:401
Aurup H et al. (1994). Nucleic Acids Res 22:20
Austin HA 3rd, Muenz LR, Joyce KM, Antonovych TT, Balow JE (1984). Diffuse proliferative lupus nephritis: identification of specific pathologic features affecting renal outcome. Kidney Int. 25:689
Baggiolini M, Dewald B, Moser B (1994). Interleukin-8 and related chemotactic cytokines - CXC and CC chemokines. Adv. Immunol. 55:97
Baggiolini M (1998). Chemokines and leukocyte traffic. Nature 392:565
Banba N, Nakamura T, Matsumura M, Kuroda H, Hattori Y, Kasai K (2000). Possible relationship of monocyte chemoattractant protein-1 with diabetic nephropathy. Kidney Int. 58:684
Banisor I, Leist TP, Kalman B (2005). Involvement of β-chemokines in the development of inflammatory demyelination. J. Neuroinflammation 2:7
Bazan JF, Bacon KB, Hardiman G, Wang W, Soo K, Rossi D, Greaves DR, Zlotnik A, Schall TJ (1997). A new class of membrane-bound chemokine with a CX3C motif. Nature 385:640
Berkhout TA (1997). J Biol Chem 272:16404
Bohle A, Wehrmann M, Bogenschutz O, Batz C, Muller CA, Muller GA (1991). The pathogenesis of chronic renal failure in diabetic nephropathy. Investigation of 488 cases of diabetic glomerulosclerosis. Pathol. Res. Pract. 187:251
Boring L, Gosling J, Chensue SW, Kunkel SL, Farese RV Jr, Broxmeyer HE, Charo IF (1997). Impaired monocyte migration and reduced type 1 (Th1) cytokine responses in C-C chemokine receptor 2 knockout mice. J. Clin. Invest. 100:2552
Boring L, Gosling J, Cleary M, Charo IF (1998). Decreased lesion formation in CCR2-/- mice reveals a role for chemokines in the initiation of atherosclerosis. Nature 394:894
Boring L, Gosling J, Monteclaro FS, Lusis AJ, Tsou CL, Charo IF (1996). Molecular cloning and functional expression of murine JE (monocyte chemoattractant protein 1) and murine macrophage inflammatory protein 1alpha receptors: evidence for two closely linked C-C chemokine receptors on chromosome 9. J. Biol. Chem. 271:7551
Bossink AW, Paemen L, Jansen PM, Hack CE, Thijs LG, Van Damme J (1995). Plasma levels of the chemokines monocyte chemotactic proteins-1 and -2 are elevated in human sepsis. Blood 86:3841
Bower G, Brown DM, Steffes MW, Vernier RL, Mauer SM (1980). Studies of the glomerular mesangium and the juxtaglomerular apparatus in the genetically diabetic mouse. Lab. Invest. 43:333
Charo IF, Myers SJ, Herman A, Franci C, Connolly AJ, Coughlin SR (1994). Molecular cloning and functional expression of two monocyte chemoattractant protein 1 receptors reveals alternative splicing of the carboxyl-terminal tails. Proc. Natl Acad. Sci. USA 91:2752
Chow FY, Nikolic-Paterson DJ, Ma FY, Ozols E, Rollins BJ, Tesch GH (2007). Monocyte chemoattractant protein-1-induced tissue inflammation is critical for the development of renal injury but not type 2 diabetes in obese db/db mice. Diabetologica 50:471
Chow FY, Nikolic-Paterson DJ, Ozols E, Atkins RC, Rollin BJ, Tesch GH (2006). Monocyte chemoattractant protein-1 promotes the development of diabetic renal injury in streptozotocin-treated mice. Kidney Int. 69:73
Chow F, Ozols E, Nikolic-Paterson DJ, Atkins RC, Tesch GH (2004). Macrophages in mouse type 2 diabetic nephropathy: Correlation with diabetic state and progressive renal injury. Kidney Int. 65:116
Cockwell P, Howie AJ, Adu D, Savage CO (1998). In situ analysis of C-C chemokine mRNA in human glomerulonephritis. Kidney Int. 54:827
Cohen CD, Gröne HJ, Gröne EF, Nelson PJ, Schlöndorff D, Kretzler M (2002). Laser microdissection and gene expression analysis on formaldehyde-fixed archival tissue. Kidney Int. 61:125
Cummins LL et al. (1995). Nucleic Acids Res 23:2019
Dalla Vestra M, Mussap M, Gallina P, Bruseghin M, Cernigoi AM, Saller A, Plebani M, Fioretto P (2005). Acute-phase markers of inflammation and glomerular structure in patients with type 2 diabetes. J. Am. Soc. Nephrol. 16 Suppl 1:S78
Dawson J, Miltz W, Mir AK, Wiessner C (2003). Targeting monocyte chemoattractant protein-1 signalling in disease. Expert Opin. Ther. Targets 7:35
De Bleecker JL, De Paepe B, Vanwalleghem IE, Schroder JM (2002). Differential expression of chemokines in inflammatory myopathies. Neurology 58:1779
Drolet DW, Nelson J, Tucker CE, Zack PM, Nixon K, Bolin R, Judkins MB, Farmer JA, Wolf JL, Gill SC, Bendele RA (2000). Pharmacokinetics and safety of an anti-vascular endothelial growth factor aptamer (NX1838) following injection into the vitreous humor of rhesus monkeys. Pharm. Res. 17:1503
Eaton BE et al. (1995). Chem Biol 2:633
Eaton BE, Gold L, Hicke BJ, Janjic N, Jucker FM, Sebosta DP, Tarasow TM, Willis MC, Zichi DA (1997). Bioorg Med Chem 5:1087
Economou E, Tousoulis D, Katinioti A, Stefanadis C, Trikas A, Pitsavos C, Tentolouris C, Toutouza MG, Toutouzas P (2001). Chemokines in patients with ischaemic heart disease and the effect of coronary angioplasty. Int. J. Cardiol. 80:55
Egashira K, Zhao Q, Kataoka C, Ohtani K, Usui M, Charo IF, Nishida K, Inoue S, Katoh M, Ichiki T, Takeshita A (2002). Importance of monocyte chemoattractant protein-1 pathway in neointimal hyperplasia after periarterial injury in mice and monkeys. Circ. Res. 90:1167
Fujinaka H, Yamamoto T, Takeya M, Feng L, Kawasaki K, Yaoita E, Kondo D, Wilson CB, Uchiyama M, Kihara I (1997). Suppression of anti-glomerular basement membrane nephritis by administration of anti-monocyte chemoattractant protein-1 antibody in WKY rats. J. Am. Soc. Nephrol. 8:1174
Furuichi K, Wada T, Iwata Y, Kitagawa K, Kobayashi K-I, Hashimoto H, Ishiwata Y, Tomosugi N, Mukaida N, Matsushima K, Egashira K, Yokoyama H (2003). Gene therapy expressing amino-terminal truncated monocyte chemoattractant protein-1 prevents renal ischemia-reperfusion injury. J. Am. Soc. Nephrol. 14:1066
Furuta T, Saito T, Ootaka T, Soma J, Obara K, Abe K, Yoshinaga K (1993). The role of macrophages in diabetic glomerulosclerosis. Am. J. Kidney Dis. 21:480
Galasso JM, Liu Y, Szaflarski J, Warren JS, Silverstein FS (2000). Monocyte chemoattractant protein-1 is a mediator of acute excitotoxic injury in neonatal rat brain. Neuroscience 101:737
Galkina E, Ley K (2006). Leukocyte recruitment and vascular injury in diabetic nephropathy. J. Am. Soc. Nephrol. 17:368-377
Gao JL, Kuhns DB, Tiffany HL, McDermott D, Li X, Francke U, Murphy PM (1993). Structure and functional expression of the human macrophage inflammatory protein 1 alpha/RANTES receptor. J. Exp. Med. 177:1421
Garcia-Zepeda EA, Combadiere C, Rothenberg ME, Sarafi MN, Lavigne F, Hamid Q, Murphy PM, Luster AD (1996). Human monocyte chemoattractant protein (MCP)-4 is a novel CC chemokine with activities on monocytes, eosinophils, and basophils induced in allergic and nonallergic inflammation that signals through the CC chemokine receptors (CCR)-2 and -3. J. Immunol. 157:5613
Gerard C, Rollins, BJ. Chemokines and disease. Nat. Immunol. 6:1182
Gong X, Gong W, Kuhns DB, Ben-Baruch A, Howard OM, Wang JM (1997). Monocyte chemotactic protein-2 (MCP-2) uses CCR1 and CCR2B as its functional receptors. J. Biol. Chem. 272:11682
Gonzalo JA, Lloyd CM, Wen D, Albar JP, Wells TNC, Proudfoot A, Martinez-A C, Dorf M, Bjerke T, Coyle AJ, Gutierrez-Ramos JC (1998). The coordinated action of CC chemokines in the lung orchestrates allergic inflammation and airway hyperresponsiveness. J. Exp. Med. 188:157
Gordillo GM, Onat D, Stockinger M, Roy S, Atalay M, Beck FM, Sen CK (2004). A key angiogenic role of moncyte chemoattractant protein-1 in hemangioendothelioma proliferation. Am. J. Physiol. Cell Physiol. 287:C866
Green LS et al. (1995). Chem Biol 2:683
Handel TM, Domaille PJ (1996). Heteronuclear (1H, 13C, 15N) NMR assignments and solution structure of the monocyte chemoattractant protein-1 (MCP-1) dimer. Biochemistry 35:6569
Harigai M, Hara M, Yoshimura T, Leonard EJ, Inoue K, Kashiwazaki S (1993). Monocyte chemoattractant protein-1 (MCP-1) in inflammatory joint diseases and its involvement in the cytokine network of rheumatoid synovium. Clin. Immunol. Immunopathol. 69:83
Hasegawa H, Kohno M, Sasaki M, Inoue A, Ito MR, Terada M, Hieshima K, Maruyama H, Miyazaki J, Yoshie O, Nose M, Fujita S (2003). Antagonist of monocyte chemoattractant protein 1 ameliorates the initiation and progression of lupus nephritis and renal vasculitis in MRL/lpr mice. Arthritis Rheum. 48:2555
Heath H, Qin S et al. (1997). Chemokine receptor usage by human eosinophils. The importance of CCR3 demonstrated using an antagonistic monoclonal antibody. J Clin Invest 99:178
Holdsworth SR, Kitching AR, Tipping PG (2000). Chemokines as therapeutic targets in renal disease. Curr. Opin. Nephrol. Hypertens. 9:505
Holgate ST, Bodey KS, Janezic A, Frew AJ, Kaplan AP, Teran LM (1997). Release of RANTES, MIP-1α, and MCP-1 into asthmatic airways following endobronchial allergen challenge. Am. J. Respir. Crit. Care Med. 156:1377
Hosaka S et al. (1994). Clin Exp Immunol 97:451
Huang DR, Wang J, Kivisakk P, Rollins BJ, Ransohoff RM (2001). Absence of monocyte chemoattractant protein 1 in mice leads to decreased local macrophage recruitment and antigen-specific T helper cell type 1 immune response in experimental autoimmune encephalomyelitis. J. Exp. Med. 193:713
Hulkower K, Brosnan CF, Aquino DA, Cammer W, Kulshrestha S, Guida MP, Rapoport DA, Berman JW (1993). Expression of CSF-1, c-fms, and MCP-1 in the central nervous system of rats with experimental allergic encephalomyelitis. J. Immunol. 150:2525
Humbert M, Ying S, Corrigan C, Menz G, Barkans J, Pfister R, Meng Q, Van Damme J, Opdenakker G, Durham SR, Kay AB (1997). Bronchial mucosal expression of the genes encoding chemokines RANTES and MCP-3 in symptomatic atopic and nonatopic asthmatics: relationship to the eosinophil-active cytokines interleukin (IL)-5, granulocyte macrophage-colony-stimulating factor, and IL-3. Am J Respir Cell Mol Biol 16:1
Ihm CG, Park JK, Hong SP, Lee TW, Cho BS, Kim MJ, Cha DR, Ha H (1998). A high glucose concentration stimulates the expression of monocyte chemotactic peptide 1 in human mesangial cells. Nephron 79:33
Iyonaga K, Takeya M, Saita N, Sakamoto O, Yoshimura T, Ando M, Takahashi K (1994). Monocyte chemoattractant protein-1 in idiopathic pulmonary fibrosis and other interstitial lung diseases. Hum. Pathol. 25:455
Johrer K, Zelle-Rieser C, Perathoner A, Moser P, Hager M, Ramoner R, Gander H, Holtl L, Bartsch G, Greil R, Thurnher M (2005). Up-regulation of functional chemokine receptor CCR3 in human renal cell carcinoma. Clin Cancer Res 11:2459
Jolicoeur C, Lemay A, Akoum A (2001). Comparative effect of danazol and a GnRH agonist on monocyte chemotactic protein-1 expression by endometriotic cells. Am. J. Reprod. Immunol. 45:86
Jose PJ, Griffiths-Johnson DA, Collins PD, Walsh DT, Moqbel R, Totty NF, Truong O, Hsuan JJ, Williams TJ. Eotaxin: a potent eosinophil chemoattractant cytokine detected in a guinea pig model of allergic airways inflammation. J. Exp. Med. 179:881
Kaburagi Y, Shimada Y, Nagaoka T, Hasegawa M, Takehara K, Sato S (2001). Enhanced production of CC-chemokines (RANTES, MCP-1, MIP-1α, MIP-1β, and eotaxin) in patients with atopic dermatitis. Arch. Dermatol. Res. 293:350
Kawasaki AM et al. (1993). J Med Chem 36:831
Kennedy KJ, Strieter RM, Kunkel SL, Lukacs NW, Karpus WJ (1998). Acute and relapsing experimental autoimmune encephalomyelitis are regulated by differential expression of the CC chemokines macrophage inflammatory protein-1α and monocyte chemotactic protein-1. J. Neuroimmunol. 91:98
Kim JS, Gautam SC, Chopp M, Zaloga C, Jones ML, Ward PA, Welch KM (1995). Expression of monocyte chemoattractant protein-1 and macrophage inflammatory protein-1 after focal cerebral ischemia in the rat. J. Neuroimmunol. 56:127
Kitamoto S, Egashira K (2003). Anti-monocyte chemoattractant protein-1 gene therapy for cardiovascular diseases. Expert Rev. Cardiovasc. Ther. 1:393
Kleinhans M, Tun-Kyi A, Gilliet M, Kadin ME, Dummer R, Burg G, and Nestle FO (2003). Functional expression of the eotaxin receptor CCR3 in CD30+ cutaneous T-cell lymphoma. Blood 101:1487
Koch AE, Kunkel SL, Harlow LA, Johnson B, Evanoff HL, Haines GK, Burdick MD, Pope RM, Strieter RM (1992). Enhanced production of monocyte chemoattractant protein-1 in rheumatoid arthritis. J. Clin. Invest. 90:772
Kouno J, Nagai H, Nagahata T, Onda M, Yamaguchi H, Adachi K, Takahashi H, Teramoto A, and Emi M (2004). Up-regulation of CC chemokine, CCL3L1, and receptors, CCR3, CCR5 in human glioblastoma that promotes cell growth. J Neurooncol 70:301
Kurihara T, Warr G, Loy J, Bravo R (1997). Defects in macrophage recruitment and host defense in mice lacking the CCR2 chemokine receptor. J. Exp. Med. 186:1757
Kusser W (2000). J Biotechnol 74:27-38
Kuziel WA, Morgan SJ, Dawson TC, Griffin S, Smithies O, Ley K, Maeda N (1997). Severe reduction in leukocyte adhesion and monocyte extravasation in mice deficient in CC chemokine receptor 2. Proc. Natl Acad. Sci. U S A 94:12053
Lesnik EA et al. (1993). Biochemistry 32:7832
Lloyd CM, Minto AW, Dorf ME, Proudfoot A, Wells TNC, Salant DJ, Gutierrez-Ramos JC (1997). RANTES and monocyte chemoattractant protein-1 (MCP-1) play an important role in the inflammatory phase of crescentic nephritis, but only MCP-1 is involved in crescent formation and interstitial fibrosis. J. Exp. Med. 185:1371
Lu BB, Rutledge BJ, Gu L, Fiorillo J, Lukacs NW, Kunkel SL, North R, Gerard C, Rollins BJ (1998). Abnormalities in monocyte recruitment and cytokine expression in monocyte chemoattractant protein-1 deficient mice. J. Exp. Med. 187:601
Lubkowski J, Bujacz G, Boque L, Domaille PJ, Handel TM, Wlodawer A (1997). The structure of MCP-1 in two crystal forms provides a rare example of variable quaternary interactions. Nat Struct Biol 4:64
Mack M, Cihak J, Simonis C, Luckow B, Proudfoot AE, Plachy J, Bruhl H, Frink M, Anders HJ, Vielhauer V, Pfirstinger J, Stangassinger M, Schlöndorff D (2001). Expression and characterization of the chemokine receptors CCR2 and CCR5 in mice. J. Immunol. 166:4697
Martinelli R, Sabroe I, LaRosa G, Williams TJ, Pease JE. The CC chemokine eotaxin (CCL11) is a partial agonist of CC chemokine receptor 2b. J Biol Chem 276:42957
Matsushima K, Morishita K, Yoshimura T, Lavu S, Kobayashi Y, Lew W, Appella E, Kung HF, Leonard EJ, Oppenheim JJ (1989). Molecular cloning of a human monocyte-derived neutrophil chemotactic factor (MDNCF) and the induction of MDNCF mRNA by interleukin 1 and tumor necrosis factor. J. Exp. Med. 167:1883
McGinnis S, Madden TL (2004). BLAST: at the core of a powerful and diverse set of sequence analysis tools. Nucleic Acids Res. 32(Web Server issue):W20-5. Meyer TW (2003). Immunosuppression for diabetic glomerular disease? Kidney Int. 63:377
Miller MD, Krangel MS (1992). Biology and biochemistry of the chemokines: a family of chemotactic and inflammatory cytokines. Crit. Rev. Immunol. 12:17
Miller LE et al. (1993). J Physiol 469:213
Mora C, Navarro JF (2005). The role of inflammation as a pathogenic factor in the development of renal disease in diabetes. Curr. Diab. Rep. 5:399
Morii T, Fujita H, Narita T, Shimotomai T, Fujishima H, Yoshioka N, Imai H, Kakei M, Ito S (2003). Association of monocyte chemoattractant protein-1 with renal tubular damage in diabetic nephropathy. J. Diabetes Complications 17:11
Murphy PM, Baggiolini M, Charo IF, Hebert CA, Horuk R, Matsushima K, Miller LH, Oppenheim JJ, Power CA (2000). International union of pharmacology. XXII. Nomenclature for chemokine receptors. Pharmacol. Rev. 52:145
Nakamura H, Weiss ST, Israel E, Luster AD, Drazen JM, Lilly CM (1999). Eotaxin and impaired lung function in asthma. Am J Respir Crit Care Med 160:1952
Nakazawa T, Hisatomi T, Nakazawa C, Noda K, Maruyama K, She H, Matsubara A, Miyahara S, Nakao S, Yin Y, Benowitz L, Hafezi-Moghadam A, Miller JW (2007). Monocyte chemoattractant protein 1 mediated retinal detachment-induced photoreceptor apoptosis. Proc Natl. Acad. Sci. U S A 104:2425
Navarro JF, Mora C, Maca M, Garca J (2003). Inflammatory parameters are independently associated with urinary albumin in type 2 diabetes mellitus. Am. J. Kidney Dis. 42:53
Myers SJ, Wong LM, Charo IF (1995). Signal transduction and ligand specificity of the human monocyte chemoattractant protein-1 receptor in transfected embryonic kidney cells. J. Biol. Chem. 270:5786
Needleman & Wunsch (1970), A general method applicable to the search for similarities in the amino acid sequence of two proteins. J Mol Biol. 48(3):443-53.
Nelken NA, Coughlin SR, Gordon D, Wilcox JN (1991). Monocyte chemoattractant protein-1 in human atheromatous plaques. J. Clin. Invest. 88:1121
Neote K, DiGregorio D, Mak JY, Horuk R, Schall TJ (1993). Molecular cloning, functional expression, and signaling characteristics of a C-C chemokine receptor. Cell 72:415
Ninichuk V, Gross O, Reichel C, Khandoga A, Pawar RD, Ciubar R, Segerer S, Belemezova E, Radomska E, Luckow B, de Lema GP, Murphy PM, Gao JL, Henger A, Kretzler M, Horuk R, Weber M, Krombach F, Schlondorff D, Anders HJ (2005). Delayed chemokine receptor 1 blockade prolongs survival in collagen 4A3-deficient mice with Alport disease. J. Am. Soc. Nephrol. 16:977
Ogata H, Takeya M, Yoshimura T, Takagi K, Takahashi K (1997). The role of monocyte chemoattractant protein-1 (MCP-1) in the pathogenesis of collagen-induced arthritis in rats. J. Pathol. 182:106
Okuno T, Andoh A, Bamba S, Araki Y, Fujiyama Y, Fujiyama M, Bamba T (2002). Interleukin-1β and tumor necrosis factor-α induce chemokine and matrix metalloproteinase gene expression in human colonic subepithelial myofibroblasts. Scand. J. Gastroenterol. 37:317
Oppenheim JJ, Zachariae CO, Mukaida N, Matsushima K (1991). Properties of the novel proinflammatory supergene "intercrine" cytokine family. Annu. Rev. Immunol. 9:617
Pawar RD, Patole PS, Zecher D, Segerer S, Kretzler M, Schlöndorff D, Anders HJ (2006). Toll-like receptor-7 modulates immune complex glomerulonephritis. J. Am. Soc. Nephrol. 17:141
Pearson & Lipman (1988), Improved tools for biological sequence comparison. Proc. Nat'l. Acad. Sci. USA 85: 2444
Perez de Lema G, Maier H, Franz TJ, Escribese M, Chilla mS, Segerer S, Camarasa N, Schmid H, Banas B, Kalaydjiev S, Busch DH, Pfeffer K, Mampaso F, Schlöndorff D, Luckow B (2005). Chemokine receptor CCR2 deficiency reduces renal disease and prolongs survival in MRL/lpr lupus-prone mice. J. Am. Soc. Nephrol. 16:3592
Perez de Lema G, Maier H, Nieto E, Vielhauer V, Luckow B, Mampaso F, Schlöndorff D. Chemokine expression precedes inflammatory cell infiltration and chemokine receptor and cytokine expression during the initiation of murine lupus nephritis. J. Am. Soc. Nephrol. 12:1369
Ponath PD, Qin S, Ringler DJ, Clark-Lewis I, Wang J, Kassam N, Smith H, Shi X, Gonzalo JA, Newman W, Gutierrez-Ramos JC, Mackay CR (1996a). Cloning of the human eosinophil chemoattractant, eotaxin. Expression, receptor binding, and functional properties suggest a mechanism for the selective recruitment of eosinophils. J. Clin. Invest. 97:604
Ponath PD, Qin S, Post TW, Wang J, Wu L, Gerard NP, Newman W, Gerard C, Mackay CR (1996b). Molecular cloning and characterization of a human eotaxin receptor expressed selectively on eosinophils. J. Exp. Med. 183:2437
Power CA, Meyer A, Nemeth K, Bacon KB, Hoogewerf AJ, Proudfoot AE, Wells TN (1995). Molecular cloning and functional expression of a novel CC chemokine receptor cDNA from a human basophilic cell line. J. Biol. Chem. 270:19495
Qi Z, Whitt I, Mehta A, Jin J, Zhao M, Harris RC, Fogo AB, Breyer MD (2004). Serial determination of glomerular filtration rate in conscious mice using FITC-inulin clearance. Am. J. Physiol. Renal Physiol. 286:F590
Qin S, LaRosa G, Campbell JJ, Smith-Heath H, Kassam N, Shi X, Zeng L, Buthcher EC, Mackay CR (1996). Expression of monocyte chemoattractant protein-1 and interleukin-8 receptors on subsets of T cells: correlation with transendothelial chemotactic potential. Eur. J. Immunol. 26:640
Ransohoff RM et al. (1993). FASEB J 7:592
Raport CJ, Gosling J, Schweickart VL, Gray PW, Charo IF (1996). Molecular cloning and functional characterization of a novel human CC chemokine receptor (CCR5) for RANTES, MIP-1β, and MIP-1α. J. Biol. Chem. 271:17161
Ritz E, Rychlik I, Locatelli F, Halimi S (1999). End-stage renal failure in type 2 diabetes: A medical catastrophe of worldwide dimensions. Am. J. Kidney Dis. 34:795-808
Rollins BJ, Stier P, Ernst T, Wong GG (1989). The human homolog of the JE gene encodes a monocyte secretory protein. Mol. Cell Biol. 9:4687
Rollins BJ (1996). Monocyte chemoattractant protein 1: a potential regulator of monocyte recruitment in inflammatory disease. Mol. Med. Today 2:198
Rovin BH, Rumancik M, Tan L, Dickerson J (1994). Glomerular expression of monocyte chemoattractant protein-1 in experimental and human glomerulonephritis. Lab. Invest. 71:536
Ruffing N, Sullivan N, et al. (1998). CCR5 has an expanded ligand-binding repertoire and is the primary receptor used by MCP-2 on activated T cells. Cell Immunol 189:160
Salcedo R, Ponce ML, Young HA, Wasserman K, Ward JM, Keinman HK, Oppenheim JJ, Murphy WJ (2000). Human endothelial cells express CCR2 and respond to MCP-1: direct role of MCP-1 in angiogenesis and tumor progression. Blood 96:34
Samson M, Labbe O, Mollereau C, Vassart G, Parmentier M (1996). Molecular cloning and functional expression of a new human CC-chemokine receptor gene. Biochemistry 35:3362
Schall TJ, Bacon KB (1994). Chemokines, leukocyte trafficking, and inflammation. Curr. Opin. Immunol. 6:865
Schneider A, Panzer U, Zahner G, Wenzel U, Wolf G, Thaiss F, Helmchen U, Stahl RA (1999). Monocyte chemoattractant protein-1 mediates collagen deposition in experimental glomerulonephritis by transforming growth factor-beta. Kidney Int. 56:135
Schwarting A, Paul K, Tschirner S, Menke J, Hansen T, Brenner W, Kelly VR, Relle M, Galle PR (2005). Interferon-beta: a therapeutic for autoimmune lupus in MRL-Faslpr mice. J. Am. Soc. Nephrol. 16:3264
Schwartz CJ, Valente AJ, Sprague EA (1993). A modern view of atherogenesis. Am. J. Cardiol. 71:9B
Segerer S, Nelson PJ, Schlöndorff D (2000). Chemokines, chemokine receptors, and renal disease: from basic science to pathophysiologic and therapeutic studies. J. Am. Soc. Nephrol. 11:152
Shimizu S, Nakashima H, Masutani K, Inoue Y, Miyake K, Akahoshi M, Tanaka Y, Egashira K, Hirakata H, Otsuka T, Harada M (2004). Anti-monocyte chemoattractant protein-1 gene therapy attenuates nephritis in MRL/lpr mice. Rheumatology (Oxford) 43:1121
Smith & Waterman (1981), Adv. Appl. Math. 2: 482
Springer TA (1995). Traffic signals on endothelium for lymphocyte recirculation and leukocyte emigration. Annu. Rev.Physiol. 57:827
Steinman L (2004). Immune therapy for autoimmune diseases. Science 305:212
Svensson M, Sundkvist G, Arnqvist HJ, Bjork E, Blohme G, Bolinder J, Henricsson M, Nystrom L, Torffvit O, Waernbaum I, Ostman J, Eriksson JW (2003). Signs of nephropathy may occur early in young adults with diabetes despite modern diabetes management: Results from the nationwide population-based Diabetes Incidence Study in Sweden (DISS). Diabetes Care 26:2903
Takebayashi K, Matsumoto S, Aso Y, Inukai T (2006). Association between circulating monocyte chemoattractant protein-1 and urinary albumin excretion in nonobese Type 2 diabetic patients. J. Diabetes Complications 20:98
Takeya M, Yoshimura T, Leonard EJ, Takahashi K (1993). Detection of monocyte chemoattractant protein-1 in human atherosclerotic lesions by an anti-monocyte chemoattractant protein-1 monoclonal antibody. Hum. Pathol. 24:534
Tang WW, Qi M, Warren JS (1996). Monocyte chemoattractant protein 1 mediates glomerular macrophage infiltration in anti-GBM Ab GN. Kidney Int. 50:665
Tashiro K, Koyanagi I, Saitoh A, Shimizu A, Shike T, Ishiguro C, Koizumi M, Funabiki K, Horikoshi S, Shirato I, Tomino Y (2002). Urinary levels of monocyte chemoattractant protein-1 (MCP-1) and interleukin-8 (IL-8), and renal injuries in patients with type 2 diabetic nephropathy. J. Clin. Lab. Anal.16:1
Tesch GH, Maifert S, Schwarting A, Rollins BJ, Kelley VR (1999). Monocyte chemoattractant protein 1-dependent leukocytic infiltrates are responsible for autoimmune disease in MRL-Fas(lpr) mice. J. Exp. Med. 190:1813
Tuaillon N, Shen de F, Berger RB, Lu B, Rollins BJ, Chan CC (2002). MCP-1 expression in endotoxin-induced uveitis. Invest. Ophthalmol. Vis. Sci. 43:1493
Tuttle KR (2005). Linking metabolism and immunology: diabetic nephropathy is an inflammatory disease. J. Am. Soc. Nephrol. 16:1537
Uguccioni M, Mackay CR et al. (1997). High expression of the chemokine receptor CCR3 in human blood basophils. Role in activation by eotaxin, MCP-4, and other chemokines. J Clin Invest 100:1137
United States Renal Data System (2004). Annual data report: Incidence and prevalence 2004. Am. J. Kidney Dis. 45:S77
Utimura R, Fujihara CK, Mattar AL, Malheiros DM, Noronha IL, Zatz R (2003). Mycophenolate mofetil prevents the development of glomerular injury in experimental diabetes. Kidney Int. 63:209
Van Riper G, Siciliano S, Fischer PA, Meurer R, Springer MS, Rosen H (1993). Characterization and species distribution of high affinity GTP-coupled receptors for human rantes and monocyte chemoattractant protein 1. J. Exp. Med. 177:851
Venkatesan N et al. (2003). Curr Med Chem 10:1973
Vestergaard C, Just H, Baumgartner Nielsen J, Thestrup-Pedersen K, Deleuran M (2004). Expression of CCR2 on monocytes and macrophages in chronically inflamed skin in atopic dermatitis and psoriasis. Acta Derm. Venereol. 84:353
Viedt C, Orth SR (2002). Monocyte chemoattractant protein-1 (MCP-1) in the kidney: does it more than simply attract monocytes? Nephrol. Dial. Transplant. 17:2043
Wada T, Furuichi K, Segada-Takaeda C, Ahimizu M, Sakai N, Takeda SI, Takasawa K, Kida H, Kobayashi KI, Mukaida N, Ohmoto Y, Matsushima K, Yokoyama H (1999). MIP-1α and MCP-1 contribute to crescents and interstitial lesions in human crescentic glomerulonephritis. Kidney Int. 56:995
Wada T, Yokoyama H, Matsushima K, Kobayashi KI (2001). Chemokines in renal diseases. Int. Immunopharmacol. 1:637
Wada T, Yokoyama H, Furuichi K, Kobayashi KI, Harada K, Naruto M, Su SB, Akiyama M, Mukaida N, Matsushima K (1996). Intervention of crescentic glomerulonephritis by antibodies to monocyte chemotactic and activating factor (MCAF/MCP-1). FASEB J. 10:1418
Wang X, Yue TL, Barone FC, Feuerstein GZ (1995). Monocyte chemoattractant protein-1 messenger RNA expression in rat ischemic cortex. Stroke 26:661
Wenzel U, Schneider A, Valente AJ, Abboud HE, Thaiss F, Helmchen UM, Stahl RA (1997). Monocyte chemoattractant protein-1 mediates monocyte/macrophage influx in anti-thymocyte antibody-induced glomerulonephritis. Kidney Int. 51:770
Yamagishi S, Inagaki Y, Okamoto T, Amano S, Koga K, Takeuchi M, Makita Z (2002). Advanced glycation end product-induced apoptosis and overexpression of vascular endothelial growth factor and monocyte chemoattractant protein-1 in human-cultured mesangial cells. J. Biol. Chem. 277:20309
Ying S, Robinson DS, Meng Q, Rottman J, Kennedy R, Ringler DJ, Mackay CR, Daugherty BL, Springer MS, Durham SR, Williams TJ, Kay AB (1997). Enhanced expression of eotaxin and CCR3 mRNA and protein in atopic asthma. Association with airway hyperresponsiveness and predominant co-localization of eotaxin mRNA to bronchial epithelial and endothelial cells. Eur J Immunol 27:3507
Ying S, Meng Q, Zeibecoglou K, Robinson DS, Macfarlane A, Humbert M, Kay AB (1999). Eosinophil chemotactic chemokines (eotaxin, eotaxin-2, RANTES, monocyte chemoattractant protein-3 (MCP-3), and MCP-4), and C-C chemokine receptor 3 expression in bronchial biopsies from atopic and nonatopic (Intrinsic) asthmatics. J Immunal 163:6321
Yla-Herttuala S, Lipton BA, Rosenfeld ME, Sarkioja T, Yoshimura T, Leonard EJ, Witztum JL, Steinberg D (1991). Expression of monocyte chemoattractant protein 1 in macrophage-rich areas of human and rabbit atherosclerotic lesions. Proc. Natl Acad. Sci. U S A 88:5252
Yoshimura T, Robinson EA, Tanaka S, Appella E, Leonard EJ (1989). Purification and amino acid analysis of two human monocyte chemoattractants produced by phytohemagglutinin-stimulated human blood mononuclear leukocytes. J. Immunol. 142:1956
Yozai K, Shikata K, Sasaki M, Tone A, Ohga S, Usui H, Okada S, Wada J, Nagase R, Ogawa D, Shikata Y, Makino H (2005). Methotrexate prevents renal injury in experimental diabetic rats via anti-inflammatory actions. J. Am. Soc. Nephrol. 16:3326
Zimmet P, Alberti KG, Shaw J (2001). Global and societal implications of the diabetes epidemic. Nature 414:782
The features of the present invention disclosed in the specification, the claims and/or the drawings may both separately and in any combination thereof be material for realizing the invention in various forms thereof.

## Claims

1. An inhibitor of Monocyte Chemoattractant Protein-1 (CCL2) activity, for use in a method for treating a subject suffering from or being at risk of suffering from an eye disease, wherein the eye disease is glaucoma and wherein the inhibitor of CCL2 activity is locally and systemically administered to the subject, preferably local administration is subconjunctival administration and systemic administration is selected from the group consisting of subcutaneous administration, intravenous administration and any combination thereof.

2. The inhibitor of Monocyte Chemoattractant Protein-1 (CCL2) activity for use of claim 1, wherein the subject suffering from glaucoma or being at risk of suffering from glaucoma is a subject who qualifies for or is amenable to surgery for reducing intraocular pressure, preferably surgery for reducing intraocular pressure comprises glaucoma filtration surgery or minimally invasive glaucoma surgery, more preferably surgery for reducing intraocular pressure is glaucoma filtration surgery.

3. The inhibitor of Monocyte Chemoattractant Protein-1 (CCL2) activity for use of any one of claims 1 to 2, wherein the subject is a subject with risk of failure of surgery for reducing intraocular pressure, preferably glaucoma filtration surgery or minimally invasive glaucoma surgery, more preferably glaucoma filtration surgery.

4. The inhibitor of Monocyte Chemoattractant Protein-1 (CCL2) activity for use of any one of claims 1 to 3, wherein the subject has undergone surgery for reducing intraocular pressure, preferably glaucoma filtration surgery or minimally invasive glaucoma surgery, more preferably the subject has undergone glaucoma filtration surgery.

5. The inhibitor of Monocyte Chemoattractant Protein-1 (CCL2) activity for use of any one of claims 1 to 4, wherein an eye of the subject shows fibrosis or is at risk of showing fibrosis, preferably fibrosis is or may be a consequence of surgery for reducing intraocular pressure, more preferably of glaucoma filtration surgery or of minimally invasive glaucoma surgery, most preferably fibrosis is or may be a consequence of glaucoma filtration surgery.

6. The inhibitor of Monocyte Chemoattractant Protein-1 (CCL2) activity for use of any one of claims 1 to 5, wherein mitomycin C and/or 5-fluorouracil is administered to the subject, preferably a subtherapeutic amount of mitomycin C and/or 5-fluorouracil is administered to the subject, more preferably a subtherapeutic amount of mitomycin C and/or 5-fluorouracil is locally administered to the subject, most preferably a subtherapeutic amount of mitomycin C and/or 5-fluorouracil is locally administered to the subject at the site of glaucoma filtration surgery.

7. An inhibitor of Monocyte Chemoattractant Protein-1 (CCL2) activity, for use in a method for treating a subject post surgery for reducing intraocular pressure, preferably post glaucoma filtration surgery or post minimally invasive glaucoma surgery, wherein the inhibitor of CCL2 activity is locally and systemically administered to the subject, more preferably surgery for reducing intraocular pressure is glaucoma filtration surgery and local administration is subconjunctival administration and systemic administration is selected from the group consisting of subcutaneous administration, intravenous administration and any combination thereof.

8. The inhibitor of Monocyte Chemoattractant Protein-1 (CCL2) activity for use of claim 7, wherein the subject is suffering from glaucoma, has been suffering from glaucoma or is at risk of suffering from glaucoma.

9. The inhibitor of Monocyte Chemoattractant Protein-1 (CCL2) activity for use of any one of claims 7 to 8, wherein the subject is a subject with risk of failure of glaucoma filtration surgery.

10. The inhibitor of Monocyte Chemoattractant Protein-1 (CCL2) activity for use of any one of claims 7 to 9, wherein the eye of the subject shows fibrosis or is at risk of showing fibrosis, preferably fibrosis causes or may cause an increase in intraocular pressure in the eye on which surgery for reducing intraocular pressure is performed, more preferably surgery for reducing intraocular pressure is glaucoma filtration surgery.

11. The inhibitor of Monocyte Chemoattractant Protein-1 (CCL2) activity for use of any one of claims 7 to 10, wherein mitomycin C and/or 5-fluorouracil is administered to the subject, preferably a subtherapeutic amount of mitomycin C and/or 5-fluorouracil is administered to the subject, more preferably a subtherapeutic amount of mitomycin C and/or 5-fluorouracil is locally administered to the subject, most preferably a subtherapeutic amount of mitomycin C and/or 5-fluorouracil is locally administered to the subject at the site of glaucoma filtration surgery.

12. An inhibitor of Monocyte Chemoattractant Protein-1 (CCL2) activity, for use in a method for treating a subject post glaucoma filtration surgery of an eye of the subject, wherein the method comprises administering the inhibitor of CCL2 activity to the subject for maintaining functionality of vasculature of a bleb resulting from glaucoma filtration surgery or for avoiding or decreasing deterioration of vasculature of a bleb resulting from glaucoma filtration surgery, preferably the inhibitor of CCL2 activity is locally administered to the subject, more preferably local administration is subconjunctival administration.

13. The inhibitor of Monocyte Chemoattractant Protein-1 (CCL2) activity for use of any one of claims 7 to 12, wherein the subject is suffering from glaucoma, has been suffering from glaucoma or is at risk of suffering from glaucoma, preferably intraocular pressure of the eye of the subject is or has been > 21 mm Hg prior to glaucoma filtration surgery.

14. The inhibitor of Monocyte Chemoattractant Protein-1 (CCL2) activity for use of any one of claims 1 to 13, wherein the inhibitor of CCL2 activity is anti-fibrotic.

15. The inhibitor of Monocyte Chemoattractant Protein-1 (CCL2) activity for use of any one of claims 1 to 14, wherein the inhibitor of CCL2 activity is a nucleic acid molecule capable of binding to CCL2, wherein the nucleic acid molecule comprises a nucleotide sequence of SEQ IQ NO: 37 or a nucleotide sequence having an identity of at least 85 % to the nucleotide sequence of SEQ ID NO: 37, preferably the nucleic acid comprises a modification, more preferably the modification comprises a polyethylene glycol (PEG) molecule having a molecular weight of about 40 kDa, most preferably the inhibitor of CCL2 activity is NOX-E36.
